# EUROPEAN PATENT APPLICATION

(11) **EP 4 342 972 A1**
(43) Date of publication of application: **27.03.2024**
(21) Application number: 22197825.7
(22) Date of filing: 26.09.2022
(51) Int. Cl.: C12M 1/00

(54) **IMMOBILIZED EXTRACELLULAR VESICLES SUITABLE FOR IN VITRO ASSAYS**

(71) Applicant: Max-Planck-Gesellschaft zur Förderung der Wissenschaften e.V., 80539 München (DE)
(72) Inventor: EICHELE, Gregor, 37077 Göttingen (DE); DITTE, Zuzana, 37077 Göttingen (DE)
(74) Representative: Wichmann, Hendrik

(57) **Abstract**

Isolation of extracellular vesicles (EVs) is a tedious and costly process and EVs can often not be stored well. The application relates to a sterile surface suitable for culturing cells, wherein the surface is coated with extracellular vesicles ('EVs') immobilized at one or more predetermined position(s). It has been surprisingly found that extracellular vesicles (EVs) can be immobilized on a sterile surface, for example by drying. Additionally, a device for culturing cells comprising said surface further comprising a removable covering to preserve sterility of said surface is disclosed. Furthermore, a method of preparing said sterile surface is disclosed. Finally, the application also discloses methods of monitoring cell differentiation, apoptosis, cell migration, proliferation, transcriptome, proteome, and/or viability of cells ('recipient cells'), wherein said recipient cells are added to said EV-coated surface and then monitored.

## Description

### Background of the invention

Extracellular vesicles (EVs) are lipid bound vesicles secreted by cells into the extracellular space. The three main subtypes of EVs are exosomes, microvesicles, and apoptotic bodies and the content, or cargo, of EVs consists of lipids, nucleic acids, and proteins. Importantly, EVs participate in cell-cell communication, cell maintenance, and tumor progression. For example, in the nervous system, EVs have been found to help promote differentiation, myelin formation, and neuronal survival, thus playing a role in tissue development, repair and regeneration. For example, EVs mediate communication *in vitro and in vivo* [26, 27] between different cell types such as neurons and oligodendrocytes [28], neurons and astroglia [29, 30] and neurons and Schwann cells [31]. In addition, EVs involved in intercellular communication do so in the pre-metastatic niche [32], for instance by promoting breast cancer cells motility via Wnt-planar cell polarity signaling [33]. EVs also play a role in the mesenchymal stem cell niche [34] and in the epithelial-mesenchyme niche [35]. Glioma-derived EVs drive differentiation of embryonic neural stem cells to astrocytes [36] and EVs are thought to regulate neurogenesis in the early postnatal mouse brain [37].

In order to study the function, content and effect of EVs in *in vitro* assays, high quality EV preparations are required. Generally, EVs need to be isolated from cells or even tissues. Generally, the purification of EVs is a very costly and tedious process, which usually requires several centrifugation steps for which the equipment is only present in specialized laboratories. There are commercially available kits to facilitate EV purification but experts view these kits often as inefficient. Furthermore, antibody markers for EV characterization are commercially available, e.g. from ThermoFisher Scientific, Milteney Biotec, Amsbio, Takarabio, Qiagene, and Miltenyibiotec. However, the purification of EVs poses several challenges. The quality and quantity of EVs obtained when purifying EVs from cells or tissue varies depending on the purification method. Furthermore, the reproducibility of results and the effect of EVs on e.g. the differentiation of cells highly dependent the quality of the preparation. In addition, buffer components and potential impurities may not be compatible with the envisioned *in vitro* assay, as e.g. the cells are sensitive to potential contaminants and/or buffer components so that the cells may respond to the potential contaminant and/or buffer components and not to the EVs and their cargo. Even when including tailored controls, contaminants and buffer components of the EV preparation are often not ideal for the *in vitro* assay.

In addition, the shelf-life of EV preparations in solution is also rather limited and often declines over days in the fridge. Moreover, freezing of EV preparations causes a loss of the quality of the preparation. For example, US20210069254 discloses various storage buffers including e.g. saccharide moieties and polypeptides so that EVs can be frozen in solution. However, this has the disadvantage that said buffer needs to be compatible with the envisioned *in vitro* assay. In addition, a buffer exchange causes a great loss of EV quantity, which is very costly as the preparation of EVs is very tedious usually requiring state-of-the art ultracentrifugation. Freezing may cause the EVs to burst and that the proteins may denature upon freezing. This results in less effective EV preparations. At the same time, the reproducibility of EV preparations greatly suffers when solutions containing the EVs are frozen as the loss of effectiveness also depends on the buffer components, the freezing conditions and possibly also the type of EVs.

As the purification of EVs is a very costly and tedious process, there is a need in the art to more reproducibly prepare EVs, which are ideally storable for at least a few weeks. Furthermore, it would be greatly advantageous if there would be EV libraries available, which are independent of buffer components and largely independent of impurities so that the skilled person is able to test and/or monitor, e.g. cell differentiation, cell migration. An EV library would also have the advantage that EVs from various sources and concentrations may be tested side by side.

### Summary of invention

EVs of various sources can be purified, wherein the EVs are dissolved in the buffer of the last purification step. To overcome the challenge of reproducibility and to obtain storable EVs, the inventors surprisingly discovered that EVs can be immobilized on a sterile surface while essentially maintaining their activity. Specifically, a solution comprising EVs may be dried on a cell culture surface to obtain a surface being coated with immobilized EVs. Advantageously, the immobilized EVs can then be frozen and kept at -20 or -80°C. Hence, said EVs have the advantage that the cell culture surface can be easily shipped and/or stored as the EVs are immobilized on the surface and as the surface is free of solutions. Furthermore, the liquid solution has ideally evaporated so that the surface comprising the immobilized EVs can be easily frozen down and thawed again. Moreover, the immobilized EVs on the surface have the advantage that the EVs are not supplied in a buffer so that any *in vitro assay* can be performed without taking into account the components of the buffer of the EVs.

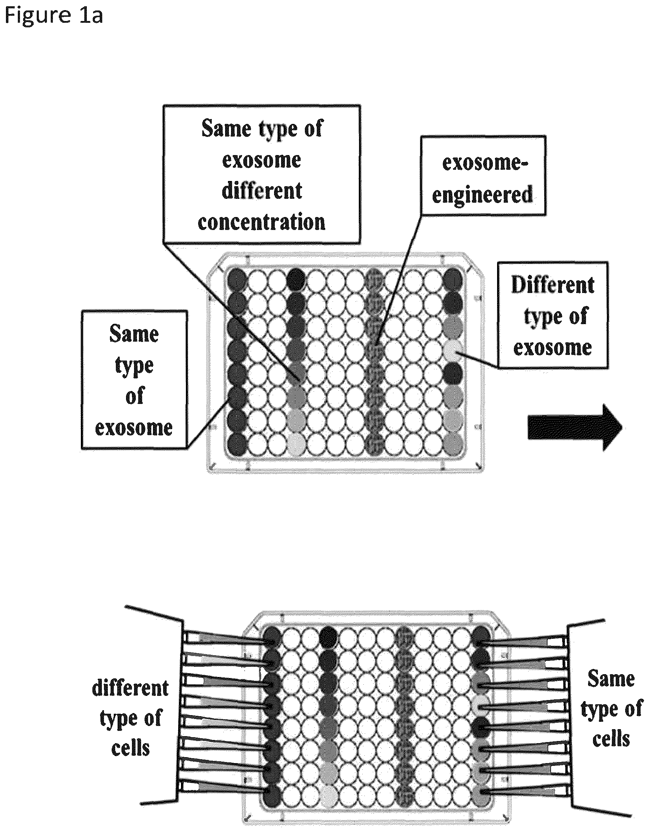

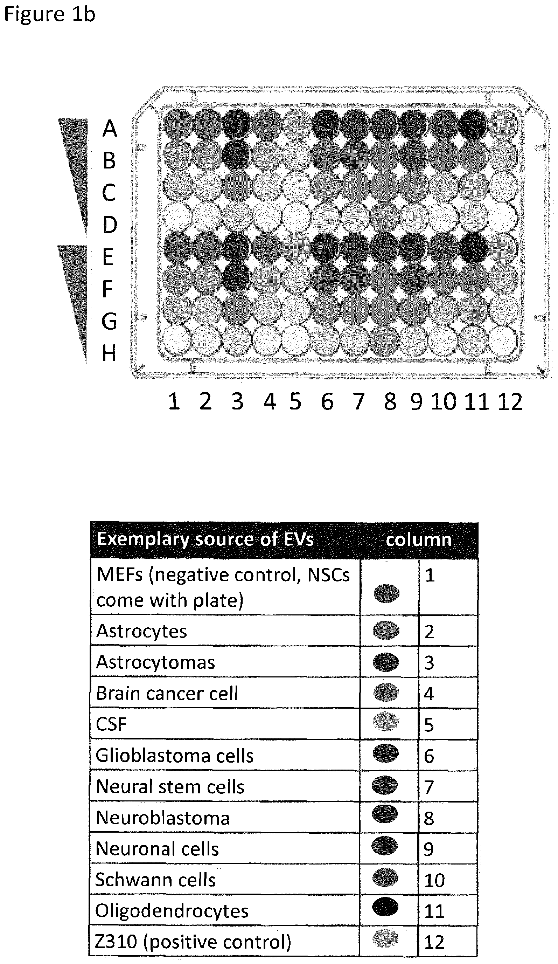

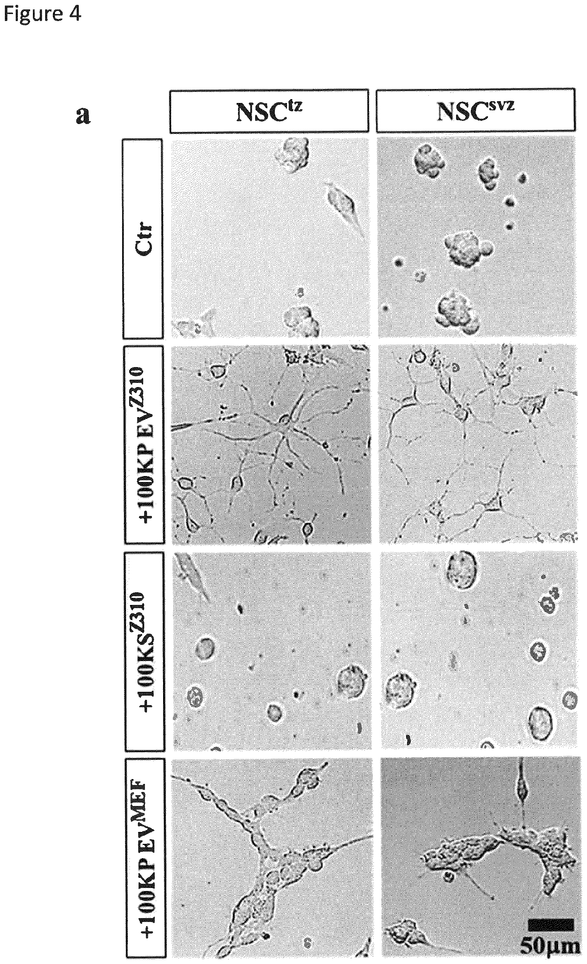

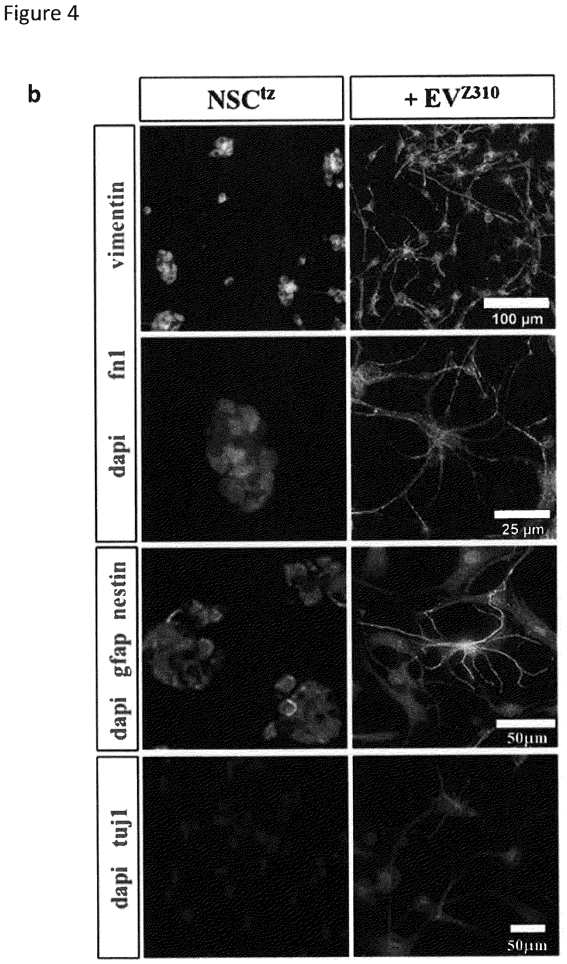

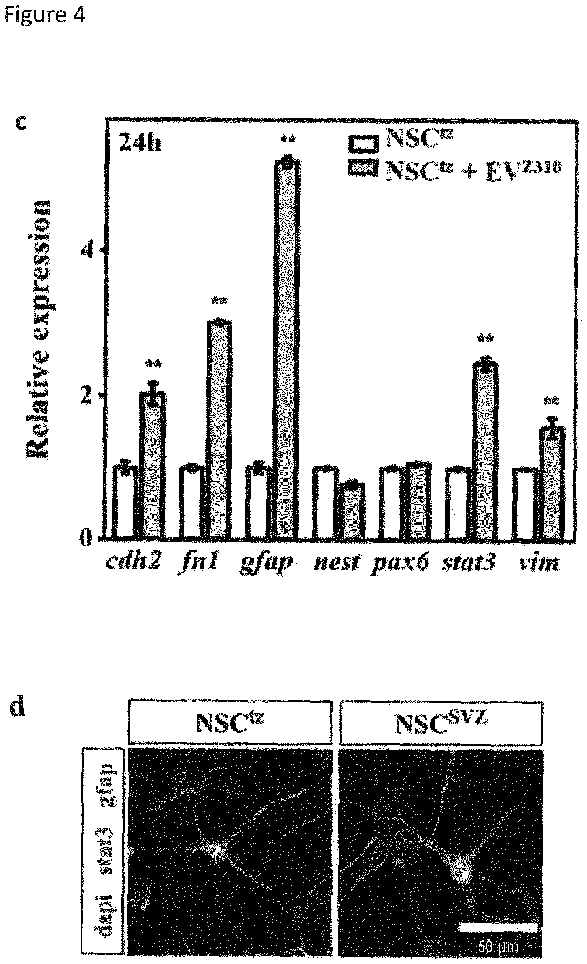

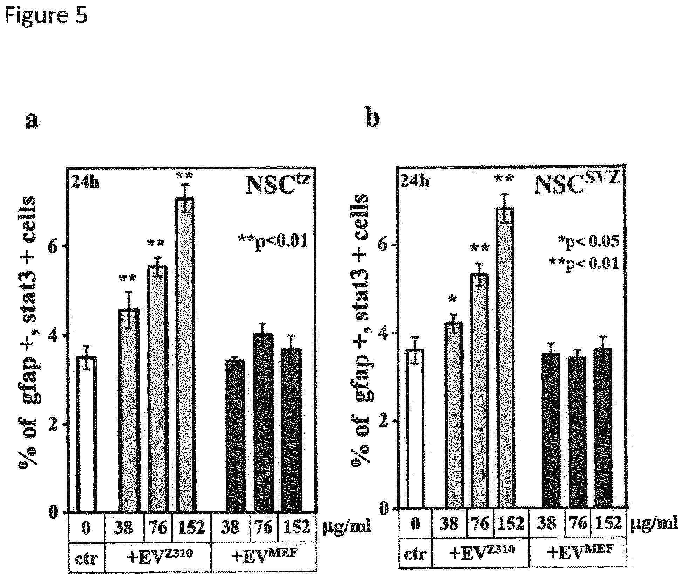

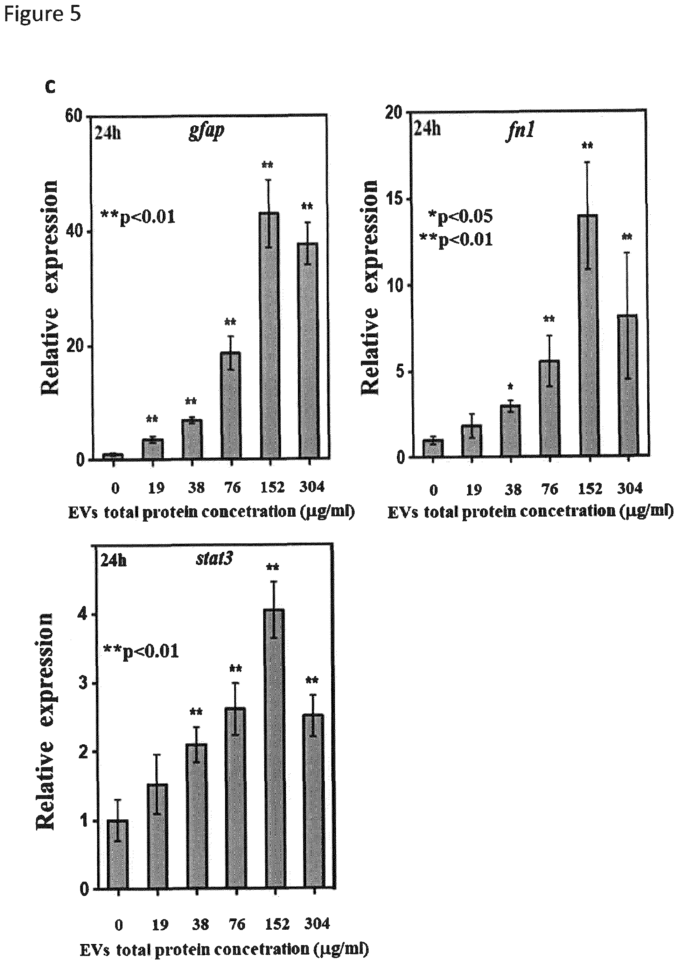

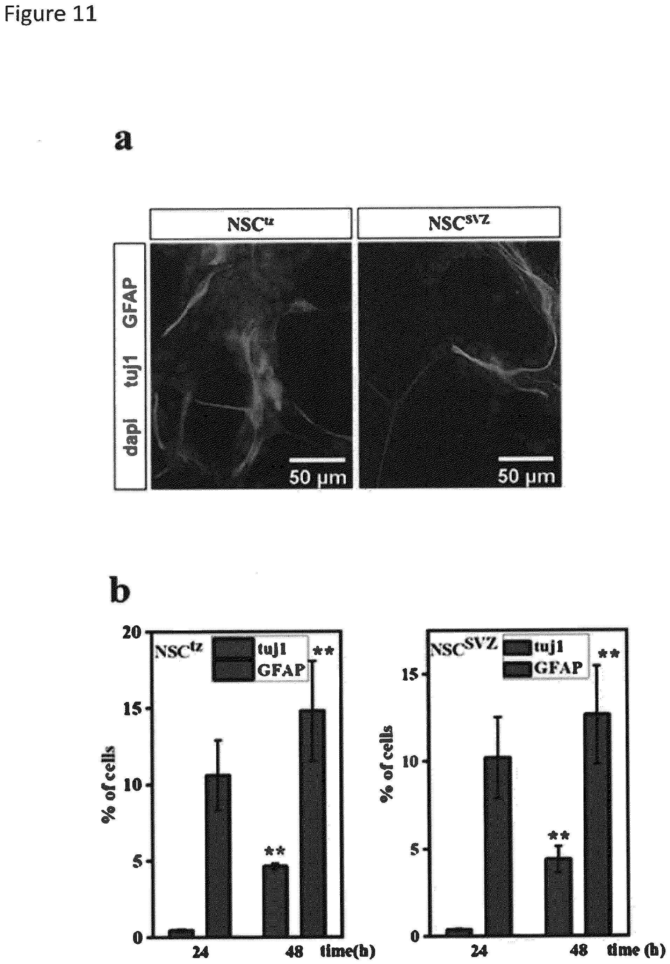

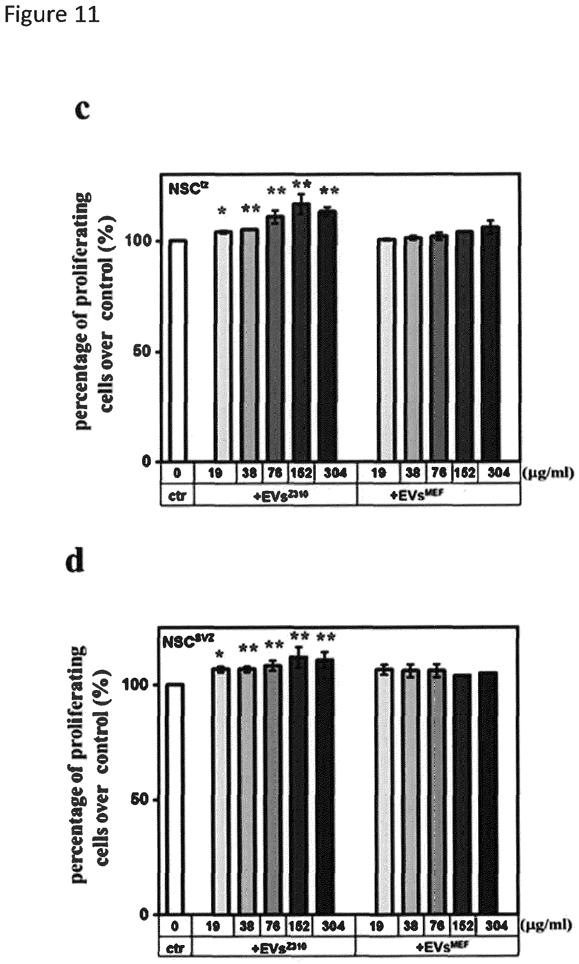

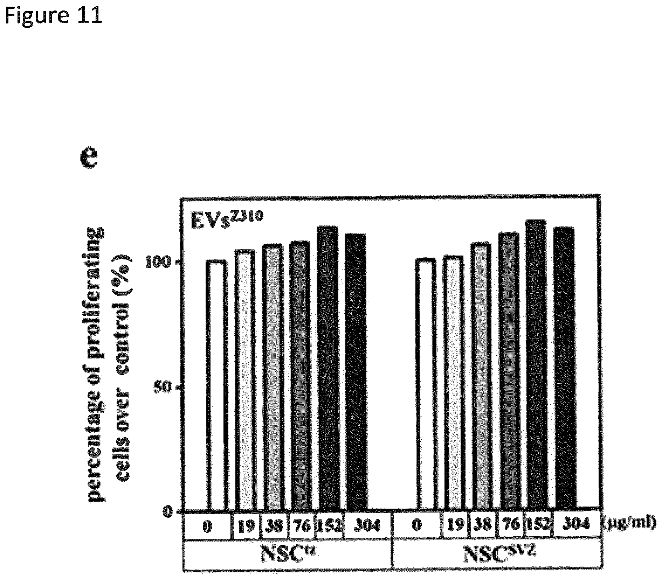

As an example, the inventors purified extracellular vesicles (EVs) from the supernatant of choroid plexus (ChP) cells by differential centrifugation followed by flotation gradient centrifugation. The choroid plexus is a secretory tissue that produces cerebrospinal fluid (CSF) that circulates in the nervous system and contains EVs. As demonstrated in Example 1 in further detail, 1µl drops of a suspension of EVs may be applied to the cell culture surface and the drops may be dried within minutes to obtain immobilized EVs on a cell culture surface. When neural stem cells (NSCs) isolated from mouse brain were added to immobilized EVs, the EVs evoked the differentiation of the NSCs (Example 1, and e.g. Figure 4 and 5). NSCs attached, differentiated and formed a cellular network in the area of the drop within just 24 hours. The inventors also surprisingly found that immobilized EVs can also be frozen and kept at e.g. -20⁰ C without impairment of their differentiation activity. In contrast, EVs purified from fibroblasts failed to evoke NSC differentiation. Thus, cellular responses are specific to particular recipient cell/EV combinations. More generally, immobilized EVs offer the opportunity to combine EVs of diverse origin with cells of different type with limited amounts of material. In Example 1, NSCs differentiate to cellular networks, which were scored by a morphometric analysis and with specific antibodies. Other types of cellular responses to EVs such as cell growth, mitotic activity, apoptosis can also be quantified (see e.g. Example 1, Figure 11 c, d, and e). The immobilized EVs as disclosed herein also offer the opportunity to examine the response of various cell types to EVs under various conditions, such as pH, hypoxia, presence of cytokines, etc. The present disclosure allows the preparation of an EV library in a multi-well format. In such an EV library, different EV sources and/or EV concentrations may be tested in parallel and compared to each other (see also Example 3 and Fig. 1A and B). Such an EV library is very useful for e.g. cell biologists, the medical community as well as scientists working in regenerative medicine. Using such an EV library may allow these communities to study the effect of EVs from e.g. the CSF on various cells of human patients as well as the differentiation of various stem cell types into differentiated cells. An EV library according to the present invention has the advantage that various EV sources and/or EV concentration can be tested in parallel and the surface can be shipped and/or stored without significant deterioration of the EV quality. This is because the EVs are immobilized on the surface and the surface no longer contains solutions. Furthermore, the EVs have ideally not been dried by lyophilization. In addition, the immobilized EVs according to the present invention also have the advantage that very small amounts of EVs can be used as basically no EVs are lost during immobilization. In contrast, during other methods such as buffer exchange or lyophilization, substantial amounts of EVs are lost. Omitting lyophilization saves material, is easier to implement and is faster. Moreover, the inventors have demonstrated Example 2 that EVs can be purified and immobilized from other sources such as NIH 3T3 cells (mouse fibroblast) neural stem cells, primary cultures of murine choroid plexus isolated from the lateral ventricle of 6-10 weeks old male mice, and primary cultures from Macaque choroid plexus. The inventors have demonstrated that purified EVs of various sources can be immobilized by e.g. drying.

The present disclosure is concerned with a sterile surface suitable for culturing cells, wherein the surface is coated with extracellular vesicles ('EVs') immobilized at one or more predetermined position(s), as defined in the claims. Furthermore, the present disclosure relates to a device for culturing cells comprising said sterile surface further comprising a removable covering to preserve sterility of said surface, as defined in the claims. Moreover, a kit comprising said device and further comprising a desiccant is disclosed, as defined in the claims. Additionally, a method of preparing said sterile surface is disclosed, comprising the steps of
a. purifying the EVs by ultracentrifugation to obtain a pellet comprising the EVs,
b. further purifying the EVs from said pellet by flotation in a density gradient to obtain a soluble fraction comprising the EVs,
c. depositing a portion of said fraction on the sterile surface at one or more predetermined position(s), and
d. letting said portion dry by evaporation,
whereby immobilized EVs on the sterile surface are obtained, as defined in the claims.

The disclosure also comprises a method of monitoring cell differentiation, apoptosis, cell migration, proliferation, transcriptome, proteome, and/or viability of cells ('recipient cells') comprising the steps of
a. adding said recipient cells to the surface as disclosed herein and
b. monitoring said cells by microscopy, qPCR, single cell sequencing, colometric detection in a plate reader, ChiP sequencing, mass spectrometry, DNA methylation and/or DNA acetylation analysis, as further defined in the claims.

### Detailed description of the invention

The present disclosure is concerned with a sterile surface suitable for culturing cells, wherein the surface is coated with extracellular vesicles ('EVs') immobilized at one or more predetermined position(s), as defined in the claims.

Extracellular vesicles ('EVs') are known in the art and are also termed microvesicles (size range of 100-1000nm) and exosomes (size range of 50- 150nm) which form by outward budding of plasma membrane or fusion of multivesicular endosomes with the plasma membrane, respectively.

It is contemplated that the EVs have been dried. It is preferred that the EVs have been dried by evaporation. Ideally, the EVs have been deposited in a solution and said solution is volatile and has therefore evaporated. Furthermore, the EVs have ideally not been dried by lyophilization. In general, lyophilization has the disadvantage that an object is first frozen so that ice crystals are formed and then the ice sublimates during lyophilization. However, the formation of ice crystals may deteriorate the quality of the EVs. Hence, the advantage of the present disclosure is that ice crystals do not form as the EVs are immobilized on the surface, by e.g. drying the EVs by evaporation, so that the surface is free of solutions before the surface is frozen. Furthermore, lyophilisation generates dried powders, which are not immobilized at a predetermined position. Once the EVs have been dried at the predetermined position(s), the EVs are immobilized to the surface. Immobilization by drying has the advantage that it is a very simple and cheap method of immobilization for which no additional devices are necessary. It is also contemplated that the EVs have not been immobilized by lyophilization. Specifically, cells may be cultured in said predetermined position(s). Said cells are 'recipient cells' as used herein. In other words, said cells are 'recipient cells' for the EVs (and its content), which are coating the sterile surface at said predetermined position(s). In particular, said recipient cells are selected from stem cells, induced pluripotent stem cells, early embryonic cells, cancer cells, nerve cells, muscle cells, blood cells, immune cells, epithelial cells, endothelial cells and bone cells. In some embodiments, the stem cells are selected from tissue-specific stem cells, mesenchymal stem cells or induced pluripotent stem cells. Tissue specific stem cells are also known as adult stem cells in the art. For example, tissue specific stem cells include hematopoietic stem cells, epidermal stem cells, epithelial stem cells and bronchoalveolar stem cells. Mesenchymal stem cells are isolated from stroma, i.e. the connective tissue that surrounds other tissues and organs. In other words, the recipient cells may be stem cells with the exception of human embryonic stem cells. Alternatively, said recipient cells may be stem cells, preferably wherein the stem cells may be selected from neural stem cells, mesenchymal stem cells, embryonic stem cells. In some embodiments, the embryonic stem cells are not human embryonic stem cells. It is especially preferred that the recipient cells are neural stem cells. Said recipient cells may also be from tissue explants, organoids, spheroids, xenografts or tumors. For example, recipient cells may be isolated from tissue explants, organoids, spheroids, xenografts or tumors. In particular, said recipient cells may have migrated from tissue explants, organoids, spheroids, xenografts or tumors. It is preferred that the recipient cells are fibroblasts.

Ideally, the surface is a cell culture plate. The skilled person is aware of cell culture plates, also called tissue culture plates, which are also commercially available. It is preferred that the cell culture plate has at least two or more predetermined positions. For example, a cover slip for microscopy may have two predetermined positions. It is even more preferred that the predetermined positions are wells. The skilled person is aware of cell culture plates having wells, such as multi-well plates. As known in the art, such multi-well plates have rows and columns of wells and such multi-well plates have, for example, six, 12, 24, 32, 48 or 96 wells. For example, the cell culture plate has at least four wells, even more preferably at least 12 wells, even more preferably at least 32 wells, even more preferably at least 48 wells, even more preferably at least 96 wells. The skilled person is aware of such multi-well plates, in particular 96 well plates. Such multi-well plates have the advantage that EVs of different sources may be immobilized in different wells and/or that EVs may be present at various concentrations in different wells. For example, EVs may be serially diluted along a part or a full row or column of the multi-well plate. Such a setup allows the preparation of an EV library in a multi-well format. In such a format, different EV sources and/or EV concentrations can be tested in parallel and compared to each other (see e.g. Fig. 1A and B). Ideally, EVs from one or more different human and/or animal cell lines are serially diluted in a multi-well plate to form an EV library. Such a library can then be stored at sub-zero degrees and/or shipped to a customer. In addition, custom-made libraries may be prepared. The response of cells to EVs could be scored in a multimode plate reader.

The surface may also be a cell culture plate. For example, the cell culture plate may be a petri dish having one predetermined position. Alternatively, the cell culture plate may be a specimen slide having one predetermined position. Such a specimen slide may be used for microscopy in any of the microscopy-based assays as disclosed herein.

The surface may be made out of any suitable material for culturing cells. Ideally, the surface is made out of a hydrocarbon polymer or glass. If the surface is made out of a hydrocarbon polymer, the hydrocarbon polymer is preferably polystyrene or polypropylene. The advantage of hydrocarbon polymers is that they can be formed in any desired shape and that many such shapes are commercially available such as multi-well cell culture plates. Ideally, the hydrocarbon polymer is polystyrene. If the surface is made out of glass, the glass is ideally borosilicate glass.

It is also contemplated that the surface is pre-coated with a protein. Protein coating of the surface has the advantage that the viability of the recipient cell, such as stem cells, can be improved by a protein coating. The inventors have tested pre-coated plates in Example 2 and found that pre-coating by proteins in combination with EVs may accelerate the differentiation process of neural stem cells. It is disclosed that the surface is pre-coated with a protein for accelerating differentiation. Ideally, the protein is selected from fibronectin, laminin, poly-L-lysine, poly D-lysine, gelatine, vitronectin, collagen or a mixture thereof. In particular, the protein may be fibronectin, laminin, or poly-L-lysine. It is more preferred that the EVs are on top of the protein. Ideally, the EVs are applied on top of the protein. In other words, the surface is first coated with a protein such as fibronectin and the EVs are applied on top of the protein so that the EVs are immobilized on top of the protein.

Ideally, the surface has two or more predetermined positions and the EVs are present in an amount which varies between the predetermined positions. The surface may have at most 3072 predetermined positions, preferably at most 1536 predetermined positions, more preferably at most 384 predetermined positions. The number of predetermined positions may be adjusted based on the experimental needs and aims. For example, the amount may vary by a dilution of the amount of EVs. The advantage of a varying amounts of EVs is that the effect of different amounts of EVs on recipient cells can be tested in parallel and that the potentially varying effect of the EVs can be directly compared in one experiment. This saves time and resources. Ideally, the amount varies by a serial dilution. In particular, the amount of EVs may vary by serial dilution between the predetermined positions. As known in the art, a serial dilution is a stepwise dilution of a substance. In the present case, the amount of EVs may be stepwise diluted along the two or more predetermined positions. Usually the dilution factor at each step is constant, resulting in a geometric progression of the concentration in a logarithmic fashion. For example, the dilution factor may be 2, so that the amount is halved in each step. It is preferred that the amount of EVs coating two or more predetermined position(s) varies by at most 1024-fold, more preferably at most 512-fold, more preferably at most 256-fold, more preferably at most 128-fold, even more preferably at most 64-fold, even more preferably at most 32-fold, even more preferably at most 16-fold, even more preferably at most 8-fold, even more preferably at most 4-fold, and most preferably at most 2-fold. For example, the amount varies by at most 1024-fold, a serial dilution of the amount may be present at the predetermined position(s) and the dilution factor may be 2. In said example, the original amount of EVs would be diluted at ten predetermined positions (2-fold, 4-fold, 8-fold, 16-fold, 32-fold, 64-fold, 128-fold, 256-fold, 512-fold, and 1024-fold) so that the effect on the recipient cells would be tested at eleven predetermined positions (original amount and ten dilutions). In case the amount of recipient cells is limited, the recipient cells may be added to some but not all serially diluted predetermined positions containing immobilized EVs. This example illustrates that the skilled person can choose the number of pre-determined positions based on the experimental needs and aims of the skilled person. This example also illustrates that the skilled person can select the dilution factor and the carrying amount of EVs at the predetermined positions independently.

It is further contemplated that each predetermined position has a deepening suitable to accommodate cells in solution. In other words, each predetermined position has a deepening suitable to accommodate the recipient cells in solution. For example, said deepening may be a container suitable to accommodate cells in solution. Said container suitable to accommodate cells in solution may be a petri dish or a well of a multi-well plate. Preferably said multi-well plate is a 6-well, a 12-well, a 24-well, a 48-well, a 96-well, a 128-well or a 384-well plate. It is preferred that said multi-well plate is a 96-well plate.

It is preferred that each predetermined position is coated with an amount ranging from 8×10⁶ to 3×10¹⁰ particles. The terms 'EVs', 'particles' and 'EV particles' are considered synonymous according to the present invention. It is also preferred that each predetermined position has been coated with an amount ranging from 8×10⁶ to 3×10¹⁰ particles. Methods to determine the amount of particles are known in the art. For example, the amount of EVs is measurable by a nanoparticle tracking analyzer. It is even more preferred that the amount ranges from 8×10⁷ to 3×10¹⁰ particles, more preferably from 8×10⁸ to 3×10¹⁰, more preferably from 1×10⁹ to 3×10¹⁰, more preferably from 2×10⁹ to 4×10¹⁰, more preferably from 4×10⁹ to 3×10¹⁰, more preferably from 6×10⁹ to 3×10¹⁰, and most preferably from 8×10⁹ to 3×10¹⁰.

Ideally, each predetermined position has been coated with a solution comprising EVs of a total protein concentration ranging from 5-500 µg/ml. The skilled person is aware of standard methods to determine the protein concentration of EVs in a solution. For example, the concentration is measurable by a bicinchoninic acid (BCA) protein assay, colorimetric detection or a Lowry protein assay. BCA protein assay kits and Lowry protein assay kits are commercially available, e.g. from ThermoFisher, which the skilled person is aware of. It is preferred that the concentration is measurable by BCA protein assay. In particular, each predetermined position may have been coated with a solution comprising EVs in a protein concentration ranging from 10-300 µg/ml, preferably 20-200 µg/ml, more preferably 30-180 µg/ml, more preferably 50-170 µg/ml, more preferably 60-140 µg/ml, more preferably 80-120 µg/ml, more preferably 90-110 µg/ml, and most preferably around 100 µg/ml. It is also contemplates that each predetermined position has been coated with a solution comprising EVs in a protein amount ranging from 1-1000 ng. The skilled person is aware that the protein amount can also be determined by standard methods in the art. For example, the concentration is measurable by a bicinchoninic acid (BCA) protein assay, colorimetric detection or a Lowry protein assay. It is preferred that the concentration is measurable by BCA protein assay. It is preferred that each predetermined position has been coated with a solution comprising EVs in a protein amount ranging from 5-500 ng, preferably 10-400 ng, more preferably 20-300 ng, more preferably 30-200 ng, more preferably 40-150 ng, more preferably 45-100 ng, more preferably 50-90 ng, more preferably 60-80 ng and most preferably around 75 ng.

It is also disclosed that the predetermined position(s) is/are coated entirely with EVs. It is preferred that the predetermined position(s) is/are (a) deepening(s), more preferably wherein the deepening(s) is/are (a) well(s) in a cell culture plate, more preferably wherein the cell culture plate is a multi-well plate. It is even more preferred that the multi-well plate is an EV library (see e.g. Fig. 1A and B). Coating the entire predetermined position with EVs has the particular advantage that the effect of EVs on recipient cells can be compared to e.g. the effect on recipient cells, which have not been exposed to EVs or to a smaller or higher amount of EVs under the same conditions. This is particularly advantageous to coat the entire predetermined positions, such as a well, in bulk-assays' such as qPCR, flow cytometry, colometric methods using a plate reader, ChIP-sequencing, DNA methylation analysis, DNA acetylation analysis, or mass spectrometry. The below Table 1 provides an exemplary overview of various methods of detection and said table also indicates for which methods the coating of the entire predetermined position is suitable:

**Table 1**

| **assay to monitor** | **Method of detection** | **predetermine d position(s) is/are coated *partially* with EVs** (useful for e.g. micro-drop assays) | **predetermined position(s) is/are coated *entirely* with EVs** (useful e.g. for bottom coated-bulk assay) |
|---|---|---|---|
| differentiation | microscopy | suitable | suitable |
| | qPCR | not suitable | suitable |
| | single cell seq | suitable | not suitable |
| viability | plate reader | not suitable | suitable |
| | flow cytometry | not suitable | suitable |
| | microscopy | suitable | suitable |
| apoptosis | plate reader | not suitable | suitable |
| | flow cytometry | not suitable | suitable |
| | microscopy | suitable | suitable |
| cell migration | microscopy | suitable | not suitable |
| proliferation | microscopy | suitable | suitable |
| | flow cytometry | no suitable | suitable |
| transcriptome/ proteome | qPCR | no suitable | suitable |
| | Mass Spec | not suitable | suitable |
| | ChIP-sequencing | suitable | suitable |
| development | DNA methylation pattern | suitable | suitable |
| | qPCR | suitable | suitable |

Table 1 also discloses methods of detection for which the coating of the partial predetermined position is suitable. For example, a partial coating is highly advantageous for analysing the effect of EVs when using microscopy as a method of detection. Then, the effect of the EVs at a coated area and an uncoated area can be directly compared. Alternatively, the effect of specific EVs can be compared to other EVs.

As also illustrated by Table 1, it is also contemplated that the predetermined position(s) is/are coated partially with EVs. In this case, it is preferred that the predetermined position(s) is/are a petri dish or a specimen slide. Alternatively, the predetermined position(s) is/are may be a well e.g. of a multi-well plate.

In case the predetermined position is coated partially, it is also disclosed that the EVs in each predetermined position originate from at least two different EV-producer cells. This has the advantage that the effect of different EVs on recipient cells can be compared side-by-side at one predetermined position. Ideally, the EVs in a predetermined position originate from two different EV-producer cells. Preferably, EVs in each predetermined position originate from two or more different EV-producer cells. More preferably, said EVs may be arranged in a format of a microarray. A "format of a microarray" is a spatial arrangement of molecules in a grid pattern as used for example in the remote field of DNA microarrays (see e.g. Sobek et al. [101]). Ideally, the EVs may originate from two or more different EV-producer cells and the EVs may be arranged in a grid format. Furthermore, the EVs may originate from one EV-producer cell type and the EVs may arranged in the format of a microarray. In the present case, the amount of EVs may be stepwise diluted in said microarray format, e.g. along a row or column. Hence, different concentrations of EVs originating of one EV-producer cell type may be tested. For example, an array spotter may produce said microarray format with EVs.

It is also disclosed that the surface according to the present disclosure is sterile. Of course, the skilled person is aware of the term 'sterile', which is commonly used in the field of cell biology. Ideally, the surface is sterile if no viable bacteria or other microorganisms are on said surface. In other words, the surface is sterile, if the surface is absent of viable life, such as bacteria, that has the potential to reproduce and spread. The sterility of the surface has the advantage that recipient cells can be cultures on said surface, ideally without antibiotic supplements such as Penicillin-Streptomycin.

Advantageously, the surface may be suitable for storage at temperature from -196 to -20 °C for at least one week. The advantage is achieved by having the EVs immobilized on the surface by air-drying. Thus, the EVs attach to the surface as the solvent in which they are dissolved evaporates. Hence, the surface is free from liquid. It is also contemplated that the EVs coating one or more predetermined positions have not been dried by lyophilization. This means that the EVs have not undergone a process termed freeze-drying in which water is removed from the sample at low temperature and under a vacuum. In air-drying the EVs are no longer in solution, so no harming water crystals can form upon freezing below 0°C and thus the surface can be stored below -20°C without significantly decreasing the effectiveness of the EVs. Ideally, the surface is suitable for storage for at least two weeks, more preferably at least three weeks, even more preferably at least four weeks, even more preferably at least five weeks, even more preferably at least six weeks, even more preferably at least two months; and at most two years. In contrast to the prior art, the EVs according to the present disclosure are not stored in solution but in an immobilized state on the surface. Hence, the storage at sub-zero conditions while essentially maintaining EV effectiveness is achieved as the solvent is absent when the surface containing the EVs is placed and maintained at sub-zero conditions. Ideally, the surface is storable at a temperature from -80 to -20°C, on dry ice or in liquid nitrogen. It is further preferred that the surface is storable at a temperature from -80 to -20°C or on dry ice, and preferably at a temperature around -80°C, at around -20°C, or on dry ice.

In case the surface is stored, it is contemplated that the EVs exhibit a suitable effectiveness after storage at -20°C or below for at least 24 hours compared to EVs not subjected to storage under such conditions but otherwise being treated identically. Additionally, the effectiveness of EVs is measurable with control EVs and control cells. For example, suitable recipient cells may be neural stem cells. Suitable EVs may be EVs originating from the choroid plexus or a choroid plexus cell line. Upon exposure to effective EVs, said neural stem cells differentiate into astrocytes, neurons and oligodendrocytes. It is preferred that the effectiveness of EVs is measurable by adding neural stem cells to the predetermined position(s) coated with EVs originating from a choroid plexus cell line and if astrocytes, neurons and oligodendrocytes are obtained, the EVs have suitable effectiveness. In general, said neural stem cells differentiate into a cell population comprising, ideally consisting, around 60-80 % astrocytes, 5-15% neurons and 10-30% oligodendrocytes. Ideally, said neural stem cells differentiate into a cell population comprising, ideally consisting, around 70 % astrocytes, 10% neurons and 20% oligodendrocytes. In case the surface is stored, it is also contemplated that the EVs may exhibit an effectiveness of at least 50% after storage at -20°C or below for at least 24 hours compared to EVs not subjected to storage under such conditions but otherwise being treated identically. It is also contemplated that the effectiveness may be measurable by differentiation of neural stem cells into astrocytes at the predetermined position(s) coated with EVs originating from choroid plexus cell line is observed. Differentiation into astrocytes may be assessed by the expression of GFAP and/or S100beta in said neural stem cells after a predetermined period by flow cytometry, qPCR and/or immunofluorescence. Specifically, the expression of GFAP and/or S100beta in said neural stem cells may be compared to EVs not subjected to storage under such conditions but otherwise being treated identically. Preferably, the cells exposed to the stored surface exhibit an expression of GFAP and/or S100beta of at least 60% compared to the surface containing the EVs not subjected to storage under such conditions but otherwise being treated identically, more preferably at least 70%, more preferably at least 80%, even more preferably at least 90%; compared to EVs not subjected to storage under such conditions but otherwise being treated identically.

Furthermore, the effectiveness may be measurable by differentiation to neurons, by adding neural stem cells to the predetermined position(s) coated with EVs originating from choroid plexus and by assessing the expression of tuj 1(beta 3 tubulin) in said added stem cells after a predetermined period by flow cytometry, qPCR and/or immunofluorescence. Ideally, the said added cells exhibit an expression of tuj 1(beta 3 tubulin) of at least 60%, more preferably at least 70%, more preferably at least 80%, even more preferably at least 90%. It is more preferred that the effectiveness is measurable by differentiation to neurons, by adding neural stem cells to the predetermined position(s) coated with EVs originating from choroid plexus and by assessing the expression of doublecortin and/or MAP2 in said added stem cells after a predetermined period by flow cytometry, qPCR and/or immunofluorescence. It is even more preferred that cells exhibit an expression of doublecortin and/or MAP2 of at least 60%, more preferably at least 70%, more preferably at least 80%, even more preferably at least 90%; compared to EVs not subjected to storage under such conditions but otherwise being treated identically.

The effectiveness of the EVs upon storage may also be measurable by differentiation to oligodendrocytes by adding neural stem cells to the predetermined position(s) coated with EVs originating from choroid plexus and by assessing the expression of Olig2 and/or MBP in said added stem cells after a predetermined period by flow cytometry, qPCR and/or immunofluorescence. It is preferred that said added stem cells exhibit an expression of Olig2 and/or MBP of at least 60%, more preferably at least 70%, more preferably at least 80%, even more preferably at least 90%; compared to EVs not subjected to storage under such conditions but otherwise being treated identically.

It is also disclosed that the surface may be storable under desiccating conditions. Furthermore, it is also disclosed that the surface may be stored under desiccating conditions. Such conditions can be obtained for example in a desiccating container at low humidity. Ideally, the dryness of the surface is protected by a desiccant such as silica gel.

The skilled person is aware that EVs are present in various different cell types and body fluids. It is also known that EVs are produced by cells and then secreted by the cells. Hence, the term 'EV producer cell' as used herein, refers to a cells producing and secreting EVs. In other words, EVs originate from EV-producer cells. For example, EV-producer cells may be an immortalized cell line, a cancer cell line, a stem cell, an induced pluripotent stem cell, a primary cell culture, or a primary tissue sample. In some embodiments, the stem cell is selected from tissue-specific stem cells, mesenchymal stem cells or induced pluripotent stem cells. For example, tissue specific stem cells include hematopoietic stem cells, epidermal stem cells, epithelial stem cells and bronchoalveolar stem cells. Mesenchymal stem cells are isolated from stroma, i.e. the connective tissue that surrounds other tissues and organs. In other words, the EV-producer cells may be stem cells with the exception of human embryonic stem cells. Alternatively, the immobilized EVs may be purified from a secretome contained in a body fluid. In case the EV-producer cells are an immortalized cell line, said immortalized cell line may be established from astrocytes, neuronal cells, Schwann cells, B cells, bone marrow mesenchymal stromal cells, oligodendrocytes, bronchial epithelial cells, cardiomyocytes, dendritic cells, embryonic kidney cells, endothelial cells, epithelial cells, leukocytes, macrophages, mesenchymal stem cells, osteoblasts, reticulocytes, or umbilical cord mesenchymal stem cells. Alternatively, the immortalized cell line may originate from a brain region, a pancreas or a lung. According to the present disclosure, it is preferred that the immortalized cell line originates from a brain region, more preferably wherein the brain region is the choroid plexus, and even more preferably wherein the immortalized cell line is rat Z310 choroid plexus-cell line. Experimental evidence on EVs from the rat Z310 choroid plexus-cell line and its effectiveness for the differentiation into neurons and astrocytes is provided in Example 1.

In case the EV-producer cells are a cancer cell line, the cancer cell line may be established from astrocytoma, brain cancer cells, breast cancer cells, bronchial epithelial cells, colorectal cancer cells, glioblastoma cells, neuroblastoma cells, kidney cancer cells, leukemia cells, lung cancer cells, melanoma cells, myeloid leukemia cells, ovarian cancer cells, pancreatic adenocarcinoma cells, or prostate cancer cells. In case the EV-producer cells are a primary cell culture, the primary cell culture may be established from adipocytes, B cells, bone marrow mesenchymal stromal cells, bronchial epithelial cells, cardiomyocytes, dendric cells, endothelial cells, epithelial cells, leukocytes, macrophages, mesenchymal stem cells, osteoblasts, platelets, reticulocytes, or umbilical cord mesenchymal stem cells. In case the EV-producer cells are a primary tissue sample, the primary tissue sample may be from brain, lung, liver, bladder, kidney, heart, stomach, intestine, or pancreas. It is also contemplated that the EV-producer cells are fibroblasts. Experimental evidence regarding fibroblasts as EV-producer cells is provided in Example 1. In particular, EVs purified from fibroblasts supernatant/ secretome are used as negative control for the differentiation of neural stem cells, as said EVs did not lead to a differentiation into neurons or astrocytes.It is also disclosed that the EVs may originate from a body fluid. For example, said body fluid may be cerebrospinal fluid (CSF), saliva, urine, serum or plasma. It is preferred that the body fluid is CSF.

The surface according to the present disclosure may be obtained by or are obtainable by
(i) purifying the EVs from EV-producer cells to obtain EVs in solution and
(ii) depositing and drying a portion of the solution of step (i) on said surface, thereby immobilizing the EVs on the surface.

The portion of the solution of step (ii) ideally has a volume which is reliably pipettable in an experimental setup and which easily dries e.g. by evaporation. For example, the portion of the solution of step (ii) has a volume of at least 1 nl, preferably at least 5 nl, more preferably at least 10 nl, more preferably at least 20 nl, more preferably at least 30 nl, more preferably at least 40 nl, more preferably at least 50 nl, more preferably at least 60 nl, more preferably at least 70 nl, more preferably at least 80 nl, more preferably at least 90 nl, more preferably at least 0.1 µl, more preferably at least 0.2 µl, more preferably at least 0.3 µl, even more preferably at least 0.4 µl, even more preferably at least 0.5 µl, even more preferably at least 0.6 µl, even more preferably at least 0.7 µl, even more preferably at least 0.8 µl, even more preferably at least 0.9 µl, and most preferably around 1 µl. In particular, the portion of the solution of step (ii) may have a volume of at most 50 µl, more preferably at most 40 µl, more preferably at most 30 µl, even more preferably at most 20 µl, even more preferably at most 10 µl, even more preferably at most 9 µl, even more preferably at most 8 µl, even more preferably at most 7 µl, even more preferably at most 6 µl, even more preferably at most 5 µl, even more preferably at most 4 µl, even more preferably at most 3 µl, even more preferably at most 2 µl, and most preferably around 1 µl. An amount between 0.1 µl and 10 µl has the advantage that it is easily pipettable by hand or by a robot and that it said volume also easily dries e.g. by evaporation. An amount between 1 nl and 1 µl, ideally around 0.1 µl, has the advantage that it is suitable for a microarray format. Preferably, the area covered in a microarray format may be from 50 to 500 µm², more preferably 100 to 300 µm² and most preferably around 150 µm². In a microarray format, around 50 recipient cells may respond to one predetermined area. It is also considered that the portion of the solution of step (ii) may be a fraction of a density gradient. The skilled person is aware of density gradients, which are for example used in the art to purify extracellular vesicles. It is preferred that the density gradient is an iodixanol gradient, a sucrose gradient or a colloidal silica particles coated with polyvinylpyrrolidone gradient. Colloidal silica particles coated with polyvinylpyrrolidone gradient are also known in the art under the trademark Percoll. It is especially preferred that the density gradient is an iodixanol gradient. The skilled person is aware of iodixanol gradients. The skilled person is provided with further guidance in Example 1 concerning iodixanol gradients. An iodixanol gradient also has the advantage that iodixanol has been developed as an X-ray contrast medium and has therefore been subjected to rigorous clinical testing. Iodixanol is non-ionic, non-toxic to cells and metabolically inert. For example, the portion of the solution of step (ii) comprises from 5% to 60 % (v/v) iodixanol in phosphate buffered saline (PBS), preferably 7.5% to 50%, and more preferably 10% to 40 %. Alternatively, the portion of the solution of step (ii) comprises from 5% to 70 % (v/v) sucrose in PBS, and preferably 10% to 60 %. Sucrose is also non-toxic to cells. It may be advantageous to remove the sucrose e.g. by dialysis or further ultracentrifugation steps, before depositing the portion in step (ii) in order to further improve the immobilization to the surface. The EVs may then be in PBS so that a portion of the solution of step (i) is deposited and dried on said surface in step (ii).

The portion of the solution of step (ii) may have a protein concentration ranging from 5-500 µg/ml, preferably 10-300 µg/ml, more preferably 20-200 µg/ml, more preferably 30-180 µg/ml, more preferably 50-170 µg/ml, more preferably 60-140 µg/ml, more preferably 80-120 µg/ml, more preferably 90-110 µg/ml, and most preferably around 100 µg/ml. Again, the skilled person is aware of methods for measuring protein concentrations in solution as also disclosed herein (see above).

It is especially preferred that the EVs are dried in step (ii) under laminar flow in sterile conditions. Furthermore, the EVs are ideally not dried in step (ii) by lyophilisation.

As known in the art, EVs can be purified by ultracentrifugation. In particular, EVs can be purified from medium in which the EVs-producer cells are cultured in step (i). In said case, the EVs have been secreted into the medium and the EVs are then purified from said medium in step (i). Alternatively, the EV-producer cells may be lysed first and then the EVs are purified to obtain EVs in solution in step (i). According to the present invention, the EVs may be purified in step (i) by ultracentrifugation followed by flotation in a density gradient, size exclusion chromatography, immune capture, flow cytometry or by combination thereof. If the EVs are purified by immune capture, the EVs may be purified by immune capture with magnetic beads. Furthermore, there are commercially available kits based on precipitation, ultrafiltration, size exclusion chromatography and affinity capture in order to obtain EVs. For example, commercial kits for purifying EVs by immune capture can be obtained from Thermofisher and/or abcam (ThermoFisher Catalog No: 10606D; Abcam CD63 Exosome Capture Beads; ab239686).

Ideally, the EVs are purified in step (i) by ultracentrifugation, ultrafiltration, density gradient, size exclusion chromatography, immune capture or combinations thereof. It is preferred that the EVs are purified in step (i) by ultracentrifugation followed by flotation in a density gradient, size exclusion chromatography or immune capture. It is more preferred that the EVs are purified in step (i) by immune capture and/or size exclusion chromatography. It is even more preferred that the EVs are purified in step (i) by ultracentrifugation followed by flotation in a density gradient, such as an iodixanol gradient, as demonstrated in Example 1.

It is also contemplated that the EVs are purified in step (i) by ultracentrifugation. For example, said differential centrifugation may be carried out in at least 2 steps above 1000× g and wherein the last step is the ultracentrifugation may be carried out from 90 000 × g to 200 000 × g so that the EVs are found in the pellet after the last centrifugation step. After the last ultracentrifugation step, it is contemplated that the EV pellet is resuspended. It is especially preferred that the last step of the ultracentrifugation is carried out at around 100 000 × g.

It is also ideal when the EVs are purified in step (i) by flotation in a density gradient. Preferred density gradients are an iodixanol gradient, a sucrose gradient or a colloidal silica particles coated with polyvinylpyrrolidone gradient. An iodixanol gradient is most preferred in step (i). An iodixanol gradient is advantageous as described herein.

In step (i), the EVs may be purified by flotation in an iodixanol gradient from 5% (v/v) to 60% (v/v) iodixanol, preferably wherein the iodixanol gradient from 10% (v/v) to 40% (v/v) iodixanol is and more preferably wherein the iodixanol gradient from 10% (v/v) to 40% (v/v) iodixanol is in PBS. The skilled person is provided with further guidance on iodixanol gradient in Example 1 herein. Alternatively, the density gradient may be a sucrose gradient, as disclosed herein. It may be advantageous to remove the sucrose after step (i) e.g. by dialysis or further ultracentrifugation steps, before depositing the portion in step (ii) in order to further improve the immobilization to the surface. The EVs may then be in PBS so that a portion of the solution of step (i) is deposited and dried on said surface in step (ii). Preferably, the portion of the solution of step (i) is not dried by lyophilisation. Furthermore, the EVs are purified in step (i) from EV-producer which are further defined above. As also further explained above, the EVs may also be purified in step (i) from a body fluid, preferably wherein the body fluid is CSF, saliva, urine, serum or plasma. CSF is the most preferred body fluid according to the present disclosure. Ideally, the EVs are purified from EV-producer cells supernatant or secretome in step (i).

In another aspect, a device for culturing cells comprising the surface as described herein further comprising a removable covering to preserve sterility of said surface is disclosed. Ideally, said covering is a foil or lid. It is preferred that said covering is a lid. For example, said device may be a multi-well plate covered with a foil or a lid. In case of a multi-well plate, the device may be an EV library in a multi-well format. In such a formal different EV sources and/or EV concentrations can be tested in parallel and compared to each other (see e.g. Fig. 1A and B). Alternatively, the device may be a petri dish or microscope slide with a removable lid. It is preferred that the removable lid is sealed to the petri dish or microscope slide. In order to preserve sterility of the surface, it is preferred that the removable lid is sealed with tape.

The device may also be a microscope slide. Ideally, the removable lid is sealed to the microscope slide to protect dryness. Furthermore, it is preferred that the removable lid is sealed with tape. In turn, once the cells are added to the surface as disclosed herein, the lid may prevent evaporation of the solution containing the cells.

In case the device is a multi-well plate, said multi-well plate may be a 6-well, a 12-well, a 24-well, a 48-well, a 96-well, a 128-well or a 384-well plate. In order to preserve sterility and protect the dryness of the surface, it is preferred that the removable lid is sealed to the multi-well plate. It is even more preferred that the removable lid is sealed with tape. In a multi-well plate, it is preferred that the entire bottom of each well are the predetermined positions. As known in the art, multi-well plates have rows and columns of wells. It is preferred that the EVs from two or more different EV-producing cells are coating different rows or columns. Preferably, EVs from two or more different sources are serially diluted and wherein said different sources are selected from a human cell line, and/or an animal cell lines, a plant cell, a protozoan and/or a body fluid. It is also preferred that the EV amount has been serially diluted along a part of the row or the column of the multi-well plate. For example, the part of the multi-well plate may be half the row or half the column. Alternatively, the EV amount may have been serially diluted along an entire row or entire column of the multi-well plate. Alternatively, the EV amount may have been serially diluted along two, three, four, five, six, seven, eight, nine ten or eleven wells along a row or column of the multi-well plate. The skilled person will adjust the number of serial dilutions depending on the experiment. Ideally, EVs from one or more different sources are serially diluted in the multi-well plate to form an EV library. Said different sources may be selected a human cell line, and/or an animal cell lines, a plant cell, a protozoan and/or a body fluid Said library can then be stored at sub-zero degrees and/or shipped to a customer (see e.g. Fig. 1A and B).

In another aspect, a kit comprising the device as disclosed herein and further comprising a desiccant is disclosed. Ideally, the desiccant is silica gel. The advantage of such a kit is that it may be easily shipped to customers as the kit comprising the device, which in turn comprises the surface coated with EVs is ideally storable and the EVs are immobilised to the surface. Hence, the surface is free of water-based solutions and the EVs have not undergone lyophilisation. In order to maintain the dryness of the surface the kit comprises a desiccant such as silica gel.

In another aspect, the disclosure also relates to a method of preparing a sterile surface, comprising the steps of
(a) purifying the EVs by ultracentrifugation to obtain a pellet comprising the EVs,
(b) further purifying the EVs from said pellet by flotation in a density gradient to obtain a soluble fraction comprising the EVs,
(c) depositing a portion of said fraction on the sterile surface at one or more predetermined position(s), and
(d) letting said portion dry by evaporation,
whereby immobilized EVs on the sterile surface are obtained, as defined in the claims.

The EVs are purified by ultracentrifugation in step (a). The skilled person is aware of ultracentrifugation for purifying EVs in general. Preferably, the EVs are purified by centrifugation followed by ultracentrifugation in step (a). Even more preferably, the EVs are purified by differential centrifugation in step (a). The term "differential centrifugation" is known in the art. Specifically, "differential centrifugation" as used herein comprises two or more centrifugation steps, wherein the centrifugation speed is stepwise increased per step and wherein at least the last step is an ultracentrifugation step. Example 1 provides further assistance and details for especially preferred embodiments of ultracentrifugation. The differential centrifugation of step (a) may be carried out in at least two steps above 1000× g and wherein the last step is carried out from 50 000 × g to 200 000 × g so that the EVs are concentrated in the pellet after the last centrifugation step. Ideally, the last step of the ultracentrifugation is carried out at around 100 000 × g. The skilled person is able to determine a suitable duration for an ultracentrifugation step. For example, an indication that an ultracentrifugation step is complete is when a visible pellet has formed. After completion of step (a), the EVs may be extracted from the pellet.

Furthermore, it is especially preferred that the pellet of step (a) is resuspended in 30-50% iodixanol (v/v), preferably 35-45% iodixanol (v/v) and most preferably around 40% iodixanol (v/v). The particular advantages of iodixanol gradients described above apply equally to the method as described herein. It is also contemplates that the pellet of step (a) is resuspended and bottom-loaded or top-loaded in the density gradient of step (b). According to the present invention it is preferred that the pellet of step (a) is resuspended and bottom-loaded. The skilled person is provided with further guidance regarding the bottom-loading in Example 1 herein.

The density gradient of step (b) may an iodixanol gradient, a sucrose gradient or a colloidal silica particles coated with polyvinylpyrrolidone gradient. It is particularly preferred that the density gradient of step (b) is an iodixanol gradient. This is because iodixanol is for example non-toxic to cells, as further detailed herein. In case a sucrose gradient is used, it may be advantageous to remove the sucrose e.g. by dialysis or further ultracentrifugation steps, after step (b) in order to further improve the immobilization to the surface. The EVs may then be in PBS so that a portion of the solution of step (b) may be deposited on said surface in step (c).

It is also contemplated that the density gradient of step (b) is a discontinuous density gradient. The skilled person is aware of discontinuous density gradients. For example, the discontinuous density gradient may range from 5% to 50% iodixanol. Alternatively, the gradient may range from 10% to 40 % iodixanol.

With regard to the speed of ultracentrifugation of step (b), the skilled person is aware of suitable ultracentrifugation speeds and durations (amounts of time) in general. Step (b) of the method is completed when a suitable EV marker can be identified in the soluble fraction comprising the EVs. For example, the soluble fraction of step (b) comprising the EVs is obtained by identifying a suitable EV marker in one or more fraction(s). The skilled person is aware of suitable markers for EVs. It is particularly preferred that said suitable EV marker is selected from a one or more of a tetraspanin, tsg101, fibronectin 1, flotillin 1, and/or flotillin 2. For example, the tetraspanin is selected from CD63, CD81 or CD9. Further assistance regarding the suitable exemplary EV marker are provided in the prior art such as references [73] and [74]. Particular guidance is also provided in Example 1 herein. In particular, the density gradient of step (b) may be established by centrifugation at 120 000 × g to 200 000 × g. It is preferred that the density gradient is established by a spin at 140 000 × g to 190 000 × g, more preferably from 170 000 × g to 185 000 × g and even more preferably around 180 000 × g. The skilled person can generate a density gradient of step (b) for a suitable amount of time. The skilled person is aware that the skilled person can test a suitable amount of time by taking a sample of each fraction of the, e.g. discontinuous, density gradient and then detect the distribution of proteins along the density gradient. Exemplary amount of time for step (b) are from 10 to 36 hours, preferably 12 to 32 hours, more preferably 14 to 28 hours, even more preferably 15 to 24 hours, even more preferably 16 to 22 hours, even more preferably 17 to 20 hours, and most preferably around 19 hours. It is also disclosed that the density gradient of step (b) may be generated in a swinging bucket rotor, such as a TH-660 rotor. The skilled person is also provided with further guidance on nature of a density gradient in Example 1.

After completion of step (b), a portion of the fraction of step (b) is deposited on the sterile surface in step (c).

In step (c), the portion of step (c) is ideally deposited on the sterile surface by pipetting. Depending on the experiment, the EV concentration in the fraction, the skilled person determines a suitable portion. The portions should ideally be reliably pipettable by hand or by a robot and need to be suitable for evaporation. Alternatively, the portions are suitable for a microarray format. Such portions are ideally pipetted by a spotting device. Specifically, an amount between 0.1 µl and 10 µl has the advantage that it is easily pipettable by hand or by a robot and that it said volume also easily dries e.g. by evaporation. An amount between 1 nl and 1 µl has the advantage that it is suitable for a microarray format and such portions also easily dry. Exemplary suitable portions in step (c) may have a volume of at least 1 nl, preferably at least 5 nl, more preferably at least 10 nl, more preferably at least 20 nl, more preferably at least 30 nl, more preferably at least 40 nl, more preferably at least 50 nl, more preferably at least 60 nl, more preferably at least 70 nl, more preferably at least 80 nl, more preferably at least 90 nl, more preferably at least 0.1µl, more preferably at least 0.2 µl, more preferably at least 0.3 µl, even more preferably at least 0.4 µl, even more preferably at least 0.5 µl, even more preferably at least 0.6 µl, even more preferably at least 0.7 µl, even more preferably at least 0.8 µl, even more preferably at least 0.9 µl, and most preferably around 1 µl. Further exemplary portions may have a volume of at most 50 µl, more preferably at most 40 µl, more preferably at most 30 µl, even more preferably at most 20 µl, even more preferably at most 10 µl, even more preferably at most 9 µl, even more preferably at most 8 µl, even more preferably at most 7 µl, even more preferably at most 6 µl, even more preferably at most 5 µl, even more preferably at most 4 µl, even more preferably at most 3 µl, even more preferably at most 2 µl, and most preferably around 1 µl.

In the step (d), the skilled person is able to determine a suitable amount of time for drying of the portion of the fraction. The skilled person is able to determine the portion is dried on the surface by standard methods. One standard method is visual inspection. It is contemplates that the completion of step (d) is assessable by visual inspection. For example, wherein step (d) is completed when the portion of the fraction of step (d) does not form ice crystals upon freezing. Alternatively, the completion of step (d) may be assessable by measuring the refractive index, liquid surface tension and/or optical tensiometer. In this case, it is preferred that the dryness is assessed by measuring the refractive index. The skilled person is aware that the refractive index of an optical medium, such as the surface as disclosed herein, is a dimensionless number that gives the indication of the light bending ability of that medium. The refractive index may, for example, be determined at a reference of λ=589 nm. Ideally, the refractive index at the predetermined position(s) remains constant after completion of step (d).

For example, the portion may be dried over a time period from 30 seconds to 60 minutes, preferably from 1 minute to 45 minutes, more preferably from 3 minutes to 30 minutes, more preferably from 5 minutes to 20 minutes. The portion is ideally not dried by lyophilisation.

It has been shown in the art that extracellular vesicles lose their effectiveness in solution a few days. For example Görgens et al (2022) [102] shows that EVs stored in PBS show drastically reduced recovery after days, e.g. 30% within a week. Preferably, the method steps (a) to (d) are carried out within 7 days, more preferably within 5 days, more preferably within 4 days, more preferably within 3 days, and most preferably within 2 days. It is preferred that the method steps (b) to (d) are carried out within 5 days, more preferably within 4 days, more preferably within 3 days, more preferably within 2 days, and most preferably 24 hours. Ideally, step (b) is performed immediately after the completion of step (a). Preferably, step (c) is performed immediately after the completion of step (b). Additionally, the method steps (a), (b), (c) and/or (d) may be performed at 4 °C.

The surface obtained by the method is suitable for culturing cells, as defined herein. Said cells may be further defined as 'recipient cells'. Exemplary 'recipient cells' are disclosed herein and said recipient cells are equally suitable to the method. In certain embodiments, the surface is further defined in as disclosed herein. It is also preferred that the EVs are further defined as disclosed herein. Ideally, the predetermined position(s) are further defined as disclosed herein.

In a further aspect, the present disclosure also relates to *in vitro* assays, which can be performed based on the invention as disclosed herein. The advantage of the surface containing EVs, as disclosed herein, is that various in vitro assays can be performed in order to study e.g. cell differentiation, responsiveness, apoptosis, viability, cell migration and proliferation in response to, e.g. various types and amounts of EVs. The advantage of the *in vitro* methods as disclosed below is that cells exposed to the EVs may be compared to (i) cells not exposed to EVs, (ii) cells exposed to a different amount of the same EVs and/or (iii) cells exposed to EVs from a different source. **Table 1** provides an overview of exemplary suitable assays and methods. In addition, some particularly preferred assays are described below.

The present disclosure also relates to a method of monitoring cell differentiation, apoptosis, cell migration and/or proliferation is comprising the steps of
a) adding cells to the surface as disclosed herein and
b) monitoring said cells by microscopy.

Ideally, the cells are monitored in real time. For example, the cells may be monitored by light microscopy or fluorescent microscopy. In this respect, the cells may be fixed and stained with suitable immunofluorescent markers. In particular, cellular networks may form by the recipient cells in response to the EVs. Hence, images of cellular network formation may be taken.

It is contemplated that cell migration is monitored. Ideally, the migration towards or away from the EVs is monitored. Such a setup has the advantage that the signalling cues of the EVs can be directly studied and compared to cells not being exposed to EVs. It is especially preferred that the migration towards the EVs is monitored. This is particularly relevant when the EVs contain signalling molecules promoting cell migration. For example, the migration of cells from tissue, explants, organoids, embryoid bodies, and/or spheroid cancer cells may be monitored. Monitoring of cell proliferation may be assessed for cancer diagnosis, for analysis of cell regeneration, for activation of the immune system and for analysis of a vaccine response. Said assay for monitoring cell differentiation, apoptosis, cell migration and/or proliferation may be performed as a high-throughput automated evaluation of the live or fixed cells are performed by microscopy to assed cellular network formation, cell differentiation, migration, wound healing. Monitoring cell differentiation may also be useful for assessing the effectiveness of stem cell therapy, tissue regeneration, and tissue development. Monitoring cell migration may also be useful for cancer research and for assessing the effectiveness of tissue regeneration.

Furthermore, a method of monitoring cell differentiation, transcriptome, proteome and/or cell development comprising the steps of
a) adding cells to the surface as disclosed herein
b) monitoring said cells by qPCR is contemplated.

The skilled person is aware of the method qPCR. The skilled person is also aware the suitable primers for qPCR markers are commercially available or can be designed and the sequence is commercially available or on public databases. Ideally, the qPCR is carried out using one or more suitable markers. Exemplary suitable marker includes gfap, klf4, nanog, nestin, sox2, oct4, pax6 and/or vimentin.

Additionally, a method of monitoring cell differentiation comprising the steps of
a) adding cells to the surface as disclosed herein
b) monitoring said cells by single cell sequencing is contemplated.

The skilled person is aware of single cell sequencing and is able to carry out said method.

A method of monitoring cell viability and/or apoptosis is also disclosed. Said method comprises the steps of
a) adding cells to the surface as disclosed herein and
b) monitoring said cells by colormetric detection in a plate reader.

The skilled person is aware of colometric detection assays. The reagents for such colometric assays are commercially available. For example, cell viability and/or apoptosis is detected by a caspase-3 or MTT assay. The assay reagents are for example commercially available from abcam or Sigma. Alternatively, cell viability may be detected by an ATP-luciferase assay. Reagents of an ATP-luciferase assay are also commercially available, such as from Abcam or ThermoFisher. In particular, cell viability and/or apoptosis may be detected by an annexin XII based polarity sensitive probe. Such probes are for example commercially available from Novusbio. Monitoring of apoptosis may be useful in cancer research, immunology and infectiology.

Moreover, a method of monitoring cell viability, apoptosis and/or proliferation is also disclosed. Said method comprises the steps of
a) adding cells to the surface as disclosed herein and
b) monitoring said cells by flow cytometry.

The skilled person is aware of flow cytometry. Furthermore, the skilled person is aware of suitable flow cytometry markers, which may be used to detect cell viability, apoptosis and/or proliferation.

In addition, the present disclosure also relates to a method of monitoring a proteome of cells comprising the steps of
a) adding cells to the surface as disclosed herein and
b) monitoring said cells by mass spectrometry.

The skilled person is aware of the technique mass spectrometry. The skilled person is provided with further guidance on mass spectrometry in Example 1 herein.

A method of monitoring protein-nucleic acid interactions comprising the steps of
a) adding cells to the surface as disclosed herein and
b) monitoring said cells by ChiP-sequencing is also contemplated.

The skilled person is aware of ChIP-sequencing and is able to carry out said method.

The present disclosure also relates to a method of monitoring cell development comprising the steps of
a) adding cells to the surface as disclosed herein and
b) monitoring said cells by analysing the DNA methylation and/or DNA acetylation pattern.

The skilled person is able to analyse the DNA methylation and/or DNA acetylation pattern based on methods known in the art.

In light of the surface as disclosed herein, the present disclosure also relates to a use of the surface as disclosed herein for monitoring cell differentiation, cell proliferation, cell migration, viability, and/or apoptosis. The skilled person may for example use said surface in any of the methods as disclosed in Table 1. Furthermore, the surface as disclose herein may be used as a research tool. Furthermore, the surface as disclosed herein may be used as a diagnostic *in vitro* assay.

The invention is further described by the following embodiments:
1. A sterile surface suitable for culturing cells, wherein the surface is coated with extracellular vesicles ('EVs') immobilized at one or more predetermined position(s).
2. The surface of embodiment 1, wherein cells are cultured in said predetermined position(s) ('recipient cells'), preferably wherein said recipient cells are selected from stem cells, induced pluripotent stem cells, early embryonic cells, cancer cells, nerve cells, muscle cells, blood cells, immune cells, epithelial cells, endothelial cells and bone cells.
3. The surface of embodiment 2, wherein said recipient cells are stem cells, preferably wherein the stem cells are selected from neural stem cells, mesenchymal stem cells, and embryonic stem cells, and more preferably wherein cells are neural stem cells.
4. The surface of embodiment 2, wherein said recipient cells are tissue explants, organoids, spheroids, xenografts or tumors, and preferably wherein the recipient cells are isolated from tissue explants, organoids, spheroids, xenografts or tumors.
5. The surface of embodiment 2, wherein said recipient cells are fibroblasts.
6. The surface of any of the preceding embodiments, wherein the surface is a cell culture plate, preferably wherein the cell culture plate has at least two or more predetermined positions, more preferably wherein the predetermined positions are wells, even more preferably wherein the cell culture plate has at least four wells, even more preferably at least 12 wells, even more preferably at least 32 wells, even more preferably at least 48 wells, even more preferably at least 96 wells, and most preferably 96 wells.
7. The surface of any of the preceding embodiments, wherein the surface is a cell culture plate, preferably wherein the cell culture plate is a petri dish having one predetermined position or a specimen slide having one predetermined position.
8. The surface of any of the preceding embodiments, wherein the surface is made out of a hydrocarbon polymer or glass.
9. The surface of embodiment 8, wherein the hydrocarbon polymer is polystyrene or polypropylene, preferably polystyrene.
10. The surface of embodiment 8, wherein the glass is borosilicate glass.
11. The surface of any of the preceding embodiments, wherein the surface is pre-coated with a protein, preferably wherein the protein is fibronectin, laminin, poly-L-lysine, poly D-lysine, gelatine, vitronectin, collagen or a mixture thereof, more preferably wherein the protein is fibronectin, laminin, or poly-L-lysine.
12. The surface of embodiment 11, wherein the EVs are on top of the protein.
13. The surface of any of the preceding embodiments, wherein the surface has two or more predetermined positions and wherein the EVs are present in an amount which varies between the predetermined positions, preferably wherein the amount varies by at most 1024-fold, preferably at most 512-fold, more preferably at most 256-fold, more preferably at most 128-fold, even more preferably at most 64-fold, even more preferably at most 32-fold, even more preferably at most 16-fold, even more preferably at most 8-fold, even more preferably at most 4-fold, and most preferably at most 2-fold; in particular wherein the amount of EVs varies by serial dilution between the predetermined positions.
14. The surface of any of the preceding embodiments, wherein the surface is sterile if no viable bacteria or other microorganisms are on said surface.
15. The surface of any of the preceding embodiments, wherein the surface is sterile if no bacteria or other microorganisms are on said surface.
16. The surface of any of the preceding embodiments, wherein the surface is suitable for storage at temperature from -196 to -20 °C for at least one week, preferably at least two weeks, more preferably at least three weeks, even more preferably at least four weeks, even more preferably at least five weeks, even more preferably at least six weeks, even more preferably at least two months; and at most two years.
17. The surface of any of the preceding embodiments, wherein the surface is storable at a temperature from -80 to -20 °C, on dry ice or in liquid nitrogen, preferably at a temperature from -80 to -20 °C or on dry ice, and more preferably at a temperature around -80 °C, at around -20 °C, or on dry ice.
18. The surface of any of the preceding embodiments, wherein the surface is storable under desiccating conditions, preferably wherein the surface is stored under desiccating conditions.
19. The surface of any of the preceding embodiments, wherein the EVs exhibit a suitable effectiveness after storage at -20°C or below for at least 24 hours compared to EVs not subjected to storage under such conditions but otherwise being treated identically, wherein the effectiveness of EVs is measurable by adding neural stem cells to the predetermined position(s) coated with EVs originating from a choroid plexus cell line and if astrocytes, neurons and oligodendrocytes are obtained, the EVs have suitable effectiveness.
20. The surface of any of the preceding embodiments, wherein the EVs exhibit an effectiveness of at least 50% after storage at -20°C or below for at least 24 hours compared to EVs not subjected to storage under such conditions but otherwise being treated identically,
   preferably wherein the effectiveness is measurable by differentiation to astrocytes, by adding neural stem cells to the predetermined position(s) coated with EVs originating from choroid plexus cell line and by assessing the expression of GFAP and/or S100beta in said added stem cells after a predetermined period by flow cytometry, qPCR and/or immunofluorescence.
   more preferably wherein the cells exhibit an expression of GFAP and/or S100beta of at least 60%, more preferably at least 70%, more preferably at least 80%, even more preferably at least 90%.
21. The surface of embodiment 20, wherein the effectiveness is measurable by differentiation to neurons, by adding neural stem cells to the predetermined position(s) coated with EVs originating from choroid plexus and by assessing the expression of tuj 1(beta 3 tubulin) in said added stem cells after a predetermined period by flow cytometry, qPCR, and/or immunofluorescence.
   more preferably wherein the cells exhibit an expression of tuj 1(beta 3 tubulin) of at least 60%, more preferably at least 70%, more preferably at least 80%, even more preferably at least 90%.
22. The surface of embodiment 20, wherein the effectiveness is measurable by differentiation to neurons, by adding neural stem cells to the predetermined position(s) coated with EVs originating from choroid plexus and by assessing the expression of doublecortin and/or MAP2 in said added stem cells after a predetermined period by flow cytometry, qPCR, and/or immunofluorescence.
   more preferably wherein the cells exhibit an expression of doublecortin and/or MAP2 of at least 60%, more preferably at least 70%, more preferably at least 80%, even more preferably at least 90%.
23. The surface of embodiment 20, wherein the effectiveness is measurable by differentiation to oligodendrocytes by adding neural stem cells to the predetermined position(s) coated with EVs originating from choroid plexus and by assessing the expression of Olig2 and/or MBP in said added stem cells after a predetermined period by flow cytometry, qPCR, and/or immunofluorescence.
   more preferably wherein the cells exhibit an expression of Olig2 and/or MBP of at least 60%, more preferably at least 70%, more preferably at least 80%, even more preferably at least 90%.
24. The surface of any of the preceding embodiments, wherein the EVs originate from EV-producer cells and wherein the EV-producer cells are an immortalized cell line, a cancer cell line, a stem cell, an induced pluripotent stem cell, a primary cell culture, or a cell of a primary tissue sample; or
   wherein the immobilized EVs are purified from a secretome contained in a body fluid.
25. The surface of embodiment 23, wherein the immortalized cell line is established from astrocytes, neuronal cells, Schwann cells, B cells, bone marrow mesenchymal stromal cells, oligodendrocytes, bronchial epithelial cells, cardiomyocytes, dendritic cells, embryonic kidney cells, endothelial cells, epithelial cells, leukocytes, macrophages, mesenchymal stem cells, osteoblasts, reticulocytes, or umbilical cord mesenchymal stem cells; and/or
   wherein the immortalized cell line originates from a brain region, a pancreas or a lung, preferably the brain region, more preferably wherein the brain region is the choroid plexus, and even more preferably wherein the immortalized cell line is rat Z310 choroid plexus-cell line.
26. The surface of embodiment 24, wherein the cancer cell line is established from astrocytoma, brain cancer cells, breast cancer cells, bronchial epithelial cells, colorectal cancer cells, glioblastoma cells, neuroblastoma cells, kidney cancer cells, leukemia cells, lung cancer cells, melanoma cells, myeloid leukemia cells, ovarian cancer cells, pancreatic adenocarcinoma cells, or prostate cancer cells.
27. The surface of embodiment 24, wherein the primary cell culture is established from adipocytes, B cells, bone marrow mesenchymal stromal cells, bronchial epithelial cells, cardiomyocytes, dendric cells, endothelial cells, epithelial cells, leukocytes, macrophages, mesenchymal stem cells, osteoblasts, platelets, reticulocytes, or umbilical cord mesenchymal stem cells.
28. The surface of embodiment 24, wherein the primary tissue sample is from brain, lung, liver, bladder, kidney, heart, stomach, intestine, or pancreas.
29. The surface of embodiment 24, wherein the EV-producer cells are fibroblasts.
30. The surface of any of embodiments 1-23, wherein the EVs originate from a body fluid, preferably wherein the body fluid is CSF, saliva, urine, serum or plasma, preferably CSF.
31. The surface of any of the preceding embodiments, wherein the EVs have been dried, preferably wherein the EVs have been dried by evaporation, more preferably wherein the EVs have not been dried by lyophilisation.
32. The surface of any of the preceding embodiments, wherein each predetermined position has a deepening suitable to accommodate cells in solution, preferably wherein the deepening is a container suitable to accommodate cells in solution.
33. The surface of any of the preceding embodiments, wherein the predetermined position(s) is/are coated entirely with EVs, preferably wherein the predetermined position(s) is/are (a) deepening(s), more preferably wherein the deepening(s) is/are (a) well(s) in a cell culture plate, more preferably wherein the cell culture plate is a multi-well plate, and even more preferably wherein the multi-well plate is an EV library.
34. The surface of any of the preceding embodiments, wherein the predetermined position(s) is/are coated partially with EVs, preferably wherein the predetermined position(s) is/are a petri dish or a specimen slide.
35. The surface of embodiment 34, wherein the EVs in each predetermined position originate from at least two different EV-producer cells, preferably wherein the EVs in each predetermined position originate from two different EV-producer cells.
36. The surface of any of the preceding embodiments, wherein each predetermined position has been coated with a solution comprising EVs in an amount ranging from 8×10⁶ to 3×10¹⁰, preferably from 8×10⁷ to 3×10¹⁰, more preferably from 8×10⁸ to 3×10¹⁰, more preferably from 1×10⁹ to 3×10¹⁰, more preferably from 2×10⁹ to 4×10¹⁰, more preferably from 4×10⁹ to 3×10¹⁰, more preferably from 6×10⁹ to 3×10¹⁰, and more preferably from 8×10⁹ to 3×10¹⁰; measurable by a nanoparticle tracking analyzer.
37. The surface of any of the preceding embodiments, wherein each predetermined position has been coated with a solution comprising EVs in a protein concentration ranging from 5-500 µg/ml, preferably 10-300 µg/ml, more preferably 20-200 µg/ml, more preferably 30-180 µg/ml, more preferably 50-170 µg/ml, more preferably 60-140 µg/ml, more preferably 80-120 µg/ml, more preferably 90-110 µg/ml, and most preferably around 100 µg/ml; measurable by a bicinchoninic acid (BCA) protein assay, colorimetric detection or a Lowry protein assay, preferably measurable by BCA protein assay.
38. The surface of any of the preceding embodiments, wherein each predetermined position has been coated with a solution comprising EVs in a protein amount ranging from 1-1000 ng, preferably 5-500 ng, more preferably 10-400 ng, more preferably 20-300 ng, more preferably 30-200 ng, more preferably 40-150 ng, more preferably 45-100 ng, more preferably 50-90 ng, more preferably 60-80 ng and most preferably around 75 ng; measurable by a bicinchoninic acid (BCA) protein assay, colorimetric detection or a Lowry protein assay, preferably measurable by BCA protein assay.
39. The surface of any of the preceding embodiments obtained by or are obtainable by
   (i) purifying the EVs from EV-producer cells to obtain EVs in solution and
   (ii) depositing and drying a portion of the solution of step (i) on said surface, thereby immobilizing the EVs on said surface.
40. The surface of embodiment 39, wherein the portion of the solution of step (ii) has a volume of at least 0.1 µl, more preferably at least 0.2 µl, more preferably at least 0.3 µl, even more preferably at least 0.4 µl, even more preferably at least 0.5 µl, even more preferably at least 0.6 µl, even more preferably at least 0.7 µl, even more preferably at least 0.8 µl, even more preferably at least 0.9 µl, and most preferably around 1 µl.
41. The surface of embodiments 39 or 40, wherein the portion of the solution of step (ii) has a volume of at most 50 µl, more preferably at most 40 µl, more preferably at most 30 µl, even more preferably at most 20 µl, even more preferably at most 10 µl, even more preferably at most 9 µl, even more preferably at most 8 µl, even more preferably at most 7 µl, even more preferably at most 6 µl, even more preferably at most 5 µl, even more preferably at most 4 µl, even more preferably at most 3 µl, even more preferably at most 2 µl, and most preferably around 1 µl.
42. The surface of any of embodiments 39-41, wherein the portion of the solution of step (ii) is a fraction of a density gradient, preferably wherein the density gradient is an iodixanol gradient, a sucrose gradient or a colloidal silica particles coated with polyvinylpyrrolidone gradient, preferably wherein the density gradient is an iodixanol gradient.
43. The surface of any of embodiments 39-42, wherein the portion of the solution of step (ii) comprises from 5% to 60 % (v/v) iodixanol in phosphate buffered saline (PBS), preferably 7.5% to 50%, and more preferably 10% to 40 %.
44. The surface of any of embodiments 39-43, wherein the portion of the solution of step (ii) comprises from 5% to 70 % (v/v) sucrose in PBS, and preferably 10% to 60 %.
45. The surface of any of embodiments 39-44, wherein the portion of the solution of step (ii) has a protein concentration ranging from 5-500 µg/ml, preferably 10-300 µg/ml, more preferably 20-200 µg/ml, more preferably 30-180 µg/ml, more preferably 50-170 µg/ml, more preferably 60-140 µg/ml, more preferably 80-120 µg/ml, more preferably 90-110 µg/ml, and most preferably around 100 µg/ml.
46. The surface of any of embodiments 39-45, wherein the EVs are dried in step (ii) under laminar flow in sterile conditions.
47. The surface of any of embodiments 39-46, wherein the EVs are purified in step (i) by ultracentrifugation followed by flotation in a density gradient, size exclusion chromatography, immune capture, flow cytometry or by combination thereof, preferably wherein the EVs are purified in step (i) by ultracentrifugation followed by flotation in a density gradient, size exclusion chromatography or immune capture, and more preferably by ultracentrifugation followed by flotation in a density gradient.
48. The surface of any of embodiments 39-47, wherein the EVs are purified in step (i) by ultracentrifugation, preferably wherein the EVs are purified in step (i) by differential centrifugation comprising at least one ultracentrifugation step, more preferably wherein the differential centrifugation is carried out in at least 2 steps above 1000× g and wherein the last step of the ultracentrifugation is carried out from 90 000 × g to 200 000 × g.
49. The surface of embodiment 48, wherein the last step of the ultracentrifugation is carried out at around 100 000 × g.
50. The surface of any of embodiments 39-49, wherein the EVs are purified in step (i) by flotation in a density gradient, preferably wherein the density gradient is an iodixanol gradient, a sucrose gradient or a colloidal silica particles coated with polyvinylpyrrolidone gradient, preferably wherein the density gradient is an iodixanol gradient.
51. The surface of embodiment 50, wherein the EVs are purified in step (i) by flotation in an iodixanol gradient from 5% (v/v) to 60% (v/v) iodixanol, preferably wherein the iodixanol gradient from 10% (v/v) to 40% (v/v) iodixanol is and more preferably wherein the iodixanol gradient from 10% (v/v) to 40% (v/v) iodixanol is in PBS.
52. The surface of any of embodiments 39-51, wherein the EVs are purified in step (i) from EV-producer cells which are further defined in any of embodiments 22-29.
53. The surface of any of embodiments 39-52, wherein the EVs are purified in step (i) from a body fluid, preferably wherein the body fluid is CSF, saliva, urine, serum or plasma, preferably CSF.
54. A device for culturing cells comprising the surface of any of embodiments 1-53 further comprising a removable covering to preserve sterility of said surface, preferably wherein the covering is a foil or lid, and even more preferably a lid.
55. The device of embodiment 54, wherein the device is a petri dish or microscope slide with a removable lid, preferably wherein the removable lid is sealed to the petri dish or microscope slide, and more preferably wherein the removable lid is sealed with tape.
56. The device of embodiments 54 or 55, wherein the device is a microscope slide, preferably wherein the removable lid is sealed to the microscope slide to protect dryness, and more preferably wherein the removable lid is sealed with tape.
57. The device of embodiments 54 or 55, wherein the device is a multi-well plate, preferably a 6-well, a 12-well, a 24-well, a 48-well, a 96-well, a 128-well or a 384-well plate, preferably wherein the removable lid is sealed to the multi-well plate to protect dryness, and more preferably wherein the removable lid is sealed with tape.
58. The device of embodiment 57, wherein the entire bottom of each well are predetermined positions.
59. The device of embodiments 57 or 58, wherein the multi-well plate has rows and columns of wells and wherein EVs from two or more different EV-producing cells are coating different rows or columns.
60. The device of embodiment 59, wherein the EV amount has been serially diluted along a part of the row or the column of the multi-well plate, preferably wherein the part is half the row or half the column.
61. The device of embodiment 60, wherein the EV amount has been serially diluted along an entire row or entire column of the multi-well plate.
62. The device of any of embodiments 57-61, wherein the multi-well plate is an EV library, preferably wherein EVs from two or more different sources are serially diluted and wherein said different sources are selected from a human cell line, and/or mouse an animal cell lines, a plant cell, a protozoan and/or a body fluid..
63. A kit comprising the device of any of embodiments 54-63 and further comprising a desiccant, preferably wherein the desiccant is silica gel.
64. A method of preparing a sterile surface according to any of embodiments 1-53, comprising the steps of
   (a) purifying the EVs by ultracentrifugation to obtain a pellet comprising the EVs,
   (b) further purifying the EVs from said pellet by flotation in a density gradient to obtain a soluble fraction comprising the EVs,
   (c) depositing a portion of said fraction on the sterile surface at one or more predetermined position(s), and
   (d) letting said portion dry by evaporation,
   whereby immobilized EVs on the sterile surface are obtained.
65. The method of embodiment 64, wherein the ultracentrifugation of step (a) is carried out in at least 2 steps above 1000× g and wherein the last step of the ultracentrifugation is carried out from 50 000 × g to 200 000 × g so that the EVs are concentrated in the pellet after the last centrifugation step.
66. The method of embodiment 65, wherein the last step of the ultracentrifugation is carried out at around 100 000 × g.
67. The method of any of embodiments 64-66, wherein the pellet of step (a) is resuspended in 30-50% iodixanol (v/v), preferably 35-45% iodixanol (v/v) and most preferably around 40% iodixanol (v/v).
68. The method of any of embodiments 64-67, wherein the pellet of step (a) is resuspended and bottom-loaded or top-loaded in the density gradient of step (b), preferably wherein the pellet of step (a) is resuspended and bottom-loaded.
69. The method of any of embodiments 64-68, wherein the density gradient of step (b) is an iodixanol gradient, a sucrose gradient or a colloidal silica particles coated with polyvinylpyrrolidone gradient, preferably wherein the density gradient of step (b) is an iodixanol gradient.
70. The method of any of embodiments 64-69, wherein the density gradient of step (b) is a discontinuous density gradient, preferably wherein the discontinuous density gradient ranges from 5% to 50% iodixanol, preferably 10% to 40 % iodixanol.
71. The method of any of embodiments 64-70, wherein the density gradient of step (b) is floated from 120 000 × g to 200 000 × g, preferably from 140 000 × g to 190 000 × g, more preferably from 170 000 × g to 185 000 × g and even more preferably around 180 000 × g.
72. The method of any of embodiments 64-71, wherein the density gradient of step (b) is floated for 10 to 36 hours, preferably 12 to 32 hours, more preferably 14 to 28 hours, even more preferably 15 to 24 hours, even more preferably 16 to 22 hours, even more preferably 17 to 20 hours, and most preferably around 19 hours.
73. The method of any of embodiments 64-72, wherein the density gradient of step (b) is floated in a swinging bucket rotor, preferably a TH-660 rotor.
74. The method of any of embodiments 64-73, wherein the soluble fraction of step (b) comprising the EVs is obtained by identifying a suitable EV marker in one or more fraction(s), preferably wherein said suitable EV marker is selected from a one or more of a tetraspanin, tsg101, fibronectin 1, flotillin 1, and/or flotillin 2, wherein the tetraspanin is selected from CD63, CD81 or CD9.
75. The method of any of embodiments 64-74, wherein the portion is deposited on the sterile surface by pipetting in step (c).
76. The method of any of embodiments 64-75, wherein the portion of step (c) has a volume of at least 0.1µl, more preferably at least 0.2 µl, more preferably at least 0.3 µl, even more preferably at least 0.4 µl, even more preferably at least 0.5 µl, even more preferably at least 0.6 µl, even more preferably at least 0.7 µl, even more preferably at least 0.8 µl, even more preferably at least 0.9 µl, and most preferably around 1 µl.
77. The method of any of embodiments 64-76, wherein the portion of step (c) has a volume of at most 50 µl, more preferably at most 40 µl, more preferably at most 30 µl, even more preferably at most 20 µl, even more preferably at most 10 µl, even more preferably at most 9 µl, even more preferably at most 8 µl, even more preferably at most 7 µl, even more preferably at most 6 µl, even more preferably at most 5 µl, even more preferably at most 4 µl, even more preferably at most 3 µl, even more preferably at most 2 µl, and most preferably around 1 µl.
78. The method of any of embodiments 64-77, wherein the portion of step (d) is dried over a time period from 30 seconds to 60 minutes, preferably from 1 minute to 45 minutes, more preferably from 3 minutes to 30 minutes, more preferably from 5 minutes to 20 minutes.
79. The method of any of embodiments 64-78, wherein the completion of step (d) is assessable by visual inspection.
80. The method of any of embodiments 64-79, wherein the completion of step (d) assessable by measuring the refractive index, liquid surface tension and/or optical tensiometer, preferably wherein the dryness is assessed by measuring the refractive index.
81. The method of embodiment 80, wherein the refractive index of the surface coated with EVs and a comparative surface without EVs is differing by less than 0.5, preferably less than 0.4, more preferably less than 0.3, even more preferably less than 0.2 and even more preferably less than less than 0.1.
82. The method of any of embodiments 64-81, wherein step (d) is completed when the portion of the fraction of step (d) does not form ice crystals upon freezing.
83. The method of any of embodiments 64-82, wherein the cells are further defined in any of embodiments 2-5, the surface is further defined in any of embodiments 6-22, wherein the EVs are further defined in any of embodiments 23-31, and/or wherein the predetermined position(s) are further defined in any of embodiments 32-38.
84. A method of monitoring cell differentiation, apoptosis, cell migration and/or proliferation comprising the steps of
   a. adding cells to the surface according to any of embodiments 1-53
   b. monitoring said cells by microscopy.
85. The method of embodiment 84, wherein the cells are monitored in real time.
86. The method of embodiments 84 or 85, wherein images of cellular network formation are taken.
87. The method of embodiment 84, wherein the cells are fixed and stained with suitable immunofluorescent markers.
88. The method of any of embodiments 84-86, wherein cell migration is monitored and wherein the migration towards or away from the immobilized EVs is monitored, preferably wherein the migration towards the EVs is monitored.
89. The method of any of embodiments 84-86 and 88, wherein cell migration of cells from tissue, explants, organoids, embryonic bodies, and/or spheroids cancer cells is monitored.
90. A method of monitoring cell differentiation, transcriptome, proteome and/or cell development comprising the steps of
   a. adding cells to the surface according to any of embodiments 1-53
   b. monitoring said cells by qPCR
91. The method of embodiment 90, wherein the qPCR is carried out using one or more suitable markers, preferably wherein the one or more suitable marker is selected from gfap, klf4, nanog, nestin, sox2, oct4, pax6 and/or vimentin.
92. A method of monitoring cell differentiation comprising the steps of
   a. adding cells to the surface according to any of embodiments 1-53
   b. monitoring said cells by single cell sequencing.
93. A method of monitoring cell viability and/or apoptosis comprising the steps of
   a. adding cells to the surface according to any of embodiments 1-53
   b. monitoring said cells by colormetric detection in a plate reader.
94. The method of embodiment 93, wherein cell viability and/or apoptosis is detected by a caspase-3 or MTT assay.
95. The method of embodiment 93, wherein cell viability is detected by an ATP-luciferase assay.
96. The method of embodiment 93, wherein cell viability and/or apoptosis is detected by an annexin Xii based polarity sensitive probe.
97. A method of monitoring cell viability, apoptosis and/or proliferation comprising the steps of
   a. adding cells to the surface according to any of embodiments 1-53 and
   b. monitoring said cells by flow cytometry.
98. A method of monitoring a proteome of cells comprising the steps of
   a. adding cells to the surface according to any of embodiments 1-53 and
   b. monitoring said cells by mass spectrometry.
99. A method of monitoring protein-nucleic acid interactions comprising the steps of
   a. adding cells to the surface according to any of embodiments 1-53 and
   b. monitoring said cells by ChIP-sequencing.
100. A method of monitoring cell development comprising the steps of
   a. adding cells to the surface according to any of embodiments 1-53 and
   b. monitoring said cells by analysing the DNA methylation and/or DNA acetylation pattern.
101. Use of the surface according to any of embodiments 1-53 for monitoring cell differentiation, cell proliferation, cell migration, viability, and/or apoptosis.
102. Use of the surface according to any of embodiments 1-53 as a research tool.
103. Use of the surface according to any of embodiments 1-53 as a diagnostic in vitro assay.

### Description of the Figures

**Figure 1A****: Overall depiction of invention.** Immobilized (dried) drops of exosomes are deposited in a multi-well plate. The concentration and the composition of EVs may be titrates along a row or column. In a next step (lower depiction) different types of cells or the same type of cells are added to the wells and the differentiation/grow of the cells is then monitored, compared and contrasted in the wells. The growth/differentiation may then be monitored by microscopy. For example, differentiation, migration, viability, apoptosis and proliferation may be assessed as well as a wound healing assay may be performed. Cellular network formation may also be assessed.
**Figure 1B****: Example of a plate used for testing of EVs from various source with user-supplied stem cells.** 10 EV types (column 2-11) are double-plated at four different particle concentrations (A-D and E-H). Thus, in this exemplary setting two user-provided cell types can be tested. Alternatively, user-provided cells can be tested as replicates. Negative and positive controls are in rows 1 and 12. NSC test cells for said rows come with the plate.

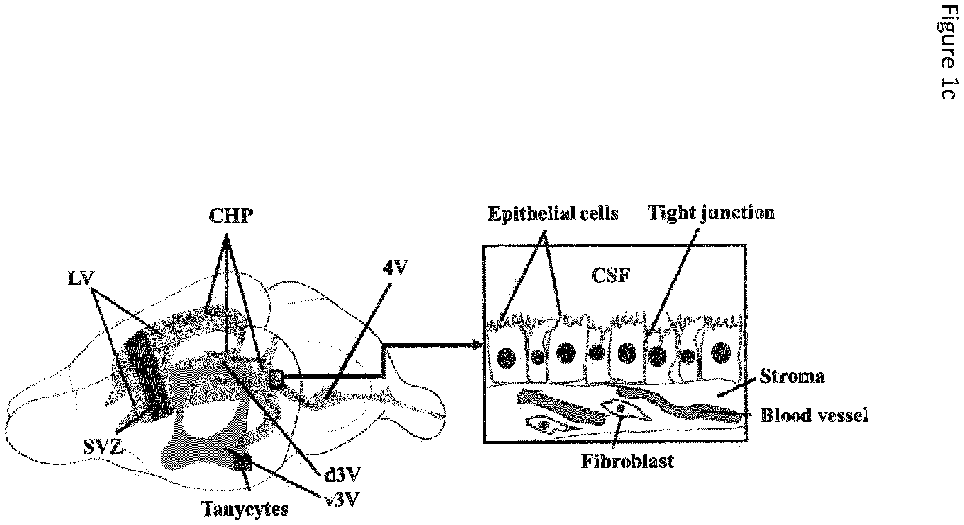

**Figure 1C****: Architecture of the ventricles of the mouse brain.**
   Lateral view of the adult mouse brain showing the ventricular system that consist of two lateral ventricles (LV, gray), the bipartite third ventricle (dorsal 3V, blue [d3V] and ventral 3V, orange [v3V]) and the fourth ventricle (4V, gray). Each ventricle has a choroid plexus (pink) that produces CSF. The choroid plexus (CHP, right box) consists of a secretory polarized epithelial cell layer and stroma composed of fibroblasts [63], immune cells and perivascular cells. The tight junctions between epithelial cells form the blood-CSF barrier [100]. Neurogenic niches are the subventricular zone (SVZ) and the tanycyte region (both green).

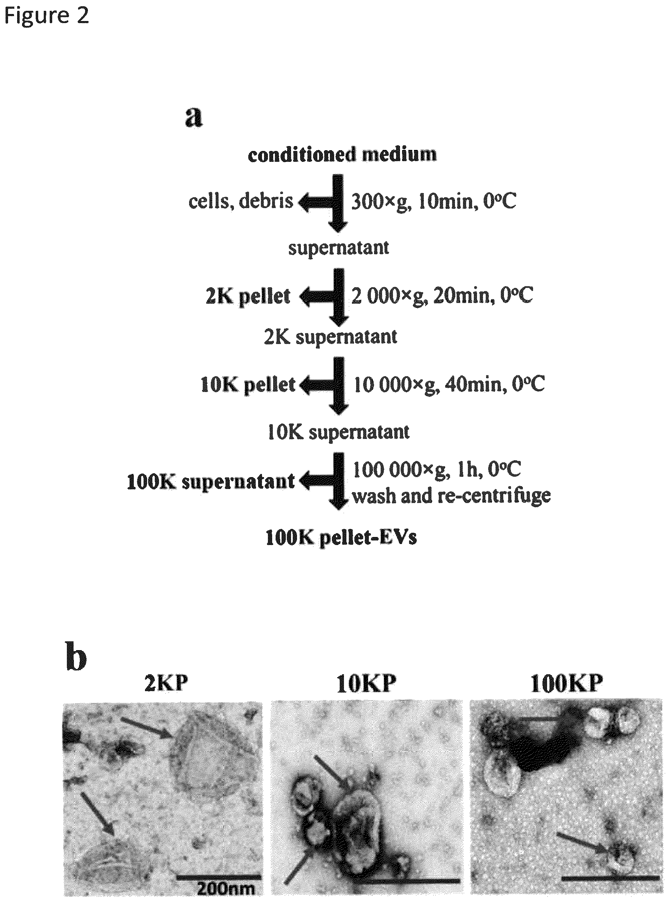

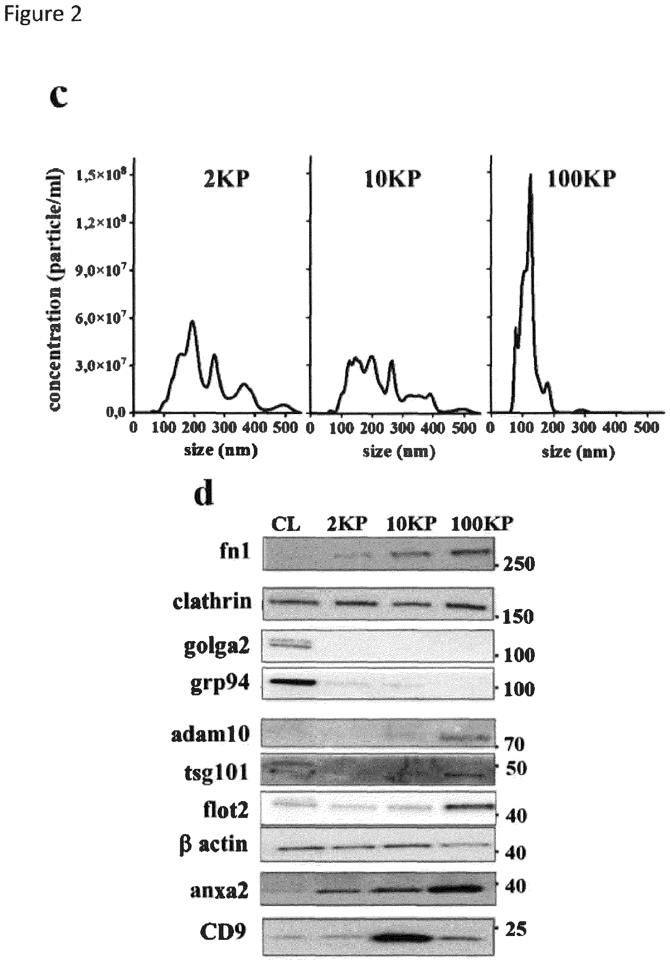

**Figure 2****. Purification and characterization of EV^{Z310} from Z310 cell conditioned medium by differential ultracentrifugation.**
   (a) Scheme of purification of EVs from Z310 or MEF conditioned medium by differential centrifugation. (b) Representative electron micrographs of Z310 conditioned medium derived vesicles (red arrows) present in the 2K, 10K and 100K Z310 pellets indicated in the scheme of Figure 2 (a). (c) Nanoparticle tracking analysis data showing the size distribution of the particles isolated from Z310 conditioned medium in the various pellets that are indicated in the purification scheme of Figure 2(a). The 100K pellet contains EVs^{Z310} in a size range of 50nm-150nm. (d) Individual fractions obtained in different steps of purification of Z310 supernatant were analyzed by Western blotting. Z310 cell lysate (CL) is the lane on the left.

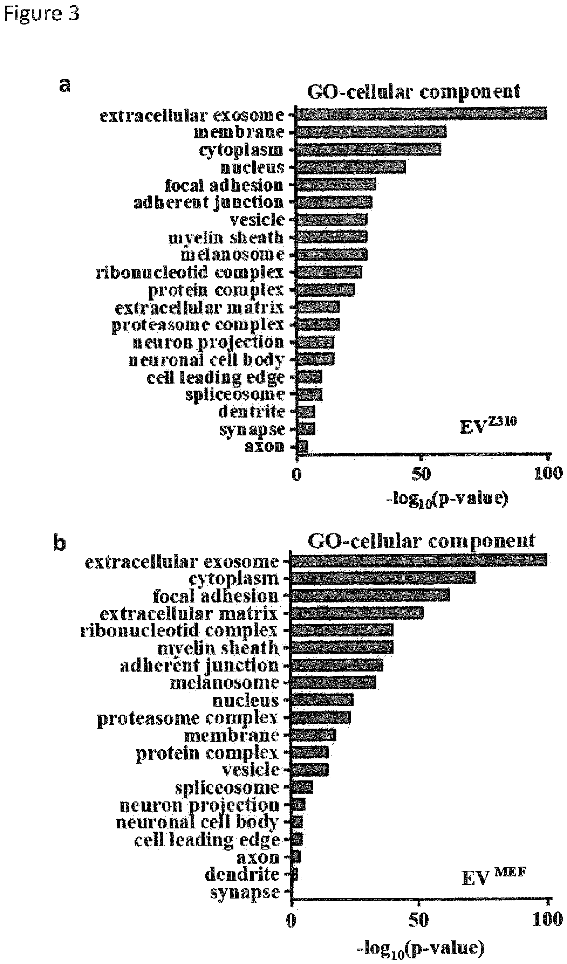

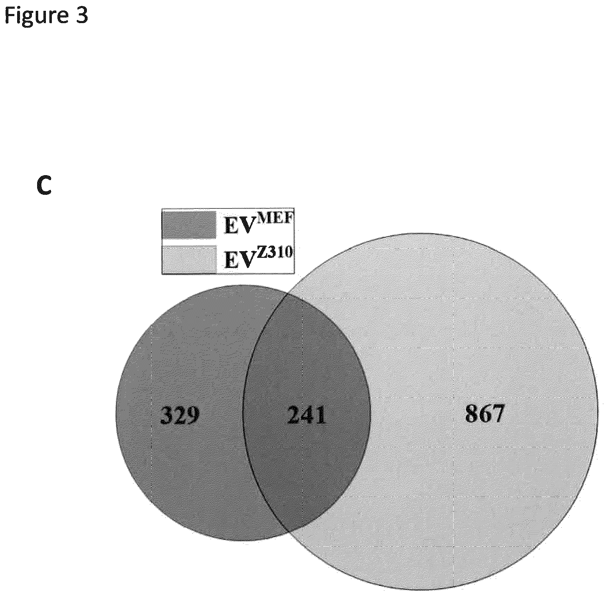

**Figure 3****. Analysis of EV^{Z310} and EV^{MEF} proteomes.**
   (a) GO-cellular components of EV^{Z310}. Note an enrichment in "extracellular exosome", "membrane", "vesicles", "neuronal projection", "neuronal cell body", "dendrite", "synapse" and "axon" sub-categories. (c) Venn diagram shows that EV^{Z310} and EV^{MEF} protein contents are different. (*d) GO-cellular components of EV^{MEF}. Comparison with EV^{Z310} show differences in ranking of several categories such as* "*membrane*" *that ranks high only in the EV^{Z310}.*
**Figure 4****. EV^{Z310} promote NSC differentiation.** (a) Bright field images of NSC^{tz} and NSC^{SVZ} co-cultured for 24 h with EV medium only, with EV^{Z310}, with 100K supernatant or with EV^{MEF}. Total EV protein concentration in all cases was 76 µg/ml. (b) Immunostaining for various markers visualize cell structure and cell processes that formed in the presence of EV^{Z310}. Astrocytes (GFAP) and early neurons (Tuj1) were seen. NSC cultured in medium without EV^{Z310} did not form processes, but just spherical aggregates of irregular shape. Note the punctate Fn1 staining in the differentiating EV^{Z310}-treated NSC^{tz}. (c) qPCR showed upregulation of *Cadh2, Fnl, Gfap, Stat3* and *vimentin* expression 24h after EV^{Z310}addition. Values are presented as means ± SD, three biological and three technical replicates were done (^{∗∗} p< 0.01, Student's t test). (d) Immunofluorescence staining for astrocyte marker (GFAP) show astrocytic cells with multiple processes and positive staining for nuclear Stat3 in NSC^{tz} and NSC^{SVZ} after a 24 h co-culture with EV^{Z310}.
**Figure 5****. EV^{Z310} promote NSC differentiation in dose dependent manner.**
   (a) Flow cytometry showed that after 24h EV^{Z310} significantly induced GFAP and Stat3 positive cells number (NSC^{tz}) in dose dependent manner (total protein concentration 38, 76, 152 µg/ml). In contrast EV^{MEF} did not elevated numbers of GFAP- and Stat3- positive cells above control (b) Similar results were obtained when NSC^{SVZ} were co-cultured with EVs. EV^{Z310} but not EV^{MEF} increased the number of GFAP- and Stat3-positive cells in dose dependent manner. (c) Three qPCR graphs demonstrated a significant dose dependent effect of EV^{Z310} (concentration range 19-304 µg/ml) on selected markers after 24h co-culture with NSC^{tz}. Values are presented as means ± SD from three independent experiment (^{∗} p < 0.05, ^{∗∗} p< 0.01, Student's t test).

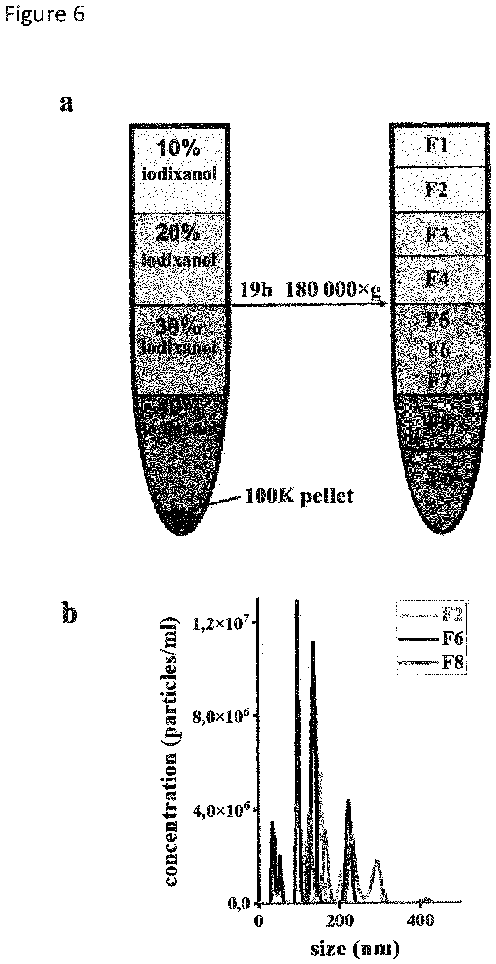

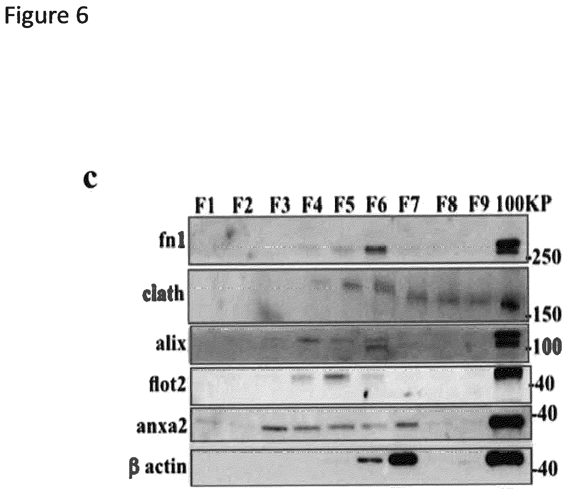

**Figure 6****. EV purification by flotation on iodixanol gradient separates subfractions of EVs.**
   (a) Scheme of EVs purification by density gradient separation. The 100K pellet obtained by differential centrifugation was resuspended in 40% iodixanol and bottom loaded. Fraction 6 (light grey) was visible as an opaque band after centrifugation. (b) Particle size distribution in three selected fractions. Fraction 6 (black) showed the highest particle concentration and contained the majority of vesicle in the size range of 50-150nm. Fractions 2 and 8 (light grey and medium grey) had a strong bias for larger vesicles (150-300nm). (c) Western blot of the nine fractions and of the 100K pellet (starting material). Fraction 6 shows the highest level of Fn1 and Alix.

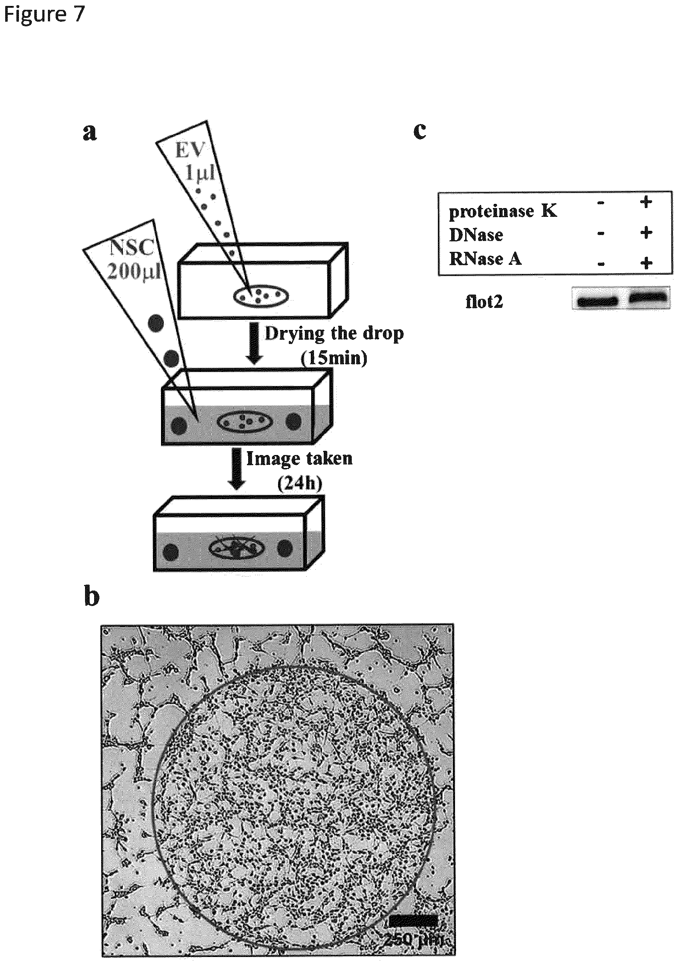

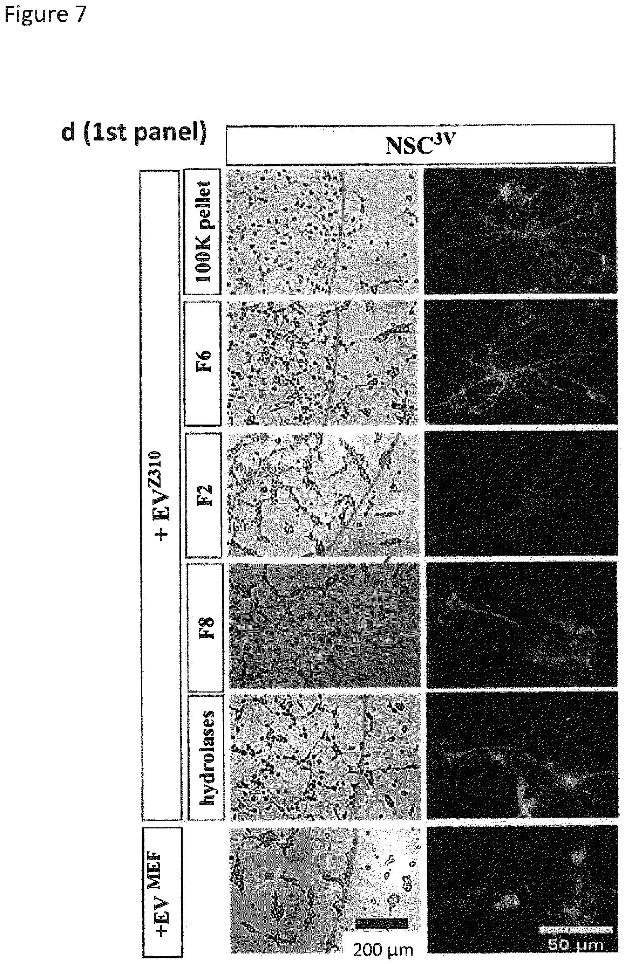

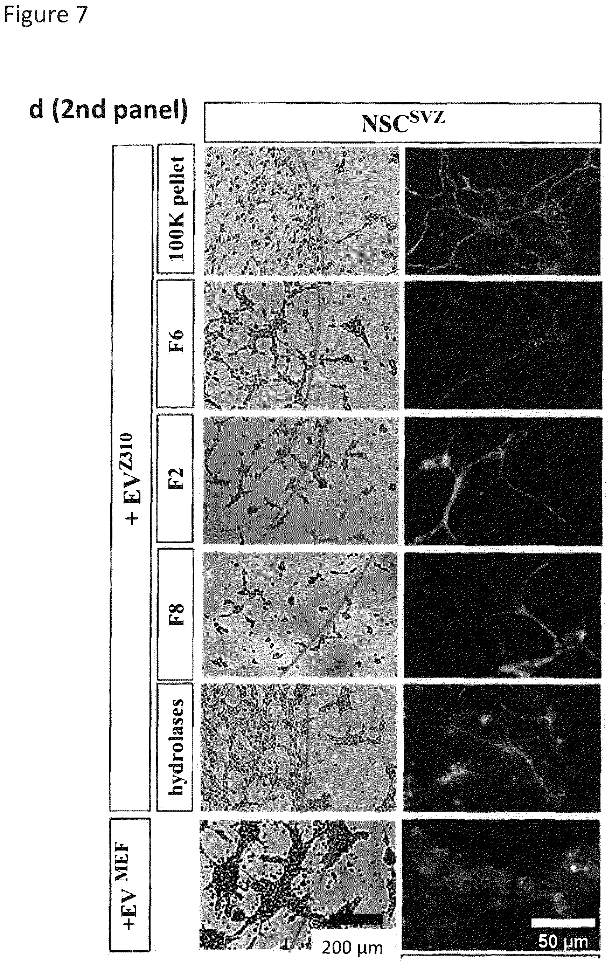

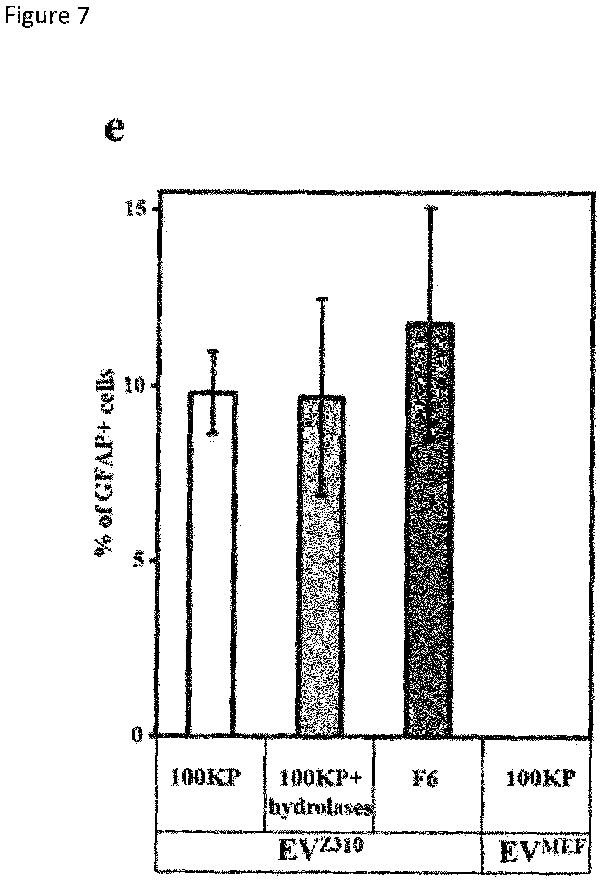

**Figure 7****. Immobilized drop assay to assess EV biological activity.**
   (a) Immobilized drop assay: A 1µl drop of EVs is deposited on the floor of an 8-chamber slide and let dry for 15 minutes. Thereafter, (up to a week later if slides are kept at -20 °C) 200 µl of the NSC suspension is added to each chamber. The chambers are incubated for 24 hours, at 37°C in a tissue culture incubator. (b) The NSC^{tz} attach and form a cellular network only in the area of the EV drop (blue boundary). Cells outside the boundary are still floating or are at best weakly attached. (c) Level of Flot2 located inside EV^{Z310} was not affected by treatment with proteinase K, DNase and RNase. (d) Fraction number 6 obtained from the iodixanol gradient provides excellent support for NSC^{tz} (first panel) and NSC^{SVZ} (second panel) network formation. When EV^{Z310} from fraction 6 were used, immunofluorescence revealed the presence of cells with astrocyte morphology that also expressed GFAP. Treatment with hydrolases had no influence on EV^{Z310} function. EV^{MEF} did not provide proper support for network formation, regardless of the type of NSC used (bottom row). The blue line marks the periphery of the dried drop. (e) Percentage of GFAP expressing cells in the area of the drop. Data were normalized to the cell number.

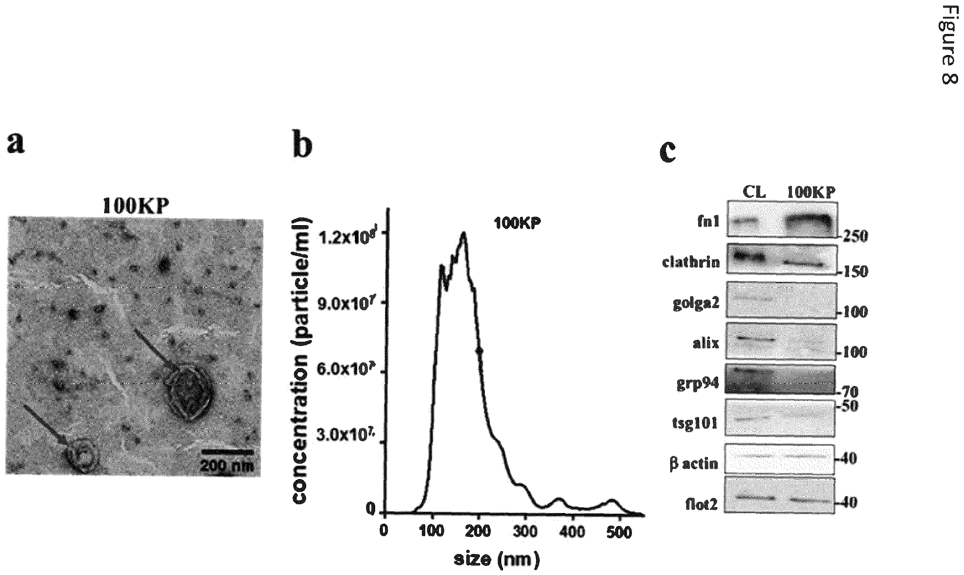

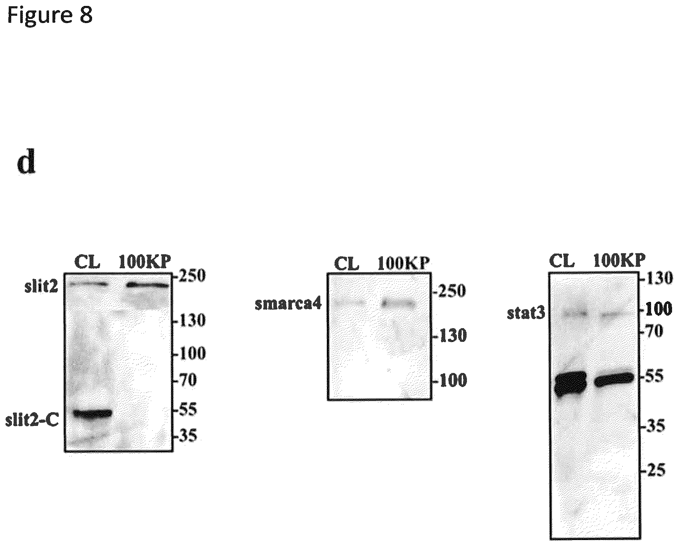

**Figure 8****: Purification and characterization of EV^{MEF} from MEF cell conditioned medium.**
   (a) Representative electron micrograph of vesicles (red arrows) present in EVs^{MEF} 100K pellet. (b) Nanoparticle tracking analysis data showing the size distribution of the particles in EVs^{MEF} 100K pellet. The 100K pellet obtained after ultracentrifugation, shows EVs in a size range of 75nm-200nm. (c) 100K pellet obtained after differential centrifugation was analyzed by Western blotting together with MEF cell lysate (CL) for EVs positive and negative markers. (d) Western blot of the Z310 cell lysate (CL) and EVs^{Z310} in the 100K pellet (100KP in Figure 2(a)) showed that selected proteins give specific bands at molecular weights provided by the manufacturer of the antibodies. Slit 2: 200kDa, smarca4: 185kDa and stat3 92kDa. Note, the specific bands are present in the cell lysate and the EVs^{Z310}.

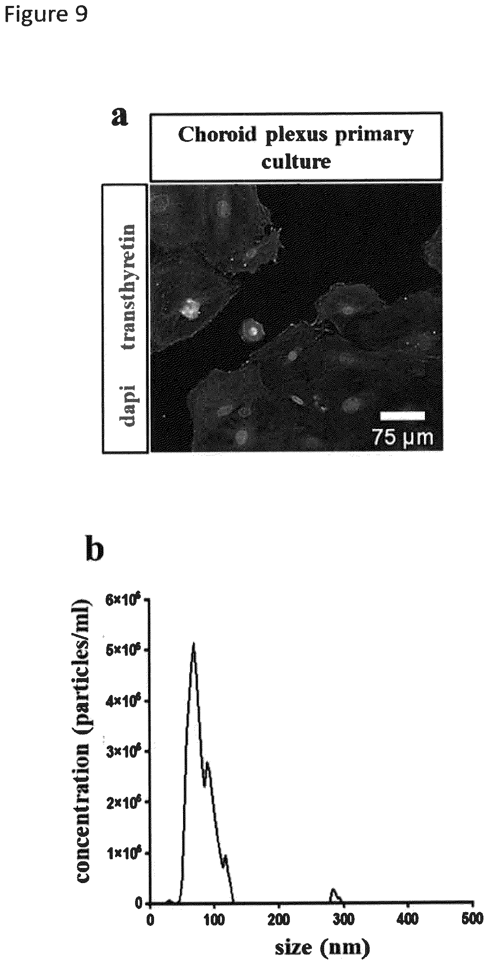

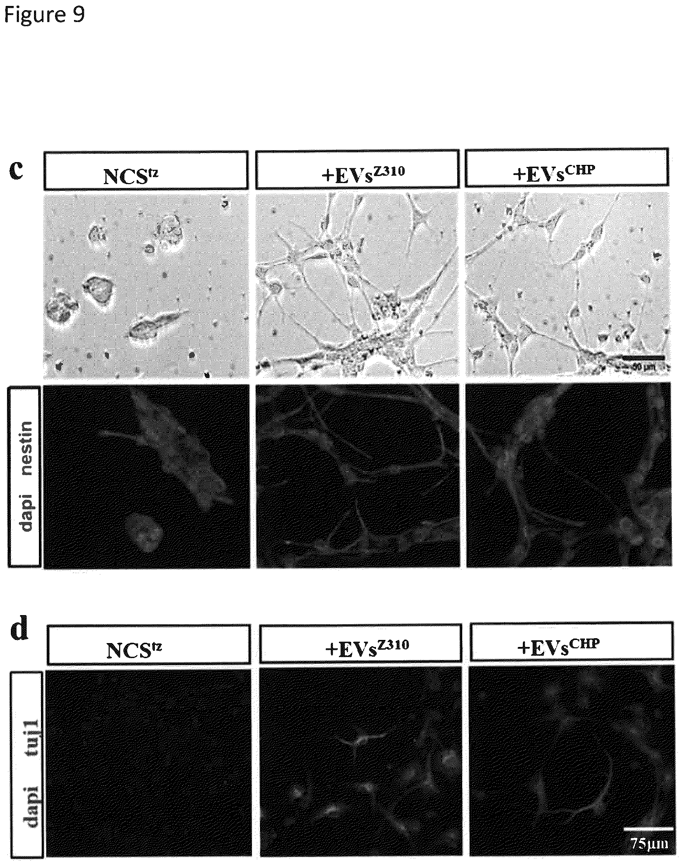

**Figure 9****: EVs produced by primary culture of choroid plexus (EVs^{CHP PC}) induce NSC network formation and differentiation.**
   (a) Positive immunostaining for choroid plexus marker transthyretin in all primary culture cells. (b) Nanoparticle tracking analysis showing the size distribution of EVs^{CHP PC} 100K pellet. The size range is between 50-120nm. (c) Both EVs^{CHP PC} and EVs^{Z310} form similar cellular networks of NSC^{tz}. Top bright field, bottom nestin staining (24h). (d) Both EVs^{CHP PC} and EVs^{Z310} induce tuj1 expression in NSC^{tz} (48h).

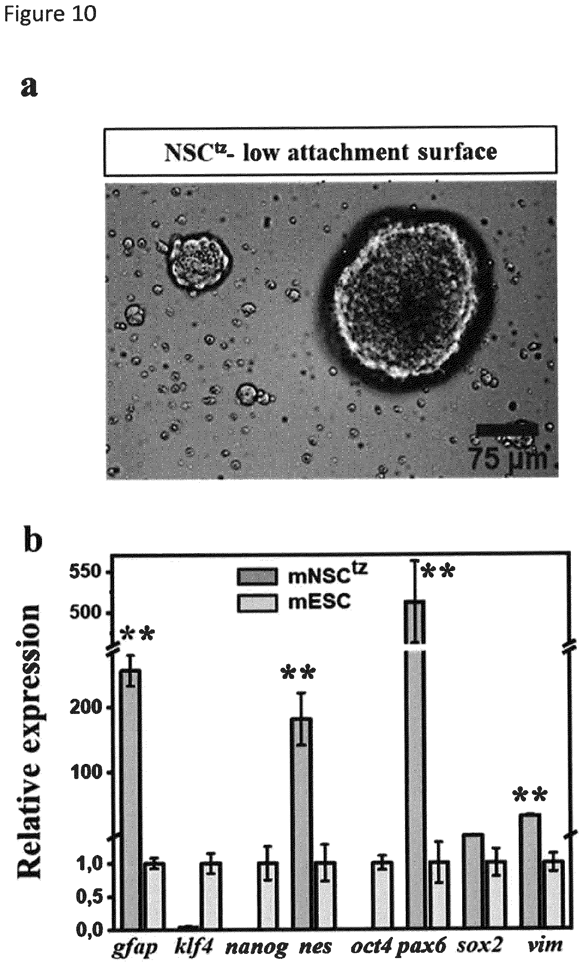

**Figure 10****: NSC^{tz} form neurospheres and express neural stem cell markers.**
   (a) Bright field image of the spherical NSC^{tz} aggregates (neurospheres) cultured in a low attachment surface dish. (b) qPCR showed significant higher level of *Gfap, Pax6, nestin* and *vimentin* expression in NSC^{tz} when compared to embryonic stem cells (ESC). Values are presented as means ± SD, three biological and three technical replicates were analyzed (^{∗∗} p< 0.01, Student's t test).
**Figure 11****: Effect of EVs treatment of NSC^{tz} and NSC^{SVZ} on the expression of Tuj1 and GFAP (a, b) and on the rate of cell proliferation (c-e).**
   (a) Examples of NSC^{tz} and NSC^{SVZ} cultures in the presence of EVs^{Z310} (total protein concentration was 304µg/ml) and immunostained for Tujl (neurons) and GFAP (astrocytes) after 48h of treatment.(b) Quantification of percentage of GFAP+ cells (astrocytes) and Tuj1+cells (early neurons) in NSC^{tz} and NSC^{SVZ} after co-culture with EVs^{Z310} (total protein concentration 304µg/ml) for 24 and 48h. Values are presented as means ± SD (^{∗∗} p< 0.01, Student's t test).(c) MTT proliferation assay showed that after 24h of treatment EVs^{Z310} significantly induced NSC^{tz} proliferation. EVs^{MEF} did not induced NSC^{tz} proliferation above the control levels. Values are means ± SD, 4 biological and 4 technical replicates were analyzed (^{∗} p < 0.05, ^{∗∗} p< 0.01, Student's t test). (d) MTT proliferation assay showed that after 24h of treatment EVs^{Z310} but not EVs^{MEF} significantly induced NSC^{SVZ} proliferation. Values are presented as means ± SD, n=16 (^{∗} p < 0.05, ^{∗∗} p< 0.01, Student's t test). (e)Trypan blue proliferation assay showed that EVs^{Z310} induce NSC^{tz} and NSC^{SVZ} proliferation after 24h.

### Examples

### Example 1 - Extracellular vesicles derived from the choroid plexus trigger the differentiation of neural stem cells

The mammalian ventricular system of the brain consists of two lateral ventricles, the third and the fourth ventricle (Figure 1). The ventricles are filled with cerebrospinal fluid (CSF) which is produced in and flows through the ventricles before being absorbed by arachnoid granulations and the lymphatic system [1]. Ependymal cells form the ventricular walls and carry each a bundle of motile cilia that mediates directional CSF flow [2]. This directional flow follows precise trajectories [3] suggesting that in this way CSF components are delivered to specific locations within the ventricular system [4]. Most of the CSF is generated and secreted by the choroid plexus [5]. CSF protects the brain from mechanical impacts and collects waste from the brain [6]. In addition, CSF contains signaling factors, ions, lipids and hormones required for proper brain development and function [7-12]. CSF also contains extracellular vesicles (EVs) [8, 13-20] that are bilayer membrane enclosed vesicles, produced and secreted by many cell types. EVs are loaded with proteins and nucleic acids [21-25]. EVs termed microvesicles (size range of 100-1000nm) form by outward budding of plasma membrane. EVs termed exosomes (size 50-150nm) form by an intracellular endocytic trafficking pathway involving multivesicular endosomes that release exosomes upon fusion with the plasma membrane [26, 27]. Because current purification procedures do not exclude the presence of microvesicles in exosome preparations and *vice versa,* one refers to these vesicles collectively as "EVs".

EVs mediate communication *in vitro* and *in vivo* [26, 27] between different cell types such as neurons and oligodendrocytes [28], neurons and astroglia [29, 30] and neurons and Schwann cells [31]. EVs involved in intercellular communication do so in the pre-metastatic niche [32], for instance by promoting breast cancer cells motility *via* Wnt-planar cell polarity signaling [33]. EVs also play a role in the mesenchymal stem cell niche [34] and in the epithelial-mesenchymal niche [35]. Glioma-derived EVs drive differentiation of embryonic neural stem cells to astrocytes [36] and EVs are thought to regulate neurogenesis in the early postnatal mouse brain [37]. Recently it was shown that Cyclin D1 contained in EVs produced by PC12 and N2A cell lines promote neural differentiation of embryonic stem cells [38].

Injecting fluorescently labelled choroid plexus-derived EVs into the lateral ventricle showed that EVs could cross the ependymal cell layer and reach brain parenchyma [17, 39]. EVs have various effects in the nervous system. Balusu *et al.* [17] showed that systemic inflammation of mice by intraperitoneal injection of lipopolysaccharide or tumor necrosis factor induced an increase in pro-inflammatory miRNAs in EVs produced by choroid plexus. These EVs also interacted with astrocytes and microglia to up regulate inflammatory genes [17]. Lepko *et al.* [40] found that miR-204 present in EVs that originate from the choroid plexus are taken up by neural stem cells (NSC) of the subventricular zone (SVZ) and maintain them in the quiescent state but primed for rapid neurogenesis [40]. Apparently, an EV based long-distance signaling pathway regulates the number of quiescent neural stem cells in the SVZ.

In the adult mammalian brain, two NSC niches directly contact CSF and may thus have access to CSF-born EVs (Figure 1). By far the best-studied NSC niche is the SVZ that contains neural progenitors some of which (B1 cells) project a primary cilium into the lateral ventricle [41-43]. A second niche lies in the posterior part of the ventral third ventricle (v3V, Figure 1), in a region populated by tanycytes. They project a primary cilium into v3V and have a single, long basal process that contacts distinct nuclei of the hypothalamus [44]. A subset of tanycytes show proliferation and neural differentiation in the postnatal rodent brain [45-48].

The CSF flow in the v3V [3] could deliver EVs secreted by the choroid plexus to the stem cells [3, 49]. A direct link between CSF-born factors and NSC-SVZ development and differentiation was seen in embryonic brain development [7, 9, 11, 18] and also in the adult brain [10, 40, 50, 51]. Several studies indicated a direct connection between EVs present in CSF and neural stem cells niches [8, 18, 40, 52-55]. Yet much needs to be learned about the chemical identity of EV-born neurogenic factors, how they are packaged into EVs, how they are delivered to and interact with the NSCs and which responses they evoke in their targets. The inventors have developed an *in vitro* assay that can address some of these questions. The inventors prepared EVs from the secretome of the rat Z310 choroid plexus cell line (EV^{Z310}), by a combination of differential centrifugation and flotation on an iodixanol density gradient [56, 57]. These EV^{Z310} were co-cultured with NSC isolated either from the SVZ or from the tanycyte region. EVs were added to NSC in medium or, alternatively, as dried-down deposits. In both cases, purified EV^{Z310} induced the compact, rounded NSCs to rapidly form intricate cellular networks in which multiple cells were contacting each other through long processes. EVs produced by choroid plexus primary culture evoked NSC differentiation in the similar manner.

These dramatic morphological changes were accompanied by the induction of genes in the differentiated cells, genes that are characteristic for early neurons and astrocytes. Gene induction was dose dependent and reached saturation at EV^{Z310} concentrations of 1.2-1.5×10⁹ particles per ml. LC-MS/MS showed that the differentiation-inducing EV^{Z310} were enriched for membrane and membrane associated proteins involved in cell differentiation, membrane trafficking and membrane organization. EV^{MEF} purified from mouse embryonic fibroblasts (MEFs), had little effect on the NSCs and did not show an enrichment of membrane and membrane associated proteins.

### Results

### Characterization of EVs and their protein composition

The inventors purified EVs secreted by rat Z310 choroid plexus cells (EV^{Z310}) by mouse choroid plexus primary culture (EV^{CHP}) and by mouse embryonic fibroblasts (EV^{MEF}). The Z310 cell line is a widely used surrogate for choroid plexus primary cells (for references for the Z310 cells usage see Materials and Methods). Conditioned medium from both cells was collected and subjected to differential centrifugation (Figure 2 (a)). Pellets obtained after each centrifugation step were analyzed for particle size, particle concentration and for the presence of EV protein markers [73] (Figure 2 (b-d); Figure 8 (a-c)). The 2K and 10K pellets had the highest total protein content and nanoparticle tracking analysis and electron microscopy revealed the presence of particles in the size range of 100-500nm (Figure 2 (b, c)). Centrifugation at 100 000×*g* removed particles >200nm and yielded predominantly particles of 100nm, the size for exosomal EVs (Figure 2 (b, c); Figure 8 (a, b)). Choroid plexus primary cultures cells expressed transthyretin (Figure 9 (a)) and produced EVs in the size range of 30 to 120nm (Figure 9 (b)).

Fibronectin 1 (Fn1), disintegrin and metalloproteinase domain containing protein 10 (Adam10) and tumor susceptibility gene 101 (Tsg101) are typical for small EVs [74] and they were noticeably enriched in the 100K pellet (Figure 2 (d)). Annexin A2 (Anxa2) and flotillin2 (Flot2) were elevated in this fraction but were also present in the 2K and 10K pellets. By contrast, endoplasmin (Gpr94, Hsp90b1), a marker for large vesicles [74], was present in the whole cell lysate (CL), barely detected in the 2K and 10K pellets, and absent in the 100K pellet. Golgi marker, golgin subfamily A member 2 (Golga2/ Gm130) was found only in the cell lysate fraction (CL). β-actin levels declined during purification, with a maximum in cell lysate and a minimum in the 100K pellet, e.g. [56] (Figure 2 (d)). Figure 8 (c) shows that in the 100K pellet containing EV^{MEF}, EV markers (fn1, clathrin, alix, tstg101 and flot2) were present. By contrast, golga2 and grp94 were absent, as expected, in the EV^{MEF} and found in MEF cell lysate.

The 100K Z310 pellet obtained after the ultracentrifugation was subjected to mass spectrometric analysis.∼1200 proteins were identified which is in the range as reported for EVs isolated from mouse CSF [17]. The MS data confirmed the presence of the typical EV markers. Examples are transmembrane or GPI-anchored tetraspanins (CD9, tetraspanin-8), integrins (integrins alfa, -beta), basement membrane-specific heparan sulfate proteoglycan core protein (Hspg2), basigin (Bsg), Adam10 and multidrug resistance-associated protein 1 (Abccl). Cytosolic proteins were also recovered in EVs including Tsg101, programmed cell death 6-interacting protein (Pdcd6ip, Alix), vacuolar protein sorting-associated protein (Vps4a), arrestin domain-containing protein 1 (Arrdc1), Flot1/2, transforming protein RhoA (Rhoa), annexins (e.g. anxa1, -2, -5, -11), heat shock cognate 71kDa protein (Hspa8) and syntenin-1 (Sdcbp). MS analysis also identified secreted protein typically recovered with EVs (e.g. lactadherin-Mfge8). By contrast, neither cytokines (interleukins, interferons) nor growth factors were detected, with the exception of bone morphogenetic protein 1 (Bmp1). Bmp1, a metalloproteinase that was previously identified in the CSF and plays a role in the development of the CNS by stimulating progenitors in the SVZ [11]. Thus, Bmp1 could be one of the CSF-born neurogenic factors that is transported inside EVs. Proteins associated with other intracellular compartments such as the nucleus (histones) or mitochondria (Tomm20) but not with Golgi apparatus (Golga2) were also detected. The 100K pellet also contained ribosomal proteins and proteasomes, large protein complexes known to cosediment with EVs [73, 75].

Gene ontology (GO) enrichment analysis of "cellular component" categories showed that proteins identified in the 100K pellet were significantly enriched for "extracellular exosome" "membrane" and "vesicle" sub-categories (Figure 3 (a)). Other enriched sub-categories were "focal adhesion", "adherent junction", "extracellular matrix", "neuron projection", "neuronal cell body", "dendrite", "synapse" and "axon". Selected typical EV proteins retrieved from the sub-category "vesicle" formed a STRING protein interaction network (Figure 3 (b)). The inventors picked this sub-category since it contained EV-typical markers discussed above. The inventor's analysis revealed a functional association and a significant number of interactions (PPI enrichment p-value < 1.e⁻¹⁶). MCL clustering (inflation parameter 1.9), revealed four major proteins clusters (Figure 3 (b)). The biggest cluster (red) included proteins associated with "SNARE, vesicle fusion and transport". Two additional clusters (yellow, green) represented proteins involved in endosomal sorting complex required for transport (ESCRT), cell adhesion, morphogenesis and cellular projections. Proteins involved in ER-Golgi transport formed the smallest cluster (blue). This result showed strong interactions of proteins relevant to EV biology.

Western blots were used to investigate whether proteins identified by mass spectrometry were present as full-length protein in EV^{Z310} lysates. The inventors choose signal transducer and activator of transcription 3-stat3 (92kDa), slit homolog 2 protein-slit2 (200kDa) and transcription activator BRG1 also know as ATP-dependent chromatin remodeler-smarca4 (185kDa). Slit2 is a secreted extracellular matrix *protein* involved in axon guidance [49, 76, 77]. Stat3 is a transcription factor and smarca4 a transcriptional activator that both regulate GFAP expression [78, 79]. Figure 8 (d) demonstrates that these proteins are present in EV^{Z310} and electrophoresis, according to the band size provided by the manufacturer.

EVs from MEFs (EV^{MEF}), were prepared and analyzed as described for EV^{Z310} (Figure 2 (a) and Figure 8 (a-c)) and underwent MS analysis. 570 proteins could be identified , less than for EV^{Z310}. The likely cause for this difference is that Z310 cells but not MEFs are secretory cells. A Venn diagram (Figure 3 (c)) reveals that ~40 % of the EV^{MEF} proteins were also present in EV^{Z310}. 329 proteins were exclusive for EV^{MEF} and 867 for EV^{Z310}. Gene ontology enrichment analysis for the category "cellular component" showed in EV^{MEF} a significant enrichment of proteins in sub-categories "focal adhesion", "extracellular matrix" and "adherent junction". This finding is consistent with a role of fibroblasts in the CNS including the choroid plexus to provide structural support by secretion of extracellular matrix proteins [80]. The sub-category "membrane" was much less enriched in EV^{MEF} than in EV^{Z310} and just a few EV^{MEF} components fell into the subcategories related to neurons.

STRING functional analysis of the 248 EV^{Z310} proteins that were classified into the subcategory "membrane" and not identified in EV^{MEF}, yielded three prominent clusters (Figure 3 (e)). Two small clusters (brown) compiled proteins involved in "RNA metabolism" and "protein biosynthesis". Examples are 60S ribosomal proteins L14 (Rpl14), Rpl27, Rpl36 and eukaryotic translation initiation factors (Eif4g2, Eif3k). Some of the ribosomal proteins that are present in 100K pellet co-purify with EVs [56] may not be EVs cargo [73]. The red cluster comprised proteins associated with "neurogenesis", "cell differentiation", "cell migration", "membrane organization and trafficking". Examples are Adam17 and a Rheb-Ras homolog enriched in brain where it plays a role in neural plasticity. Cdk5 which is also in the red cluster is important for neural migration and CNS development and regulates cytoarchitecture, axon guidance and membrane transport [81]. Proteins involved in sub-category "synapse" are sorting nexin-4 (Stx4) and N-ethylmaleimide-sensitive factor attachment protein alpha (Napa). Examples of the proteins found in sub-category "cell migration" and "membrane organization" are integrins, Itgb4 and integrin-linked protein kinase-Ilk, which are involved in cell adhesion, cell architecture and cell motility. Proteins of the "membrane trafficking and organization" sub-categories are Ras-related protein Rab-10 (Rab10), EH domain-containing protein 2 (Ehd2), sodium-hydrogen antiporter 3 regulator (Slc9a3r1). The inventors took the composition of the red cluster as an indicator that EV^{Z310} contains proteins that could control the NSC biology.

### EVs from the Z310 cell line induce NSC differentiation in a dose dependent manner

As shown by MS analysis, EVs from the choroid plexus derived Z310 cell line contain many proteins associated with membrane function and neuronal differentiation (Figure 3). It is possible, that after their secretion by the choroid plexus into the CSF some EVs are transported to the neural stem cell niches (Figure 1) where they might affect NSCs membrane and/or evoke the differentiation of the NSCs. To test this hypothesis, the inventors prepared NSCs from the tanycyte region and the SVZ of mouse brain (Figure 1) and generated NSC aggregates (neurospheres) using standard methods (see Materials and Methods and [72]). In accordance with previous work [45, 46, 72] the NSCs from either region formed neurospheres (Figure 10 (a)), that expressed typical NSC markers, such as *nestin, paired box protein 6 (Pax6), Gfap, Sox2 and vimentin* (Figure 10 (b)).

After the neurospheres were dissociated into single cells, EV^{Z310} were added to them. The inventors found that within 24 hours, the NSCs from the tanycyte niche (NSC^{tz}) formed complex cellular networks in which individual cells cross-connected with each other through long processes (Figure 4 (a), first column, second row). NSCs from the SVZ niche (NSC^{SVZ}) yielded similar cellular networks (Figure 4 (a), second column, second row). In the case of EV^{Z310}-exposed NSC^{tz}, 50 ± 2% of the attached cells were in contact with at least one neighbor, had ≥3 processes that were ≥ 20µm long. In the case of NSC^{SVZ} this percentage was 50 ± 5%. Therefore, network-forming cells account for half of all attached cells. The inventors rarely saw such processes in controls in which only culture medium was added to NSCs (Figure 4 (a), top row). Likewise, EV^{MEF} evoked clustering of just a few NSC^{tz} or NSC^{SVZ} and the inventors did not observe a cell process-based network (Figure 4 (a), bottom row). The EV-free 100K supernatant from the differential centrifugation step (Figure 2 (a)) added to NSC^{tz} or NSC^{SVZ} had no effect on these cells (Figure 4 (a), third row).

Immunostaining of fixed NSC^{tz} cultures with antibodies against intermediate filament proteins vimentin and nestin visualized the fine structure of the cellular processes and showed that processes originating from a particular cell can make multiple contacts with the neighboring cells (Figure 4 (b) right column). Note, the glial fibrillary acidic protein (GFAP) expressing cell (Figure 4 (b) right column, third row), probably an astrocyte, contacts multiple times with the nestin expressing cellular network. Network-forming cells account for half of all attached cells and the GFAP positive cells belong chiefly to this group of attached, stellate cells. Single cells with a compact morphology (presumably residual NSCs), express GFAP at very low levels in the cytoplasm surrounding the nucleus ((Figure 4 (b), first column, third row). The extracellular matrix protein fibronectin1 (fn1) co-localized with the tip of the processes (Figure 4 (b) right column, second row). Tuj1 (β3 tubulin) staining for early neurons revealed groups of interconnected cells (Figure 4 (b), right column, bottom row). Figure 4 (d) shows staining for Stat3 protein in the nucleus of the GFAP-expressing astrocytes. Stat3 activates Gfap expression [78, 79]. qPCR analyses showed that NSC morphological changes were accompanied by a significant transcriptional upregulation of the genes encoding for *fnl, stat3, vimentin, gfap* and also of the neuronal cadherin, *cadherin-2 (Cdh2)* (Figure 4 (c)).

EVs^{CHP} produced by choroid plexus primary cells had similar effect on NSCs as EVs^{Z310}. Both induced cellular network formation and cell differentiation (Figure 9 (c, d)). Our data suggest that EV^{Z301} can substitute for primary culture-derived EVs.

Since this study focusses primary on the early response of NSC to EVs, the expression data the inventors present cover, for the most part, the first 24h after adding EV^{Z310}. As discussed below, during this period the effect of the EVs on cell proliferation is very limited (Figure 11 (c-e)). The percentage of cells expressing Tuj1 or GFAP is rather small by 24h but by 48h both proteins show a modest increase, at least, when a high dose of EV^{Z310} was added. After 24h the fraction of Tuj1 or GFAP expressing cells was 0.5% and 10%, respectively (304 µg/ml; Figure 11 (a, b)). At 304 µg/ml of EVs, and 48h of incubation, these percentages increased to 5% (Tuj1) and 12-15 % (GFAP), respectively. Previous work [10] found that after 7 days of incubation with choroid plexus secretome, the corresponding percentages were 15% for neurons and 80% for astrocytes. Evidently, providing the entire choroid plexus secretome for a longer time leads to more pronounced differentiation of NSC than seen with a short treatment with Z310-derived EV^{Z310}.

Flow cytometry was used to quantify the expression levels for GFAP and Stat3 proteins after coincubation of NSC^{tz} or NSC^{SVZ} with increasing doses of EVs (Figure 5 (a, b)). The fraction of GFAP/stat3 ⁺ cells increased with increasing amounts of EVs^{Z310} (assessed by total protein content) added to the culture. When using EV^{MEF}, the fraction of GFAP/stat3 ⁺ cells remained at control levels (Figure 5 (a, b)). The flow cytometry data are fully consistent with transcript quantification by qPCR. Levels of *gfap, fnl* and *stat3* expression showed a dose dependent upregulation upon EV^{z310}addition. Expression reached a saturation (Figure 5 (c)) and the 50% effective dose (ED₅₀) was in the range of 50-70 µg/ml EV protein.

CSF from the cisterna magna, and the choroid plexus secretome induced proliferation of neural stem cells [10]. This research as well as work by other authors [11] focusses on NSC treatments with the inducing agent for 7 days, which is well beyond the time it takes to form cellular networks in the assay. Using an MTT or Trypan Blue proliferation assay, the inventors found that the EV^{Z310} have a small effect on NSC proliferation. Proliferation was increased by 10-15 percent within 24h, depending on the source of the NSC and the concentration of EVs (Figure 11 (c, d)). Adding EV^{MEF} had no effect on NSC proliferation. Similar data were obtained with the Trypan Blue assay (Figure 11 (e)). The inventors conclude that the main effect of EVs is a cell shape change and cell network formation and, within the treatment time of <48h, not a massive increase of cell proliferation.

### Efficient dry-drop assay to study the differentiation of NSCs

It is still considered challenging to purify EVs by centrifugation. A general recommendation is that EVs purified by differential centrifugation should be further purified on a density gradient, which separates vesicles according to their floatation speed and equilibrium density into vesicle subpopulations and also removes contaminants [56, 74]. Therefore, the 100K EV^{Z310} pellet (Figure 2 (a)) was suspended in iodixanol and subjected to iodixanol density gradient centrifugation (Figure 6 (a)). After the centrifugation, the gradient was partitioned into nine fractions. Fraction 6 was an opaque band that could easily be seen and hence be recovered as a single fraction. In this fraction, nanoparticle tracking analysis revealed a high concentration of particles (red lines in Figure 6 (b)) in a size range of 100 to 150nm with smaller peaks at 50nm and 220nm. Fractions 2 and 8 contained mostly particles >150nm. Thus, this method provides an enrichment of particles in the 50 to 150nm range. All nine fractions were analyzed by Western blotting (Figure 6 (c)). Clathrin was detected in fractions 4-9, flot2 in fractions 4-6 and both had maximum intensity in fraction 5. Fn1 and alix were detected only in fractions 4-6 and showed highest levels in fraction 6. Annexin2 was detected in fractions 3-7, and β-actin in fractions 6 and 7. A comparison of the marker composition and particle size of the various fractions with the composition of the starting material showed that the iodixanol gradient further separated the EVs of the 100K pellet into biochemically diverse subpopulations of EVs.

To efficiently examine multiple EV fractions for their ability to induce differentiation of NSC^{tz} and NSC^{SVZ} cultures, the inventors developed a material saving "dry-drop" activity assay. The dry-drop activity assay is an example for immobilized EVs as described herein. A 1µl drop of each EV fraction (protein concentration 76µg/ml) was applied to the bottom of each chamber of an 8-chamber tissue culture glass slide and left to dry for 15 minutes. Then, 200µl of the NSC cell suspension (200,000 cells per ml) were added to the chambers and the slides were incubated for 24h in a tissue culture incubator (Figure 7 (a)). The organized network of attached NSC^{tz} or NSC^{SVZ} was readily visible and did not extend beyond the peripheral boundary of the dried drop (Figure 7 (b, d)). The EVs from the 100K pellet and from fraction 6 provided excellent support for NSC attachment, network formation and robustly showed many attached cells that were GFAP-positive (top two rows in Figure 7 (d) and Figure 7(e) first and third bar). This agreement means that EVs from the 100K pellet obtained by differential centrifugation and those in fraction 6 of the density gradient have a very similar NSC differentiation capacity, but fraction 6 of the iodixanol gradient was biochemically purer than the initial 100K pellet (Figure 6 (c)). This additional purification will help in the eventual purification of the active factors. Note that NSC attachment, network formation and development of the GFAP-positive astrocytes was less distinct for fractions 2 and 8 (Figure 7 (d) rows 3 and 4). The rest of the fractions were such that they lead to cell attachment only very sparsely and therefore only a few NSCs differentiated to astrocytes.

The inventors also used the dry-drop assay to determine, whether the differentiation activity is merely co-purifying with EVs or is intrinsic to the vesicle. 100KP EVs were treated with proteinase and nucleases as previously described (Materials and Methods) [82]. A Western blot showed that Flot2 was proteinase K resistant, as expected, because this protein is contained in the interior of EVs (Figure 7 (c)). Figures 7 (d) (row 5) and Figure 7 (e) show that adding proteinase and nucleases to the EVs, had little effects on the percentage of the GFAP positive cells developing within the drop. These results indicate that EVs themselves and not a spurious contamination in 100K pellet is causal for the effect of EVs on NSC network formation and differentiation. The inventors also examined EV^{MEF} in the dry-drop assay (Figure 7 (d) bottom row and 7 (e)). Consistent with the results shown above (Figure 4 (a) and Figure 5 (a, b)), EV^{MEF} from the 100K pellet had no detectable activity in the dry-drop assay. When slide chambers containing EV^{Z310} drops were stored at -20°C for a week, there was no reduction in NSC attachment, network formation and differentiation to of GFAP-positive astrocytes. Thus, the dry-drop assay provides a convenient method that allows an economic use of EV preparations.

### Discussion

The interconnected, CSF-filled cavities of the ventricular system (Figure 1) with their motile cilia-powered transport machinery [83], allow a targeted distribution of a variety of substances inside the brain. Many substances found in the CSF originate from the secretory epithelium of the choroid plexi that reach into the ventricular cavities. This secretome comprises metabolites, hormones, proteins, EVs, etc. In Example 1, the inventors focus on two likely EV targets, the NSC niche of the subventricular zone [41, 43] and the NSCs that reside in the tanycyte region [45, 46]. Both niches are in contact with CSF and hence in contact with EVs. The inventors established an assay in which EV^{Z310} secreted by the Z310-choroid plexus cell line are brought into contact with NSCs obtained from neurospheres that were generated either from the subventricular zone or the tanycyte region. NSC^{SVZ} and NSC^{tz} formed, within 24h, complex cellular networks that begun to express neuronal (Tuj-1, *Cdh2*) and astrocytic markers (GFAP, *Gfap*)*.* The differentiation-inducing activity resided in EV^{Z310} with a diameter of 50 to 150nm that were purified by differential centrifugation and/or iodixanol density gradient centrifugation. The activity contained in EV^{Z310} was resistant to DNase, RNase and proteinase K treatment, suggesting that the inducing activity was an EV component protected by the EV bilayer membrane and was not a contaminant co-purifying with the EVs. It should be recalled that the NSC^{SVZ} used in this study are a mixture of quiescent, active NSCs and transit-amplifying cells that differ in both, expression levels of marker genes and the rate of proliferation [10]. In the case of NSC^{tz} there is presently no evidence for the existence of such subtypes. The inventor's neurospheres may not fully replicate a neural stem cell niche because they are deprived of the influence of other niche cells [43, 84]. Nevertheless, neurospheres have been effectively used in many studies investigating the process of NSC differentiation [10, 40, 45, 46].

The inventor's use of a choroid plexus cell line and of primary culture has some limitations. Choroid plexus is the main producer of CSF but not the only one [5]. CSF-born EVs also originate e.g. from ependymal cells that form the walls of the ventricles. Such EVs are obviously missing in this preparation. Z310 cells are used as a surrogate for choroid plexus and they are derived from adult choroid plexus tissue (see references in Materials and Methods). In the adult animal, the composition of CSF, and by implication the cargo of CSF-born EVs will depend on physiological state of the organism [20, 55, 85, 86]. It is unlikely that the Z310-derived EVs reflects this complexity in full. Nevertheless, the inventors were able to isolate a biological activity (EVs^{Z310} and EVs^{CHP}) that rapidly induces cell networks from two types of NSCs.EV^{CHP} was as effective in this process as EV^{Z310}, so the Z310 cells can be use as substitute source for choroid plexus-born EVs. This activity purifies over multiple centrifugation steps and yields a distinct EV fraction containing vesicles in 50 to 150nm range. This simple purification scheme can readily be extended to a whole spectrum of other types of EVs isolated from different cells (astrocytes, ependymal cells) and from other brain tissues. The high-throughput dry drop assay according to the disclosure offers a test bed for cellular responses such as the formation of cellular processes or the expression of particular marker genes.

EV^{MEF} secreted by mouse embryonic fibroblasts had only a minor effect on NSCs. Cells formed small aggregates but never any of those cellular networks seen after treatment with EV^{Z310} and EV^{CHP}. In addition, none of the neural/glial differentiation markers were induced by EV^{MEF}. EV^{Z310} and EV^{MEF} showed marked differences in their protein composition. This difference might shed light on the factors that evoke cell differentiation. The most significant difference was the presence of ~250 "membrane" proteins in the EV^{Z310}. String analysis of possible protein-protein interaction revealed interacting proteins involved in "neurogenesis", "cell differentiation", "membrane trafficking and organization".

Slit homolog 2 protein (Slit2), which is one of the proteins identified only in EV^{Z310} cargo by mass spectrometry and Western blotting, could contribute to the formation of cell processes that characterize the cell network. Slit proteins serve as repulsive axon guiding molecules *via* Slit-Robo signaling and it was suggested that choroid plexus is a source of Slit [49, 76, 77]. Synaptogenesis is required for proper neuron function and the inventors identified several synaptic proteins in EV^{Z310}, such as synaptotagmin1, and -2, and the synaptic vesicle membrane protein VAT-1. Synaptotagmins are calcium sensors participating in triggering neurotransmitter release at the synapse and also play a role in synaptic vesicle trafficking.VAT-1 is involved in synaptic vesicle transport.

EV^{Z310} induced astrocytes, that expressed a characteristic marker, GFAP. Gfaptranscription is up regulated by the transcription factor Stat3 and the transcriptional activator Brg1 (also known as Smarca4) [78, 79]. Stat3 and Smarca4 were identified by MS in EV^{Z310} and confirmed by Western blotting. Stat3 also regulates cdh2 expression via Jak/Stat pathway [87] and the qPCR data herein showed that *cdh2* expression was upregulated upon EV^{Z310} addition. The EV^{Z310} also carry integrins and small GTPases, such as Ras Homolog Gene Family Member A (Rhoa) and RAC, which also regulate cdh2 expression [88]. *Vimentin* is also induced by Stat3 signaling [89]. While GFAP and Cdh2 were not identified in EV^{Z310}, these vesicles contain vimentin. There is also vimentin in NSCs prior to their exposure to EV^{Z310}. In this case, vimentin seen after EV treatment could be a mixture of cellular and EV-derived vimentin. In the assays, the inventors observed intense fn1 staining at or near the tip of the cellular processes. Such staining may arise from fibronectin1 contained in EV^{Z310} that locally aggregate. Note however, that cells also express and secret endogenous fn1 that could also be present at the tip, either inside or outside of the cell. Other adhesion proteins and scaffolding proteins (integrins, tetraspanins) are present in EV^{Z310} and could act as guidance cues that control the outgrowth of the nascent NSC processes. Growing tips of axons can rapidly detect and react to the local guidance cues [90]. Such guidance cues can also initiate local synthesis of different proteins in the distal part of axons [91]. In this case, EVs may not be taken up by the NSCs, but are acting as exogenous guidance posts. There are precedents for cellular responses to EVs that do not require EV internalization [92, 93]. EVs may collapse on the cell surface and release the protein cargo that subsequently interacts with cell surface receptors [94].

Since the purified EVs are heterogeneous, only a subset of the EV^{Z310}-specific proteins would be present in any given EV. EVs with a diameter of 150nm have a volume that is nearly fifty times greater than that of a synaptic vesicle (40nm) that contains about 160 protein molecules [95, 96]. Hence, a single EV could contain thousands of protein molecules. Assuming that the NSC targets incorporate multiple EVs, collectively such vesicles could confer entire signaling pathways to the target cells. In developmental biology, and the change in cell morphology is a developmental process, multiple signals converge on a single tissue. For example, the developing limb of vertebrates is controlled by a combination of growth- and transcription factors [97]. In this case, the signals are not packaged in EVs, but are soluble local mediators. This is different for the NSC niches in the adult brain where signals must travel several millimeters even in mice and much farther in larger brains. A long-range transport of the multiple signals would benefit from an enclosure of signals in a carrier vesicle.

Induction of *gfap, stat3* and *fn1* expression was dependent on the amount of EVs added to the culture. Such dose dependence and the accompanying saturation suggest that EVs are involved in a receptor-mediated process. This could be an interaction with cell surface receptors or receptor-mediated EV uptake [98]. Work by others show that target cells internalize EVs in a time- and dosage-dependent manner [38, 99]. Bonsergent *et al.* suggested that EV uptake is a low-yield process, and only about 30% of internalized EVs were capable of delivering their contents. In their study, a dose response was observed but saturation could not be reached, even when a dose >100µg/ml (doses refer to EV protein content) was used [99]. Song *et al.* observed EV uptake by mouse embryonic stem cells was dose- and time-dependent over a one-day period. EV internalization was detected at an EV level of 2×10⁹ but increased significantly, when the concentration was fivefold higher [38]. Sharma *et al.* showed that EV addition rescued the decrease of the total cell number and neurons in a loss-of-function neural model in dose dependent manner (47.5-190µg/ml) [37]. EV^{MEF} were inactive in the assay herein, even when a very high dose (608µg/ml) was provided. The cause for this may be that the NSC target and fibroblast-derived EV^{MEF} are not compatible. The combination of choroid plexus-derived EV^{Z310} and NSCs, however, reproduces a naturally occurring situation. At a dose of 76µg/ml protein (approximate the ED₅₀) a dry drop contains 1.6×10⁶ EVs. A typical dry drop contains∼5500 NSCs. Thus, there are ~800 EVs per cell. As pointed out above, EVs are heterogeneous and only a fraction may be acting in the network forming process. Thus, a rather small number of EVs may be sufficient to evoke cell differentiation.

Adding EV^{Z310} suspended in buffer to the NSCs or, alternatively providing EVs as a dried down drop induced network formation. Even when the dry drops are kept at -20°C for a week, they were still very active. This indicates that the activity is stable in the EV for a significant length of time. Thus, the dry-drop assay according to this disclosure allows testing of diverse EVs and multiple responsive cells in a short time and with minimal amounts of testing material. Moreover, this assay is high throughput. This allows a more efficient purification of EV proteins and other factors (e.g. miRNAs [15, 40]) that mediate the effects that EVs exert on their target cells.

### Materials and Methods

A list of the antibodies and primers, which were used, is provided in the Supplementary information.

### Cell culture

Immortalized Z310 rat choroidal epithelial cells were used as a choroid plexus substitute. Zhang *etal.* established the Z310 cell line in 2002 [58], starting with choroid plexus tissue collected from Sprague-Dawley rats (4-6 weeks old, both sexes). Such Z310 cells have been used in multiple studies, including those in which the choroid plexus primary culture and Z310 cells were compared side by side and were shown to be similar [59, 60]. Based on this, various groups used Z310 cells as a choroid plexus primary culture substitute [39, 61, 62]. Mouse embryonic fibroblasts (MEFs), were isolated from 13.5-day-old C57BL/6N mouse embryos. The choroid plexus contains a small number of fibroblasts (in addition to immune cells and perivascular cells) [63] and thus using EVs from a cell type that also occurs in the choroid plexus, provides a control. After the removal of head, liver and heart, embryos were cut into small pieces which were digested with 0.05% trypsin-EDTA (Gibco) for 30min at 37°C. The trypsin was inactivated by addition of culture medium (see below), the cell suspension was pipetted repeatedly with a P1000 pipette and centrifuged for 5min at 200xg. Cell viability and count were determined by flow cytometry. Both, Z310 and MEF cells were grown in DMEM supplemented with 10% FBS, 1x GlutaMAX and 50 units/Penicillin/Streptomycin (all Gibco) in a humidified incubator with 95% air, 5% CO₂ at 37°C. They were passaged twice a week and were regularly checked for Mycoplasma contamination.

### Purification of EVs by differential centrifugation

Cells with a confluency of 70-80% were washed twice with PBS. To exclude carrying along EVs from serum, Z310 cells and MEFs were maintained for two days in serum-free conditioned medium. The medium was then aspirated without disturbing the cells and subjected to differential centrifugation, as described by Kowal *et al.* [56] (Figure 2 (a)). The percentage of live cells at the time of harvesting was determined by Trypan Blue (Sigma). The cell pellet was collected and resuspended in 0.4% (v/v) Trypan Blue. Dead and live cells were counted using a hemocytometer (Nexcelon, Bioscience). > 90% of the cells were viable, e.g. in the case of Z310 cells viability was 92.5± 2.1%. Centrifugation steps (Figure 2 (a)): 300×*g* for 10min to remove cells and cell debris, then at 2 000×*g* for 20min (Eppendorf 5702R) and 10 000×*g* for 40min (Eppendorf 5417R) to remove larger vesicles. The 10K supernatant was concentrated at 0°C using Vivaspin (300 000 MWCO, Sartorius, Göttingen, Germany). This concentrated supernatant was centrifuged at 100 000×*g*, for 60min (Sorvall WX-Ultra 80, Thermo Fisher Scientific, USA) in a TH-660 rotor (Thermo Fisher Scientific). The resulting pellet was resuspended in ice cold PBS and centrifugation was repeated under the same condition. The resulting pellet was resuspended in PBS and stored at -80°C.

### Size distribution and particle concentration

EVs size and concentration were determined using a nanoparticle tracking analyzer (Nanosight NS300, Malvern Panalytical, Kassel, Germany). Five 60s videos with more than 200 tracks were taken per sample and analyzed using the Nanoparticle Analysis software. Results represent the mean of all 5 measurements per sample.

### Transmission electron microscopy analysis

The 2KP, 10KP and 100KP pellets were visualized by negative staining. Samples were adsorbed on the surface of a carbon-coated copper grid and stained with 1% uranyl acetate. Grids were imaged by transmission electron microscopy, using a Talos L120C instrument (Thermo Fisher Scientific, Netherlands) equipped with a CMOS camera.

### Western blotting

80% confluent Z310 cells were washed twice with PBS and lysed in lysis buffer (25mM Tris-HCI pH 7.4, 150mM NaCl, 1mM EDTA, 1% NP-40 und 5% Glycerin) according to the manufacturer's protocol, supplemented with a protease inhibitor cocktail (Halt^{™} Protease and Phosphatase inhibitor cocktail) (all Thermo Fisher Scientific). The protein concentration of each pellet and the cell lysate was determined using a BCA kit (Pierce^{™}, Thermo Fisher Scientific). Equivalent quantities of proteins in pellets or in cell lysates were mixed with SDS loading buffer (1M Tris pH 6.8, 4% SDS, 20% glycerol, 10% mercaptoethanol, 0.2% bromophenol blue) heated at 95°C for 10min and loaded on a 4-20% polyacrylamide gradient gel (Bio-Rad Laboratories). Subsequently, samples were transferred to a PVDF membrane (Bio-Rad Laboratories). Membranes were blocked with 5% non-fat dry milk powder in TBST (0.1% Tween 20 in Tris-buffer saline) for 30min, followed by overnight incubation with the appropriate primary antibody, at 4°C. After washing, membranes were incubated with the secondary antibody for 1h at RT. All antibodies were diluted in blocking buffer according to the manufacturer's recommendations. After washing, membranes were developed using ECL Super signal West Femto (Thermo Fisher Scientific), protein bands were visualized on an Image Quant LAS 4000 imager (GE Healthcare Bio-science AB, Sweden).

### Sample preparation for LC-MS/MS

Isolated EVs were lysed by adding an equal volume of lysis buffer (4% [wt/v] SDS 1mM EDTA 100mM Hepes, pH 8) supplemented with a protease inhibitor cocktail (cOmplete, Roche) and sonicated in a BioRuptor (Diagenode) with 30s on-and-off cycles for 5min. Proteins were reduced, alkylated and purified as described [64, 65]. The digestion was accomplished overnight in 50mM ammonium bicarbonate buffer using trypsin (Promega)-to-protein ratio (wt/wt) of 1:20. Vacuum-dried samples were re-dissolved in 2% (v/v) acetonitrile 0.1% (v/v) trifluoroacetic acid in water and subjected to LC-MS/MS analysis.

### LC-MS/MS acquisition

Samples were analyzed using an Orbitrap Fusion Tribrid or a Q-Exactive HF-X (both Thermo Fisher Scientific) mass spectrometer interfaced *via an* LC set-up as described [64]. Peptides were separated using 118min linear gradients ranging either (A) from 4 to 5.6, 16, 25.6, 40, 90% and back to 4% (v/v) acetonitrile over 3, 57, 30, 16, 6 and 6min, respectively (Orbitrap Fusion) or (B) from 1.8 to 4, 33.6, 72 and back to 1.8% over 5, 101, 6, and 6min (Orbitrap HF-X). The mass spectrometer was operated in a data-dependent acquisition mode. A survey scan was performed at 120.000 resolution and 50ms maximum injection time (MaxIT). 30 top-abundant peptide precursors (or keeping a constant duty cycle of 3s) were selected for sequencing using a 1.6 *m*/*z* isolation window. Precursor ions were fragmented in an HCD cell and the normalized collision energy setting of 28% or 30%. MS/MS spectra were acquired in Orbitrap operated using 15000 resolution and 54ms MaxIT or 30000 resolution and 120ms MaxIT. Precursors were excluded from repeated sequencing for 30s.

### LC-MS/MS data analysis

Raw MS data were processed by MaxQuant (version 1.6.2.10) [66] using default parameters. MS/MS spectra were searched against *Rattus norvegicus* and *Mus musculus* canonical protein sequences from Uniprot [67] (December 2020, 29940 and 17051 sequences, respectively). Protein groups with at least two razor or unique peptides were considered as identified. IBAQ values [68] attributed to the same gene name but several protein groups due to different origin (rat/mouse) were summed in order to achieve a single quantitative value per gene. In the following, only genes quantified in at least two EV^{Z310} replicates were considered for further analysis: iBAQ values were log2-transformed, normalized by median-subtraction, and missing values were imputed by random sampling from a normal distribution centered at the 5%-intensity quantile and a standard deviation equal to a half of the standard deviation of each replicate's intensities. *Limma* R package [69] was used to test for differential protein abundance in EV^{Z310} and EV^{MEF}. Candidates satisfying the criteria of q-value < 0.01 [70] and an absolute log₂FC > 1 were considered as differentially expressed.

### GO pathway enrichment analysis and protein-protein interaction network functional enrichment analysis

Proteins identified in EVs by MS were analyzed by DAVID (Database for Annotation, Visualization and Integrated Discovery). Proteins identified in EVs^{Z310} belonging to the cellular component category "vesicle" were used as input for STRING (protein-protein interaction networks functional enrichment analysis). To visualize functional interactions, MCL clustering was used. To contrast EV^{Z310} and EV^{MEF} proteomes, the GO category "cellular component" was selected after functional annotation clustering. For each cellular component, the Benjamini-corrected p value was displayed as bar graph, from the highest-ranking to lowest one. Analysis revealed main differences in sub-category "membrane". Proteins present EV^{Z310} but not in EV^{MEF} were used as input for the subsequent STRING analysis.

### Data availability

The mass spectrometry data have been deposited on the ProteomeXchange Consortium via the PRIDE [71] partner repository with the dataset identifier PXD031862.

### Iodixanol density gradient centrifugation

Flotation in an iodixanol gradient was performed as described in Crescitelli *et al.* [57] with some modifications. The 100K pellet obtained after differential centrifugation was resuspended in 1ml of 40% iodixanol (v/v) (OptiPrep^{™} Density Gradient Medium, Sigma) in PBS and bottom loaded. For the discontinuous iodixanol gradient equal volumes of solutions of 30, 20 and 10% iodixanol were layered on the top of the sample (Figure 6 (a)) and centrifuged in a 4ml tube (Beckman Coulter, 328874) at 180 000×g for 19h, at 0°C (Sorvall WX-Ultra 80, Thermo Fisher Scientific) in a TH-660 rotor (Thermo Fisher Scientific). Fractions of 500µl from top to bottom were collected from the tube. An opaque band of EV was recovered in fraction 6.

### NSC dissection and neurosphere expansion

Animal experiments were carried out in accordance with regulation of the Office of Consumer Protection and Food Safety of Lower Saxony and conformed to German Laws on Animal Welfare. To isolate NSC from SVZ of the lateral ventricle and the tanycyte region of v3V, 6-8 weeks old male mice of the strain C57BL/6N were sacrificed. To isolated stem cells from the tanycyte area of the v3V, intact brain was placed in a brain matrix (Plano, GmbH) submerged in HBSS (Hank's balanced solution with Ca and Mg, Gibco) supplemented with glucose (0.45%). Two razor blades were used to cut 3mm thick coronal section. Under the stereomicroscope, v3V was isolated and the tanycyte area was separated, using tungsten needles (for details see [3]). The NSC from SVZ of the lateral ventricle were isolated according to Walker and Kempermann [72]. SVZ and tanycyte tissues were minced with a scalpel blade, transfered to pre-warmed 0.05% Trypsin-EDTA and incubated for 10min at 37°C with mixing every 3 minutes. Then the tissue was dissociated by gentle pipetting. The enzymatic reaction was stopped by mixing with pre-warmed trypsin inhibitor (0.125mg/ml containing DNase I, 0.01mg/ml), then spun down for 5min at 300xg. The pellet was resuspended in medium and spun again. The final pellet was resuspended in complete growth medium (Neurobasal Medium with supplement, see below) and passed through cell strainers (first 70µm, then 40µm) and placed into an ultra-low attachment surface 6cm polystyrene dish (Corning, USA). After 7 days, neurospheres were visually inspected. The growth medium was changed every second day.

### Dissociation of neurospheres

The NSC were grown in Neurobasal Medium supplement with 2% B27,1% N2 supplement, 1x GlutaMAX, 50units/ml Penicillin/Streptomycin (all Gibco), 20ng/ml purified mouse receptor-grade epidermal growth factor (EGF), and 20ng/ml recombinant bovine fibroblast growth factor (FGF-2) (both Peprotech). Neurospheres were passaged by centrifugation at 300xg for 5min. The pelleted spheres were resuspended in prewarmed 0.05% Trypsin-EDTA and incubated at 37°C for 5min, then an equal volume of trypsin inhibitor (0.125mg/ml containing DNase I, 0.01mg/ml) was added. The resulting individual cells were centrifuged for 5min at 300xg, and the cell pellet was resuspended in fresh medium. The cells were counted using a hemocytometer and seeded at a concentration 2×10⁵ cells/ml for all experiments.

### Choroid plexus primary culture

To isolate choroid plexus from the lateral ventricle, 6-8 weeks old male mice (C57BL/6N) were sacrificed. Isolation and culture of primary mouse choroid plexus was performed essentially as previously described [73] at ZT 4. Choroid plexus was removed from the lateral ventricle and rinsed in HBSS supplemented with glucose. Thereafter, the HBSS was aspirate, pre-warmed Pronase (2mg/ml) was added, followed by incubation at 37°C for 5min. Growth medium was added to stop digestion, the cells were centrifuged for 5min 300xg, the supernatant was removed and the cell pellet was suspended in fresh medium. Another step of centrifugation followed (5min 300xg). The final pellet was suspended in complete DMEM growth medium (10% FBS, 50 units/ml Penicillin/Streptomycin, 10ng/ml EGF) supplemented with 20 ·M cytosine arabinose and plated on poly-D-lysine coated plates which were kept in humidified incubator (95% air, 5% CO₂) at 37°C. The medium was changed every 48h until cells became fully confluent.

### Differentiation assay with EV in suspension

NSC in Neurobasal Medium were seeded on an appropriate size petri dish (Thermo Fisher Scientific, Denmark, 153066) and EVs from the 100K pellets, resuspended in PBS were added. Amounts of added EVs, were normalized to their total protein concentration, as determined by a BCA assay. The NSC were cultured in a CO₂ incubator for 24h and bright field images of live cells were taken. After photography the NSCs were processed for immunohistochemistry, qPCR or flow cytometry.

### Differentiation assay using EV dry drops

EV were applied on the surface of the 8-chamber tissue culture slide (Corning, USA, 354108) as a 1µl drop. The drop was dried (approximately 15min) and then NSC (2×10⁵ cells/ml) in culture medium were added and chambers were placed into a CO₂ incubator. 24h later images of the chamber wells were taken. For the testing of stability of dried-down EVs, drops were applied to the wells of the 8-chamber slides which were then kept for 7 days at - 20°C.

### Protease and nucleases protection assay

Proteinase K (Bioline) was added to a final concentration of 10µg/ml to the EVs. Samples were incubated for 30min on ice. Proteinase K was inactivated for 10min by incubating with tetrapeptidyl chloromethyl ketone at a final concentration of 10µg/ml (EMD Millipore) on ice. Then DNase (2U final concentration) was added and the sample was incubated at 37°C for 30min. The reaction was stopped with DNase inhibitor (2U final concentration) (DNA free kit, DNase treatment and removal, Invitrogen, Thermo Fisher Scientific). Then RNase A (40U final concentration, 37°C, 30min) (Qiagen) was added and inactivated by RNase inhibitor (40U/ml) (Invitrogen, Thermo Fisher Scientific). Samples were cleaned using an Amicon ultra centrifuge unit (Merck, Millipore) with PBS. The resulting EV^{Z310} were then added to NSC^{tz} or NSC^{SVZ} for assessing their differentiation-inducing capacity.

### Immunofluorescence

Cells on slides or in Petri dishes were fixed with 4% formaldehyde, washed with PBS (0.1% Triton) and blocked for 30min (0.25% Triton and 0.25% BSA in PBS) at room temperature. Then cells were incubated overnight at 4°C with primary antibody. After the three washing steps with PBS (0.1% BSA, 0.1% Triton), incubation with secondary antibody for 1h at room temperature followed. After five washes coverslips were mounted with mounting medium with DAPI (Vectashield, Vectorlabs) and images were acquired using a Leica DMI 6000B fluorescence microscope, with LAS X software (Leica Microsystems, Germany).

### RNA preparation for qPCR analysis

Total RNA was extracted from cells using Trizol reagent (Ambion) or RNeasy kit (Qiagen) according to the manufacturer's instructions. First genomic DNA was removed and then 1µg RNA was reverse transcribed by iScript gDNA Clear cDNA synthesis Kit (Bio-Rad Laboratories) according manufacturer's instructions. *Gapdh* was used for normalization. qPCR was performed with SsoAdvanced Universal SYBR Green Supermix (Bio-Rad Laboratories) on CFX96- Real Time system (Bio-Rad Laboratories, Germany). For primers see Supplementary information.

### Flow cytometry

NSC were analyzed after 24h of culturing in the presence of EVs. Single-cell suspensions were obtained after accutase-mediated (ESGRO Complete^{™} Accutase, EMD Millipore) dissociation. Cells were then fixed with 4% formaldehyde for 15min in the dark at room temperature. Washing and blocking steps were followed by overnight incubation with primary antibody at 4°C. After the washing steps, incubation with corresponding fluorescence secondary antibody for 1h at RT followed. The cells were analyzed using a flow cytometer BD Accuri C6 (BD Bioscience, USA) and BD Accuri^{™} C6 software.

### Proliferation assay

NSC were seeded on 96-well flat-bottom tissue culture plates of good optical quality (Falcon) at a density of 2×10⁵ cells per ml. Cells were cultured for 24h in Neurobasal Medium (control) or in medium containing an increasing concentration of EV^{Z310} or EV^{MEF}. Thereafter, MTT solution was added and all subsequent steps were performed according manufactures' instructions (Merck, Colorimetric (MTT) kit for cell survival and proliferation). Absorbance was measured with an ELISA plate reader (Infinite M2000 Pro Tecan) with a test and reference wavelength of 570nm and 630nm, respectively. For the Trypan Blue assay, NSC were seeded on a 6cm Petri dish at a density of 2×10⁵ cells per ml and cultured in increasing concentration of EV^{Z310} or EV^{MEF} for 24h. Trypan Blue staining was performed according to manufacturer's instruction (Sigma) and the number of cells was determined with a hemocytometer.

### Example 2 - EVs from various cell types may be dried on various surfaces

EVs produced by three different cell lines were tested by the inventors. Three (Z310 cells [an immortalized choroid plexus epithelial cell line]) and MEF (mouse embryonic fibroblast) and choroid plexus primary culture were described in the Example Section as "Example 1". A third tested cell line - from which EVs were purified - were NIH 3T3 cells (mouse fibroblast). A suitable yield of EVs was obtained and hence they were tested for their ability to induce differentiation of NSCs.

The inventors also established primary cultures from Macaque choroid plexus. Finally, the inventors tested EVs isolated from NSCs. The purification of EVs from these additional cell types were those described for Example 1.

Importantly, these sources yielded sufficient EVs that could be tested in the NSC assay. EVs from primary choroid plexus cultures exhibited high differentiation capacity. NIH 3T3 EVs had minor activity while NSC- and MEF-derived EVs were inactive in the said assay.

### Further recipient cells

As described for Example 1, cells that respond to EV exposure were NSCs from the stem cell niches of the third ventricle and the lateral ventricle. The inventors also examined the response of mouse embryonic stem cells (ES cells, C57BL/6N) to EV treatment. These cells were not responsive to any of the EVs purified. Responsiveness was assayed for morphologic changes such as formation of processes or change in cell type. Additionally, qPCR analysis was carried out in which the inventors quantified the induction of expression of genes such as *gfap, klf4, nanog, nestin, sox2, oct4, pax6* and *vimentin.* None of the mentioned genes were induced in ES cells.

### Surface coating

Apart from applying EVs to the dish surface (Example 1), the inventors tested surface and/or other coatings of different forms. Fibronectin, laminin, poly-L-lysine coating were tested with regard to sustaining growth and differentiation of NSCs. Coating in combination with EVs did accelerate the differentiation process. However, with coating only, the percentage of the differentiated cells was altered (e.g. fewer astrocytes). Coating together with EVs drops can be tested for the possible enhancement of EV's effect beyond the effects seen with EVs only. Coating and EVs combination should be chosen according their physiological relevance.

### Variation of drop size

In Example 1, a one (1) microliter (µl) drop was deposited and approximately 40 000 NSCs were added to the well (area of 0.7 cm²). Since there was a rapid and robust response just in the area of the drop, a single drop was sufficient to get statistically significant differences between experimental and control data. This conclusion comes from comparing drop size ranges from 1 to 20 microliters (µl). Given that the response to EVs were already obvious with the smallest volume, the inventors homed in on this material-saving volume of 1 microliter (µl). One can also augment the response by pipetting multiple small drops. The inventors found that the latter approach also works if one compares drops of EVs isolated from different sources. For example, EVs produced by immune cells, stem cells could be tested side-by-side in the same well, as an alternative to placing drops of different EVs in individual wells.

An important alternative to drop EV deposits is a complete coating of the bottom of the well with EVs (as depicted in e.g. Fig. 1A and B). This is advisable to do for biochemical assays in which the entire well content is subjected to analysis ("bulk assay"). For example, if EV-mediated changes in the transcriptome/proteome is investigated, then the wells with all the responding cells are completely extracted and this will yield enough material for analysis. By contrast, in a "cell-based assay" looking e.g. for changes in cell morphology or expression of a new antigen (detected e.g. by immunohistochemistry) small drops are sufficient as the outcome can be assessed by microscopy.

### Other purification methods

The inventors tested sucrose gradients but in the presence of sucrose, EV immobilization is slightly impaired and responding cells can be affected by sucrose. Thus, steps to remove the sucrose (dialysis or ultracentrifugation steps) may be added to improve EV immobilization.

EVs can be isolated by methods other than ultracentrifugation. This includes size exclusion chromatography, immune capture with magnetic beads, ultrafiltration or flow cytometry. There are commercial EV purification kits based on precipitation, ultrafiltration, size exclusion chromatography or affinity capture. The inventors found that immune capture and size exclusion chromatography do result in suitable EV preparations that can be immobilized well on sterile surfaces as disclosed herein.

### Potential experiment performed with EVs from other sources using different recipient cell types

Almost every cell type, tissue, and organ produce EVs. EVs are also present in various body fluids (saliva, blood, urine, etc.). Their composition varies and changes according age, physiological condition (health-disease, pregnancy, drug treatment, etc.). All those EVs can be isolated and used for activity assessment.

Not only cell lines as responding targets can be used. Also usable are embryoid bodies, tissue explants, organoids, tumors, spheroids (3D cell aggregates that can mimic tissues and microtumors). Thus, many experiments with combination of diverse EVs and responding cells are possible. This need is served by an EV-library that is provided in a multiplex format, as presented herein (see e.g. Fig. 1A and B).

EVs produced by various cell lines or tissue could be tested for their ability to induce differentiation of various types of (embryonic) stem cells, embryoid bodies,inducible pluripotent stem cells, or organoids. For instance, EVs produced by various cells types present in stem cell niche in brain and other organs could be tested for influence on (other) stem cells or (brain) organoids.

EVs produced by cancer cell line or isolated from body fluids of patient or animals could be tested for their ability to control migration and proliferation of cancer cells.

For instance, spheroid, xenografts, tumor tissues could be incubated with immobilized EVs to assess ability of cells to migrate from such samples and also for the ability to attach and proliferate in area of EV drop. This would evaluate the ability of EVs to promote/inhibit tumor metastasis. In addition, EVs produced by immune cells could be tested for their ability to prevent this process.

### Example 3- A multi-well plate with immobilized EVs (EV library) and in vitro methods using immobilized EVs

Frequently sought by scientists are EVs produced by cells, which fit the following criteria: Such EVs need to be well characterized, provide ample amounts of EVs and are amenable to genetically manipulation. Exemplary EV producer cells can be found e.g. on Vesiclepedia (http)://microvesicles.org/) and said EV producer cells (source) include: adipocytes, astrocytoma, B cells, bone marrow mesenchymal stromal cells, brain cancer cells, breast cancer cells, bronchial epithelial cells, cardiomyocytes, colorectal cancer cells, dendric cells, embryonic kidney cells, endothelial cells, epithelial cells, glioblastoma cells, kidney cancer cells, leukemia cells, leukocytes, lung cancer cells, macrophages, melanoma cells, mesenchymal stem cells, myeloid leukemia cells, osteoblasts, ovarian cancer cells, pancreatic adenocarcinoma cells, platelets, prostate cancer cells, reticulocytes, and umbilical cord mesenchymal stem cells. Alternatively, EVs can be purified from CSF, saliva, urine, serum or plasma.

The amount of EVs can be measured according to the number of particle (EVs) or protein concentration based on the protein content in the EVs. The inventors suggest a particle amount ranging from 8×10⁸ -3×10¹⁰ (8×10⁸, 1×10⁹, 2×10⁹, 4×10⁹, 6×10⁹, 8×10⁹, 1×10¹⁰, 3×10¹⁰) per coated predetermined position, which may be the full bottom of a tissue culture well. In order to obtain said amount of EVs, the solution deposited on the predetermined position may have a protein concentration ranging from 20-200 µg/ml (20, 50, 80, 110, 140, 170 and 200 µg/ml).

In Examples 1 and 2, it is demonstrated that EVs, which are produced by MEFs do not evoke neural stem cell differentiation. By contrast, EVs from Z310 cells are very potent in inducing neural stem cell differentiation. Thus, EVs which are produced by MEFs and EVs from Z310 cells can serve as suitable quality-controls in the EV libraries (see also Fig. 1A and B, which suggests to use these EVs as positive and negative controls).

Upon storage, a 50% reduction in effectiveness relative to freshly purified EVs may still be considered a suitable response.

With immobilized EVs on surfaces different assays can be performed. For example, for the drop micro assay, the EVs are deposited as a micro/nano-drop and the resulting response is detected with the help of a microscope. So, such assays assess the response of individual cells or a small group of cells by visual inspection (see **Table 1** herein, third column). By contrast, the full bottom coated assay (e.g. in full bottom coating of a well in a tissue culture plate) involves the analysis of the complete well content of a (microtiter) plate (see **Table 1** herein, last column). Furthermore, the EVs may be used in different assays to assess, e.g. differentiation, viability, apoptosis, cell migration, proliferation, transcriptome and proteome analysis and cell development. The skilled person is aware of these general assays.

### Supplementary Information

| Primary antibody | | Source |
|---|---|---|
| Anti-fibronectin 1 | ab2413 | Abcam |
| Anti-clathrin heavy chain | ab21679 | Abcam |
| Anti-tsg101 [EPR7130(B)] | ab125011 | Abcam |
| Anti-anxa2 [EPR13053(2)(B)]-C terminal | ab185857 | Abcam |
| Anti-cd9 [2949] | ab92726 | Abcam |
| Anti-alix | ab88388 | Abcam |
| Anti-β actin HRP conjugated (8H10D10) | #12262 | Cell Signaling Technology |
| Anti-adamlO | #14194 | Cell Signaling Technology |
| Anti-grp94 | # 2104 | Cell Signaling Technology |
| Anti flotillin 2 | #PA5-81037 | Invitrogen |
| Anti-GM 130 (C-terminal), Golga2 | G7295 | Sigma- Aldrich |
| Anti-vimentin | ab24525 | Abcam |
| Anti-nestin | ab 134017 | Abcam |
| Anti-slit2 | ab 134166 | Abcam |
| Anti-transthyretin | SAB3500378 | Sigma-Aldrich |
| Anti-smarca4 | SAB4300727 | Sigma-Aldrich |
| Anti-stat3 [EPR787Y] | ab68153 | Abcam |
| Anti-tubulin beta 3, Tuj 1 | MCA2047 | Bio-Rad Laboratories |
| Anti-GFAP | ab4674 | Abcam |

| Secondary antibody | | Source |
|---|---|---|
| Goat anti rabbit IgG HRP conjugated | A24537 | Novex |
| Alexa Fluor 488 conjugated goat anti-chicken IgG | A-11039 | Invitrogen |
| Alexa Fluor 555 conjugated donkey anti-rabbit IgG | A-31572 | Invitrogen |
| Alexa Fluor 555 conjugated goat anti-mouse IgM | A-21426 | Invitrogen |

| **SEQ ID NO.** | **Primer name** | **Sequence (5'-3')** |
|---|---|---|
| 1 | Cdh2 S | TTATGCAAGACTGGATTTCCTGAAG |
| 2 | Cdh2 A | CTTGAAATCTGCTGGCTCGC |
| 3 | GFAP S | TGGCCACCAGTAACATGCAA |
| 4 | GFAP A | GTCGTTAGCTTCGTGCTTGG |
| 5 | GAPDH S | GTGTTCCTACCCCCAATGTGT |
| 6 | GAPDH A | ATTGTCATACCAGGAAATGAGCTT |
| 7 | Fibronectin S | CCTCCTGATAGTGTGGTGTCTG |
| 8 | Fibronectin A | CGTACACCCAGCTTGAAGCCAAT |
| 9 | Klf 4 S | GGCGAGTCTGACATGGCTG |
| 10 | Klf 4 A | GCTGGACGCAGTGTCTTCTC |
| 11 | Nanog S | TCTTCCTGGTCCCCACAGTTT |
| 12 | Nanoq A | GCAAGAATAGTTCTCGGGATGAA |
| 13 | Nestin S | CCCTGAAGTCGAGGAGCTG |
| 14 | Nestin A | CTGCTGCACCTCTAAGCGA |
| 15 | Oct 4 S | ATCGGACCAGGCTCAGAGGTATTG |
| 16 | Oct4 A | GTTCTCATTGTTGTCGGCTTCC |
| 17 | Pax 6 S | TACCAGTGTCTACCAGCCAAT |
| 18 | Pax 6 A | TGCACGAGTATGAGGAGGTCT |
| 19 | Sox 2 S | GACAGCTACGCGCACATGA |
| 20 | Sox 2 A | GGTGCATCGGTTGCATCTG |
| 21 | Stat3 S | GACATTCCCAAGGAGGAGGC |
| 22 | Stat3 A | CTTGGTCTTCAGGTACGGGG |
| 23 | Vimentin S | GCTTCTCTGGCACGTCTTGA |
| 24 | Vimentin A | CGCAGGGCATCGTTGTTC |

### References

1. Hladky, S.B. and M.A. Barrand, Mechanisms of fluid movement into, through and out of the brain: evaluation of the evidence. Fluids Barriers CNS, 2014. 11(1): p. 26.
2. Mirzadeh, Z., et al., Cilia organize ependymal planar polarity. J Neurosci, 2010. 30(7): p. 2600-10.
3. Faubel, R., et al., Cilia-based flow network in the brain ventricles. Science, 2016. 353(6295): p. 176-8.
4. Eichele, G., et al., Cilia-driven flows in the brain third ventricle. Philos Trans R Soc Lond B Biol Sci, 2020. 375(1792): p. 20190154.
5. Damkier, H.H., P.D. Brown, and J. Praetorius, Epithelial pathways in choroid plexus electrolyte transport. Physiology (Bethesda), 2010. 25(4): p. 239-49.
6. Cserr, H.F., Physiology of the choroid plexus. Physiol Rev, 1971. 51(2): p. 273-311.
7. Zappaterra, M.W. and M.K. Lehtinen, The cerebrospinal fluid: regulatorof neurogenesis, behavior, and beyond. Cell Mol Life Sci, 2012. 69(17): p. 2863-78.
8. Lun, M.P., E.S. Monuki, and M.K. Lehtinen, Development and functions of the choroid plexus-cerebrospinal fluid system. Nat Rev Neurosci, 2015. 16(8): p. 445-57.
9. Chau, K.F., et al., Progressive Differentiation and Instructive Capacities of Amniotic Fluid and Cerebrospinal Fluid Proteomes following Neural Tube Closure. Dev Cell, 2015. 35(6): p. 789-802.
10. Silva-Vargas, V., et al., Age-Dependent Niche Signals from the Choroid Plexus Regulate Adult Neural Stem Cells. Cell Stem Cell, 2016. 19(5): p. 643-652.
11. Lehtinen, M.K., et al., The cerebrospinal fluid provides a proliferative niche for neural progenitor cells. Neuron, 2011. 69(5): p. 893-905.
12. Kaiser, K., et al., WNT5A is transported via lipoprotein particles in the cerebrospinal fluid to regulate hindbrain morphogenesis. Nature Communications, 2019. 10.
13. Vella, L.J., et al., Enrichment of prion protein in exosomes derived from ovine cerebral spinal fluid. Vet Immunol Immunopathol, 2008. 124(3-4): p. 385-93.
14. Street, J.M., et al., Identification and proteomic profiling of exosomes in human cerebrospinal fluid. J Transl Med, 2012. 10: p. 5.
15. Yagi, Y., et al., Next-generation sequencing-based small RNA profiling of cerebrospinal fluid exosomes. Neuroscience Letters, 2017. 636: p. 48-57.
16. Murillo, O.D., et al., exRNA Atlas Analysis Reveals Distinct Extracellular RNA Cargo Types and Their Carriers Present across Human Biofluids. Cell, 2019. 177(2): p. 463-477 e15.
17. Balusu, S., et al., Identification of a novel mechanism of blood-brain communication during peripheral inflammation via choroid plexus-derived extracellular vesicles. EMBO Mol Med, 2016. 8(10): p. 1162-1183.
18. Feliciano, D.M., et al., Embryonic cerebrospinal fluid nanovesicles carry evolutionarily conserved molecules and promote neural stem cell amplification. PLoS One, 2014. 9(2): p. e88810.
19. Fame, R.M. and M.K. Lehtinen, Emergence and Developmental Roles of the Cerebrospinal Fluid System. Dev Cell, 2020. 52(3): p. 261-275.
20. Tietje, A., et al., Cerebrospinal fluid extracellular vesicles undergo age dependent declines and contain known and novel non-coding RNAs. PLoS One, 2014. 9(11): p. e113116.
21. van Niel, G., et al., Exosomes: a common pathway for a specialized function. J Biochem, 2006. 140(1): p. 13-21.
22. Valadi, H., et al., Exosome-mediated transfer of mRNAs and microRNAs is a novel mechanism of genetic exchange between cells. Nat Cell Biol, 2007. 9(6): p. 654-9.
23. Johnstone, R.M., et al., Vesicle formation during reticulocyte maturation. Association of plasma membrane activities with released vesicles (exosomes). J Biol Chem, 1987. 262(19): p. 9412-20.
24. www.exocarta.org.
25. www.microvesicles.org.
26. van Niel, G., G. D'Angelo, and G. Raposo, Shedding light on the cellbiology of extracellular vesicles. Nat Rev Mol Cell Biol, 2018. 19(4): p. 213-228.
27. Mathieu, M., et al., Specificities of secretion and uptake of exosomes and other extracellular vesicles for cell-to-cell communication. Nat Cell Biol, 2019. 21(1): p. 9-17.
28. Fruhbeis, C., et al., Neurotransmitter-triggered transfer of exosomes mediates oligodendrocyte-neuron communication. PLoS Biol, 2013. 11(7): p. e1001604.
29. Men, Y., et al., Exosome reporter mice reveal the involvement of exosomes in mediating neuron to astroglia communication in the CNS. Nat Commun, 2019. 10(1): p. 4136.
30. Morel, L., et al., Neuronal exosomal miRNA-dependent translational regulation of astroglial glutamate transporter GLT1. J Biol Chem, 2013. 288(10): p. 7105-16.
31. Lopez-Verrilli, M.A., F. Picou, and F.A. Court, Schwann Cell-Derived Exosomes Enhance Axonal Regeneration in the Peripheral Nervous System. Glia, 2013. 61(11): p. 1795-1806.
32. Shurtleff, M.J., M.M. Temoche-Diaz, and R. Schekman, Extracellular Vesicles and Cancer: Caveat Lector. Annual Review of Cancer Biology, Vol 2, 2018. 2: p. 395-411.
33. Luga, V., et al., Exosomes mediate stromal mobilization of autocrine Wnt-PCP signaling in breast cancer cellmigration. Cell, 2012. 151(7): p. 1542-56.
34. Hayashi, T., et al., ExosomalMicroRNA Transport from Salivary Mesenchyme Regulates Epithelial Progenitor Expansion during Organogenesis. Dev Cell, 2017. 40(1): p. 95-103.
35. Nakano, S., et al., Role of extracellular vesicles in the interaction between epithelial and mesenchymal cells during oviductal ciliogenesis. Biochem Biophys Res Commun, 2017. 483(1): p. 245-251.
36. Sharma, K.D., et al., Glioma-derived exosomes drive the differentiation of neural stem cells to astrocytes. PLoS One, 2020. 15(7): p. e0234614.
37. Sharma, P., et al., Exosomes regulate neurogenesis and circuit assembly. Proc Natl Acad Sci U S A, 2019. 116(32): p. 16086-16094.
38. Song, L., X. Tian, and R. Schekman, Extracellular vesicles from neurons promote neural induction of stem cells through cyclin D1. J Cell Biol, 2021. 220(9).
39. Grapp, M., et al., Choroid plexus transcytosis and exosome shuttling deliver folate into brain parenchyma. Nat Commun, 2013. 4: p. 2123.
40. Lepko, T., et al., Choroid plexus-derived miR-204 regulates the number of quiescent neural stem cells in the adult brain. EMBO J, 2019. 38(17): p. e100481.
41. Doetsch, F., et al., Subventricularzone astrocytes are neural stem cells in the adult mammalian brain. Cell, 1999. 97(6): p. 703-716.
42. Seri, B., et al., Cell types, lineage, and architecture of the germinal zone in the adult dentate gyrus. J Comp Neurol, 2004. 478(4): p. 359-78.
43. Obernier, K. and A. Alvarez-Buylla, Neural stem cells: origin, heterogeneity and regulation in the adult mammalian brain. Development, 2019. 146(4).
44. Yoo, S. and S. Blackshaw, Regulation and function of neurogenesis in the adult mammalian hypothalamus. Prog Neurobiol, 2018. 170: p. 53-66.
45. Haan, N., et al., Fgf10-Expressing Tanycytes Add New Neurons to the Appetite/Energy-Balance Regulating Centers of the Postnatal and Adult Hypothalamus. Journal of Neuroscience, 2013. 33(14): p. 6170-6180.
46. Robins, S.C., et al., alpha-Tanycytes of the adult hypothalamic third ventricle include distinct populations of FGF-responsive neural progenitors. Nat Commun, 2013. 4: p. 2049.
47. Horiguchi, K., et al., Isolation and characterization of cluster of differentiation 9-positive ependymal cells as potential adult neural stem/progenitor cells in the third ventricle of adult rats. Cell Tissue Res, 2020. 379(3): p. 497-509.
48. Pellegrino, G., et al., A comparative study of the neural stem cell niche in the adult hypothalamus of human, mouse, rat and gray mouse lemur (Microcebus murinus). Journal of Comparative Neurology, 2018. 526(9): p. 1419-1443.
49. Sawamoto, K., et al., New neurons follow the flow of cerebrospinal fluid in the adult brain. Science, 2006. 311(5761): p. 629-32.
50. Kokovay, E., et al., VCAM1 is essential to maintain the structure of the SVZ niche and acts as an environmental sensor to regulate SVZ lineage progression. Cell Stem Cell, 2012. 11(2): p. 220-30.
51. de Sonnaville, S., et al., The adult human subventricularzone: partial ependymal coverage and proliferative capacity of cerebrospinal fluid. Brain Commun, 2020. 2(2): p. fcaa150.
52. Batiz, L.F., et al., Exosomes as Novel Regulators of Adult Neurogenic Niches. Front Cell Neurosci, 2015. 9: p. 501.
53. Losurdo, M. and M. Grilli, Extracellular Vesicles, Influential Players of Intercellular Communication within AdultNeurogenic Niches. International Journal of Molecular Sciences, 2020. 21(22).
54. Willis, C.M., et al., Soluble factors influencing the neural stem cell niche in brain physiology, inflammation, and aging. Exp Neurol, 2022. 355: p. 114124.
55. Zhang, Y., et al., Hypothalamic stem cells controlageing speed partly through exosomal miRNAs. Nature, 2017. 548(7665): p. 52-57.
56. Kowal, J., et al., Proteomic comparison defines novel markers to characterize heterogeneous populations of extracellular vesicle subtypes. Proc Natl Acad Sci U S A, 2016. 113(8): p. E968-77.
57. Crescitelli, R., et al., Subpopulations of extracellular vesicles from human metastatic melanoma tissue identified by quantitative proteomics after optimized isolation. Journal of Extracellular Vesicles, 2020. 9(1).
58. Zheng, W. and Q. Zhao, Establishment and characterization of an immortalized Z310 choroidal epithelial cell line from murine choroid plexus. Brain Res, 2002. 958(2): p. 371-80.
59. Szmydynger-Chodobska, J., et al., Expression of junctional proteins in choroid plexus epithelial cell lines: a comparative study. Cerebrospinal Fluid Res, 2007. 4: p. 11.
60. Shi, L.Z., et al., Use of Z310 cells as an in vitro blood-cerebrospinal fluid barrier model: tight junction proteins and transport properties. Toxicol In Vitro, 2008. 22(1): p. 190-9.
61. Klas, J., et al., Characterization of immortalized choroid plexus epithelial cell lines for studies of transport processes across the blood-cerebrospinalfluid barrier. Cerebrospinal Fluid Res, 2010. 7: p. 11.
62. Hasselblatt, M., et al., TWIST-1 is overexpressed in neoplastic choroid plexus epithelial cells and promotes proliferation and invasion. Cancer Res, 2009. 69(6): p. 2219-23.
63. Dorrier, C.E., et al., Emerging roles for CNS fibroblasts in health, injury and disease. Nat Rev Neurosci, 2022. 23(1): p. 23-34.
64. Silbern, I., et al., Relative Quantification of Phosphorylated and Glycosylated Peptides from the Same Sample Using Isobaric Chemical Labelling with a Two-Step Enrichment Strategy. Quantitative Methods in Proteomics, 2 Edition, 2021. 2228: p. 185-203.
65. Hughes, C.S., et al., Ultrasensitive proteome analysis using paramagnetic bead technology. Molecular Systems Biology, 2014. 10(10).
66. Cox, J., et al., Andromeda: A Peptide Search Engine Integrated into the MaxQuant Environment. Journal of Proteome Research, 2011. 10(4): p. 1794-1805.
67. Bateman, A., et al., UniProt: a worldwide hub of protein knowledge. Nucleic Acids Research, 2019. 47(D1): p. D506-D515.
68. Schwanhausser, B., et al., Global quantification of mammalian gene expression control. Nature, 2011. 473(7347): p. 337-342.
69. Smyth, G.K., limma: LinearModels forMicroarray Data. Bioinformatics and Computational Biology Solution Using R and Bioconductor, 2005: p. 397-420.
70. Storey, J.D., A direct approach to false discovery rates. Journal of the Royal Statistical Society Series B-Statistical Methodology, 2002. 64: p. 479-498.
71. Perez-Riverol, Y., et al., The PRIDE database and related tools and resources in 2019: improving support for quantification data. Nucleic Acids Research, 2019. 47(D1): p. D442-D450.
72. Walker, T.L. and G. Kempermann, One mouse, two cultures: isolation and culture of adult neural stem cells from the two neurogenic zones of individual mice. J Vis Exp, 2014(84): p. e51225.
73. Thery, C., et al., Minimal information for studies of extracellular vesicles 2018 (MISEV2018): a position statement of the International Society for Extracellular Vesicles and update of the MISEV2014guidelines. J Extracell Vesicles, 2018. 7(1): p. 1535750.
74. Tkach, M., J. Kowal, and C. Thery, Why the need and how to approach the functional diversity of extracellular vesicles. Philos Trans R Soc Lond B Biol Sci, 2018. 373(1737).
75. Konoshenko, M.Y., et al., Isolation of Extracellular Vesicles: General Methodologies and Latest Trends. Biomed Research International, 2018. 2018.
76. Kaneko, N., et al., New neurons use Slit-Robo signaling to migrate through the glialmeshwork and approach a lesion for functional regeneration. Sci Adv, 2018. 4(12): p. eaav0618.
77. Nguyen-Ba-Charvet, K.T. and A. Chedotal, Role of Slit proteins in the vertebrate brain. J Physiol Paris, 2002. 96(1-2): p. 91-8.
78. Ito, K., et al., Gfap and Osmr regulation by BRG1 and STAT3 via interchromosomal gene clustering in astrocytes. Molecular Biology of the Cell, 2018. 29(2): p. 209-219.
79. Brenner, M. and A. Messing, Regulation of GFAP Expression. Asn Neuro, 2021. 13.
80. Dorrier, C.E., et al., CNS fibroblasts form a fibrotic scar in response to immune cell infiltration. Nat Neurosci, 2021. 24(2): p. 234-244.
81. Dhavan, R. and L.H. Tsai, A decade ofCDK5. Nature Reviews Molecular Cell Biology, 2001. 2(10): p. 749-759.
82. Shurtleff, M.J., et al., Broad role for YBX1 in defining the small noncoding RNA composition of exosomes. Proc Natl Acad Sci U S A, 2017. 114(43): p. E8987-E8995.
83. Del Bigio, M.R., Ependymal cells: biology and pathology. Acta Neuropathol, 2010. 119(1): p. 55-73.
84. Langlet, F., et al., Tanycyte-like cells form a blood-cerebrospinal fluid barrier in the circumventricular organs of the mouse brain. J Comp Neurol, 2013. 521(15): p. 3389-405.
85. Cravatt, B.F., et al., Chemical characterization of a family of brain lipids that induce sleep. Science, 1995. 268(5216): p. 1506-9.
86. Myung, J., et al., The choroid plexus is an important circadian clock component. Nat Commun, 2018. 9(1): p. 1062.
87. Loh, C.Y., et al., The E-Cadherin and N-Cadherin Switch in Epithelial-to-Mesenchymal Transition: Signaling, Therapeutic Implications, and Challenges. Cells, 2019. 8(10).
88. Barcelona-Estaje, E., et al., You Talking to Me? Cadherin and Integrin Crosstalk in Biomaterial Design. Advanced Healthcare Materials, 2021. 10(6).
89. Wu, Y.Z., et al., Stat3 enhances vimentin gene expression by binding to the antisilencer element and interacting with the repressor protein, ZBP-89. Oncogene, 2004. 23(1): p. 168-178.
90. Holt, C.E., K.C. Martin, and E.M. Schuman, Local translation in neurons: visualization and function. Nat Struct Mol Biol, 2019. 26(7): p. 557-566.
91. Cagnetta, R., et al., Rapid Cue-Specific Remodeling of the NascentAxonal Proteome. Neuron, 2018. 99(1): p. 29-46 e4.
92. McKelvey, K.J., et al., Exosomes: Mechanisms of Uptake. J Circ Biomark, 2015. 4: p. 7.
93. Margolis, L. and Y. Sadovsky, The biology of extracellular vesicles: The known unknowns. PLoS Biol, 2019. 17(7): p. e3000363.
94. Alabi, A.A. and R.W. Tsien, Perspectives on kiss-and-run: role in exocytosis, endocytosis, and neurotransmission. Annu Rev Physiol, 2013. 75: p. 393-422.
95. Takamori, S., et al., Molecular anatomy of a trafficking organelle. Cell, 2006. 127(4): p. 831-46.
96. Taoufiq, Z., et al., Hidden proteome of synaptic vesicles in the mammalian brain. Proc Natl Acad Sci U S A, 2020. 117(52): p. 33586-33596.
97. Zuniga, A. and R. Zeller, Dynamic and self-regulatory interactions among gene regulatory networks control vertebrate limb bud morphogenesis. Curr Top Dev Biol, 2020. 139: p. 61-88.
98. Russell, A.E., et al., Biological membranes in EV biogenesis, stability, uptake, and cargo transfer: an ISEV position paper arising from the ISEV membranes and EVs workshop. J Extracell Vesicles, 2019. 8(1): p. 1684862.
99. Bonsergent, E., et al., Quantitative characterization of extracellular vesicle uptake and content delivery within mammalian cells. Nat Commun, 2021. 12(1): p. 1864.
100. Ghersi-Egea, J.F., et al., Molecular anatomy and functions of the choroidalblood-cerebrospinal fluid barrierin health and disease. Acta Neuropathol, 2018. 135(3): p. 337-361.
101. Sobek, J., Aquino, C., Weigel, W. et al. Drop drying on surfaces determines chemical reactivity - the specific case of immobilization of oligonucleotides on microarrays. BMC Biophys 6, 8 (2013).
102. Görgens, A., Corso, G., Hagey, D. W., Jawad Wiklander, R., Gustafsson, M. O., Felldin, U., Lee, Y., Bostancioglu, R. B., Sork, H., Liang, X., Zheng, W., Mohammad, D. K., van de Wakker, S. I., Vader, P., Zickler, A. M., Mamand, D. R., Ma, L., Holme, M. N., Stevens, M. M., ... & EL Andaloussi, S. (2022). Identification of storage conditions stabilizing extracellular vesicles preparations. Journal of Extracellular Vesicles, 11, e12238.

## Claims

1. A sterile surface suitable for culturing cells, wherein the surface is coated with extracellular vesicles ('EVs') immobilized at one or more predetermined position(s).

2. The surface of claim 1, wherein the surface is storable at a temperature from - 80 to -20 °C, on dry ice or in liquid nitrogen, preferably at a temperature from - 80 to -20 °C or on dry ice, and more preferably at a temperature around -80 °C, at around -20 °C, or on dry ice; and/or
wherein the surface is storable under desiccating conditions, preferably wherein the surface is stored under desiccating conditions.

3. The surface claim 1 or 2, wherein the immobilized EVs originate from EV-producer cells and wherein the EV-producer cells are an immortalized cell line, a cancer cell line, a stem cell, an induced pluripotent stem cell, a primary cell culture, or a cell of primary tissue sample; or
wherein the immobilized EVs are isolated from a secretome contained in a body fluid.

4. The surface of any of the preceding claims, wherein the EVs have been dried, preferably wherein the EVs have been dried by evaporation, and more preferably wherein the EVs have not been dried by lyophilisation.

5. The surface of any of the preceding claims, wherein the predetermined position(s) is/are coated entirely with immobilized EVs, preferably wherein the predetermined position(s) is/are (a) deepening(s), more preferably wherein the deepening(s) is/are (a) well(s) in a cell culture plate, more preferably wherein the cell culture plate is a multi-well plate; or
wherein the predetermined position(s) is/are coated partially with immobilized EVs, preferably wherein the predetermined position(s) is/are a petri dish or a specimen slide.

6. The surface of any of the preceding claims, wherein each predetermined position has been coated with a solution comprising EVs in an amount ranging from 8×10⁶ to 3×10¹⁰, preferably from 8×10⁷ to 3×10¹⁰, more preferably from 8×10⁸ to 3×10¹⁰, more preferably from 1×10⁹ to 3×10¹⁰, more preferably from 2×10⁹ to 4×10¹⁰, more preferably from 4×10⁹ to 3×10¹⁰, more preferably from 6×10⁹ to 3×10¹⁰, and more preferably from 8×10⁹ to 3×10¹⁰; measurable by a nanoparticle tracking analyzer.

7. The surface of any of the preceding claims obtained by or obtainable by
(i) purifying the EVs from EV-producer cells to obtain EVs in solution and
(ii) depositing and drying a portion of the solution of step (i) on said surface thereby immobilizing the EVs on said surface.

8. The surface of claim 7, wherein the portion of the solution of step (ii) is a fraction of a density gradient, preferably wherein the density gradient is an iodixanol gradient, a sucrose gradient or a colloidal silica particles coated with polyvinylpyrrolidone gradient, preferably wherein the density gradient is an iodixanol gradient.

9. The surface of claims 7 or 8, wherein the EVs are isolated in step (i) by ultracentrifugation followed by flotation in a density gradient, size exclusion chromatography, immune capture, flow cytometry or by combination thereof, preferably wherein the EVs are isolated in step (i) by ultracentrifugation followed by flotation in a density gradient, size exclusion chromatography or immune capture, and more preferably by ultracentrifugation followed by flotation in a density gradient.

10. A device for culturing cells comprising the surface of any of claims 1-9 further comprising a removable covering to preserve sterility of said surface, preferably wherein the covering is a foil or lid, and even more preferably a lid.

11. The device of claim 10, wherein the device is a multi-well plate, preferably a 6-well, a 12-well, a 24-well, a 48-well, a 96-well, a 128-well or a 384-well plate, preferably wherein the removable lid is sealed to the multi-well plate to protect dryness, and more preferably wherein the removable lid is sealed with tape.

12. The device of claim 11, wherein a predetermined amount of EVs has been serially diluted along a part of a row or a column of the multi-well plate, preferably wherein the multi-well plate is an EV library, more preferably wherein EVs from two or more different sources are serially diluted and wherein said different sources are selected from a human cell line, an animal cell line, a plant cell, a protozoan and/or a body fluid.

13. A kit comprising the device of any of claims 10-12 and further comprising a desiccant, preferably wherein the desiccant is silica gel.

14. A method of preparing a sterile surface according to any of claims 1-9, comprising the steps of
a. purifying the EVs by ultracentrifugation to obtain a pellet comprising the EVs,
b. further purifying the EVs from said pellet by flotation in a density gradient to obtain a soluble fraction comprising the EVs,
c. depositing a portion of said fraction on the sterile surface, and
d. letting said portion dry by evaporation on the sterile surface,
whereby immobilized EVs on the sterile surface are obtained.

15. A method of monitoring cell differentiation, apoptosis, cell migration, proliferation, transcriptome, proteome, and/or viability of cells ("recipient cells') comprising the steps of
a. adding said recipient cells to the surface according to any of claims 1-9 and
b. monitoring said cells by microscopy, qPCR, single cell sequencing, colometric detection in a plate reader, ChiP sequencing, mass spectrometry, DNA methylation and/or DNA acetylation analysis.
